# EUROPEAN PATENT APPLICATION

(11) **EP 4 122 426 A1**
(43) Date of publication of application: **25.01.2023**
(21) Application number: 21770911.2
(22) Date of filing: 18.03.2021
(51) Int. Cl.: A61F 2/24, A61B 17/10

(54) **VALVE CLAMPING DEVICE AND VALVE CLAMPING SYSTEM**

(30) Priority: 18.03.2020 CN 202010192630; 17.08.2020 CN 202021717762 U; 15.01.2021 CN 202120119689 U; 15.01.2021 CN 202120115656 U; 15.01.2021 CN 202110057559; 15.01.2021 CN 202110057563
(71) Applicant: Hangzhou Valgen Medtech Co., Ltd., Hangzhou, Zhejiang 310052 (CN)
(72) Inventor: ZHANG, Tingchao, Hangzhou, Zhejiang 310052 (CN); ZHANG, Weiwei, Hangzhou, Zhejiang 310052 (CN); WANG, Zetao, Hangzhou, Zhejiang 310052 (CN); ZHENG, Xianzhang, Hangzhou, Zhejiang 310052 (CN)
(74) Representative: J A Kemp LLP
(86) International application number: PCT/CN2021/081606
(87) International publication number: WO 2021/185324

(57) **Abstract**

Disclosed are a valve clamping device and a valve clamping system, wherein the valve clamping device comprises: a supporting portion provided along an axial direction; a hollow adjusting portion, wherein at least part of the supporting portion is provided in the adjusting portion, one end of the adjusting portion is sleeved outside the supporting portion, and the other end of the adjusting portion is movable with respect to the supporting portion; a clamping portion surrounding an outside of the adjusting portion; and a driving portion connected to the clamping portion to drive the clamping portion to be close to or away from the adjusting portion.

## Description

### CROSS-REFERENCES TO RELATED APPLICATIONS

The present disclosure claims all the benefits of the Chinese patent application No. 202010192630.9, filed on March 18, 2020 to the China National Intellectual Property Administration of the People's Republic of China, entitled "Valve Clamping Device And Valve Clamping System"; the Chinese patent application No. 202110057563.4, filed on January 15, 2021 to the China National Intellectual Property Administration of the People's Republic of China, entitled "Adequately Fitted Valve Clamping Device And Valve Clamping System"; the Chinese patent application No. 202110057559.8, filed on January 15, 2021 to the China National Intellectual Property Administration of the People's Republic of China, entitled "Adaptive Valve Clamping Device And Valve Clamping System"; the Chinese Utility Model application No. 202120115656.3, filed on January 15, 2021 to the China National Intellectual Property Administration of the People's Republic of China, entitled "Adequately Fitted Valve Clamping Device And Valve Clamping System"; the Chinese Utility Model application No. 202021717762.0, filed on August 17, 2020 to the China National Intellectual Property Administration of the People's Republic of China, entitled "Valve Clamping Device And Valve Clamping System"; and the Chinese Utility Model application No. 202120119689.5, filed on January 15, 2021 to the China National Intellectual Property Administration of the People's Republic of China, entitled "Adaptive Valve Clamping Device And Valve Clamping System", which are explicitly incorporated herein by reference in their entirety.

### FIELD

The present disclosure generally relates to the technical field of medical instruments, particularly to a valve clamping device and a valve clamping system.

### BACKGROUND

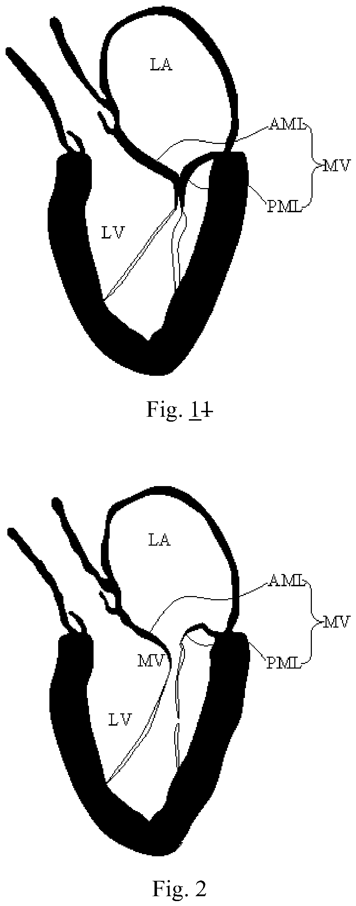

Referring to Fig. 1, mitral valve MV is a one-way valve located between left atrium LA and left ventricle LV of the heart. A normal and healthy mitral valve MV can control blood to flow from the left atrium LA to the left ventricle LV while avoiding blood flowing from the left ventricle LV to the left atrium LA. The mitral valve MV comprises a pair of valve leaflets, called an anterior mitral leaflet AML and a posterior mitral leaflet PML. The anterior mitral leaflet AML and the posterior mitral leaflet PML are fixed to papillary muscles of the left ventricle LV through chordae tendineae. Under normal circumstances, when the left ventricle LV contracts, edges of the anterior mitral leaflet AML and the posterior mitral leaflet PML are completely coapted to avoid blood flowing from the left ventricle LV to the left atrium LA. Referring to Fig. 2, when the valve leaflet of the mitral valve MV or a related structure thereof changes organically or functionally, for example, chordae tendineae partially ruptures, the anterior mitral leaflet AML and the posterior mitral leaflet PML of the mitral valve MV are poorly coapted. Therefore, when the left ventricle LV contracts, the mitral valve MV cannot be completely closed, resulting in blood regurgitation from the left ventricle LV to the left atrium LA and thus a series of pathophysiological changes, called "mitral regurgitation".

Mitral valve interposed clamping refers to treatment of mitral regurgitation by pulling the anterior mitral leaflet and the posterior mitral leaflet toward each other by a pair of rotatably connected closable clamp arms with a valve clamping device so as to reduce or eliminate a valve leaflet space. A valve clamping device in the related art adds an elastomer between two clamp arms, and valve leaflets on each side are clamped between one clamp arm and one side of an elastomer respectively. A space between the clamp arms on both sides is filled with the elastomer to reduce central regurgitation, and spacing of the valve leaflets is adapted by deformation of the elastomer so as to adjust pulling degree of the clamp arm to the valve leaflet. However, when the clamp arm is closed, the closer to a clamp arm connection, the smaller the space is. When the valve leaflet is gripped by the clamp arm, part of the valve leaflet fills and accumulates in the space, which will affect closing of the clamp arms. Meanwhile, if a valve leaflet filling condition cannot be found in time through a medical image, the clamp arm will damage the valve leaflet when an operator forcibly closes the clamping device.

### SUMMARY

In an aspect, the present disclosure relates to a valve clamping device comprising:
a supporting portion provided along an axial direction;
a hollow adjusting portion, wherein at least a part of the supporting portion is provided in the adjusting portion, one end of the adjusting portion is sleeved outside the supporting portion, and the other end of the adjusting portion is movable with respect to the supporting portion;
a clamping portion surrounding an outside of the adjusting portion; and
a driving portion connected to the clamping portion to drive the clamping portion to be close to or away from the adjusting portion.

In some embodiments, the supporting portion comprises a first seat body and a second seat body connected along the axial direction, and the first seat body is provided in the adjusting portion; and the adjusting portion comprises a first end and a second end provided opposite along the axial direction, and a self-expanding body between the first end and the second end.

In some embodiments, the first end is fixedly sleeved outside the second seat body.

In some embodiments, the second end hangs freely with respect to the supporting portion to be movable with respect to the supporting portion.

In some embodiments, the second end is movably sleeved outside the first seat body and is movable along the axial direction with respect to the supporting portion.

In some embodiments, the second end is fixedly sleeved outside the first seat body, the first end is movably sleeved outside the second seat body and is movable along the axial direction with respect to the supporting portion.

In some embodiments, the first end is movably sleeved outside the second seat body and is movable along the axial direction with respect to the supporting portion.

In some embodiments, the second end is sleeved outside the first seat body and is movable along the axial direction with respect to the supporting portion.

In some embodiments, the second end hangs freely with respect to the supporting portion to be movable with respect to the supporting portion.

In some embodiments, the self-expanding body is an elastomer, and the adjusting portion is provided with an opening at the second end.

In some embodiments, the elastomer is of at least one structure selected from the group consisting of a mesh structure, a frame structure, a dense structure and a porous structure.

In some embodiments, the elastomer is of a mesh structure or a frame structure, and at least part of an outer surface and/or at least part of an inner surface of the elastomer are covered with a biocompatible film.

In some embodiments, the second end of the adjusting portion is a head with a central through hole, a part of the elastomer penetrates and is fixed in the head, and the central through hole forms the opening; or an edge of the second end is sleeved with a hollow sleeve structure to form the opening; or the edge of the second end is enclosed to form the opening.

In some embodiments, the elastomer is of a mesh structure, the elastomer is woven from a shape memory material, and a mesh wire woven to form the mesh structure is bent back at the second end to form the opening.

In some embodiments, the elastomer is of a frame structure, the frame structure is cut from a shape memory material, the frame structure comprises a plurality of supporting rods, the adjacent supporting rods are spaced apart from or cross-linked with each other, and proximal ends of a plurality of the supporting rods converge to form the opening.

In some embodiments, the elastomer is of a dense structure made of silica gel; or the elastomer is of a porous structure made of sponge; and an edge of the second end of the dense structure or the porous structure forms the opening.

In some embodiments, a diameter of at least a part of the self-expanding body in a natural state gradually increases from the first end of the adjusting portion to the second end of the adjusting portion.

In some embodiments, the self-expanding body has a recessed area connected to the second end, the recessed area is recessed toward the first end, and the second end is located between two end faces of the self-expanding body along an axis.

In some embodiments, the self-expanding body comprises a first section, a second section and a third section successively connected;
the first section extends from the second end of the adjusting portion toward a second end of the supporting portion, and the first section surrounds an outside of the second end of the supporting portion; the second section continues to extend radially outward from the first section; and the third section extends radially inward from the second section toward a first end of the supporting portion to the first end of the adjusting portion.

In some embodiments, the self-expanding body further comprises a bending section connected between the second end of the adjusting portion and the first section.

In some embodiments, a radial dimension of the second section of the self-expanding body ranges from 4 mm to 15 mm, and a radial dimension of the first end of the adjusting portion ranges from 1 mm to 5 mm.

In some embodiments, the self-expanding body comprises a plurality of first curved surfaces and a plurality of second curved surfaces along a circumferential direction, the first curved surface and the second curved surface are adjacent to each other, the two oppositely provided first curved surfaces face the clamping portion respectively, and an area of the second curved surface is less than an area of the first curved surface.

In some embodiments, the first seat body comprises an interface end connected to the second seat body and a free end provided oppositely to the interface end, and the free end is located in the adjusting portion.

In some embodiments, the self-expanding body is an elastomer, and the adjusting portion is provided with an opening at the second end; and a size of the opening is less than or equal to a size of the free end.

In some embodiments, a hollow sleeve structure is sleeved outside an edge of the end of the adjusting portion, and the sleeve structure is sleeved outside the supporting portion.

In some embodiments, the clamping portion comprises at least two clamp arms, the at least two clamp arms are symmetrically provided with respect to the adjusting portion, and the driving portion is connected to each of the clamp arms to drive each of the clamp arms to be close to or away from the adjusting portion.

In some embodiments, the supporting portion further comprises a base connected to the second seat body, each of the clamp arms is rotationally connected to the base, and there is axial spacing between the first end and a connecting position of the clamp arm and the base.

In some embodiments, the driving portion comprises a driving shaft, a connecting seat and at least two connecting rods; wherein one end of each of the connecting rods is connected to the clamping portion, and the other end of each of the connecting rods is pivotally connected to the connecting seat; and one end of the driving shaft is connected to the connecting base, and the other end of the driving shaft movably penetrates in the base.

In some embodiments, the valve clamping device further comprises a locking portion provided in the base, and the locking portion restricts relative movement of the driving shaft and the base.

In some embodiments, the driving portion comprises a driving shaft, an automatic closing unit and at least two connecting rods; the driving shaft movably penetrates in the supporting portion, one end of each of the connecting rods is rotationally connected to one of the clamp arms, and the other end of each of the connecting rods is rotationally connected to the driving shaft; and the automatic closing unit is connected to the clamp arm to cause the clamp arm to abut against the adjusting portion in a natural state.

In some embodiments, the driving portion comprises a driving shaft and at least two elastic driving arms, one end of the driving shaft movably penetrates in the supporting portion, one end of each of the elastic driving arms is fixedly connected to the other end of the driving shaft, and the other end of each of the elastic driving arms is respectively connected to one of the clamp arms; and the elastic driving arms are configured to cause the clamp arms to abut against the adjusting portion in a natural state.

In some embodiments, a terminal end of the clamp arm is provided with a flanging section which is a cambered surface overturned toward an outside of the terminal end of the clamp arm, and the self-expanding body protrudes from the flanging section in the axial direction after the clamp arm abuts against the adjusting portion.

In some embodiments, the self-expanding body is provided with an adaptation section corresponding to the flanging section, and a shape of the adaptation section toward the flanging section is complementary to the cambered surface.

In some embodiments, the valve clamping device further comprises a gripping portion provided between the clamping portion and the adjusting portion, the gripping portion is able to be close to or away from the adjusting portion, and when the gripping portion and the clamping portion are away from the adjusting portion, the gripping portion is at least partially accommodated in an inner surface of the clamping portion.

In another aspect, the present disclosure relates to a valve clamping device comprising:
a supporting portion which comprises a connecting end and a free end provided oppositely;
a hollow adjusting portion, at least a part of the supporting portion is provided in the adjusting portion, one end of the adjusting portion is sleeved outside the connecting end and connected to the supporting portion, and the other end of the adjusting portion hangs freely;
a clamping portion surrounding an outside of the adjusting portion; and
a driving portion connected to the clamping portion to drive the clamping portion to open or close around the adjusting portion.

In some embodiments, a free end of the supporting portion is within the adjustment portion.

In some embodiments, the adjusting portion comprises an elastomer, one end of the elastomer is connected to the supporting portion, and the other end of the elastomer has an opening and hangs freely.

In some embodiments, a size of the opening is less than or equal to a size of the free end.

In some embodiments, a proximal edge of the elastomer is sleeved with a hollow sleeve structure to form the opening.

In some embodiments, a proximal edge of the elastomer is enclosed to form the opening.

In some embodiments, the elastomer is of at least one structure selected from the group consisting of a mesh structure, a frame structure, a dense structure and a porous structure.

In some embodiments, when the elastomer is of the mesh structure or the frame structure, at least part of an outer surface of the elastomer is covered with a film.

In some embodiments, when the elastomer is of the mesh structure or the frame structure, the elastomer is woven or cut from a shape memory material.

In some embodiments, when the elastomer is the mesh structure, a mesh wire of the mesh structure is bent back at a proximal end to form the proximal edge.

In some embodiments, when the elastomer is of the frame structure, adjacent supporting rods of the frame structure are spaced apart from or cross-linked with each other, and the supporting rods of the frame structure converge at a proximal end to form the proximal edge.

In some embodiments, when the elastomer is of the dense structure, the dense structure is made of silica gel; when the elastomer is of the porous structure, the porous structure is made of sponge; and a proximal edge of the dense structure or the porous structure forms the opening.

In some embodiments, a distal end of the elastomer is fixedly sleeved on the supporting portion, or a hollow sleeve structure is sleeved outside a distal edge of the elastomer, and the sleeve structure is fixedly sleeved on the supporting portion.

In some embodiments, the clamping portion comprises at least two clamp arms, the at least two clamp arms are symmetrically provided with respect to the adjusting portion, and the driving portion is connected to each of the clamp arms to drive each of the clamp arms to rotate around the adjusting portion.

In some embodiments, the adjusting portion comprises a plurality of first curved surfaces and a plurality of second curved surfaces, the first curved surface and the second curved surfaces are adjacent to each other, the two oppositely provided first curved surfaces face one of the clamp arms respectively, and an area of the second curved surface is smaller than an area of the first curved surface.

In some embodiments, the valve clamping device further comprises a gripping portion provided between the clamping portion and the adjusting portion and being able to close or open with respect to the adjusting portion, and when both of the gripping portion and the clamping portion open, the gripping portion is at least partially accommodated in an inner surface of the clamping portion.

In some embodiments, the valve clamping device further comprises a base fixedly connected to the supporting portion, and the clamping portion is rotationally connected to the base.

In some embodiments, the driving portion comprises a driving shaft, a connecting seat and at least two connecting rods; wherein one end of each of the connecting rods is connected to the clamping portion, and the other end of each of the connecting rods is pivotally connected to the connecting seat; and one end of the driving shaft is connected to the connecting base, and the other end of the driving shaft movably penetrates in the base.

In some embodiments, the valve clamping device further comprises a locking portion provided in the base, and the locking portion restricts relative movement of the driving shaft and the base.

In still another aspect, the present disclosure provides an adequately fitted valve clamping device comprising:
a supporting portion with a certain axial length comprising a first end and a second end provided oppositely;
an adjusting portion comprising a first end and a second end provided oppositely and a self-expanding body between the first end and the second end of the adjusting portion; the first end of the adjusting portion is movably sleeved outside the supporting portion, the second end of the adjusting portion is sleeved outside the supporting portion and fixedly connected to the supporting portion, and the first end of the adjusting portion is located between the first end of the supporting portion and the second end of the adjusting portion; and
a clamping portion provided on the outside of the supporting portion and being able to open or close with respect to the adjusting portion.

In some embodiments, a diameter of the self-expanding body in a natural state gradually increases from the first end of the adjusting portion to the second end of the adjusting portion.

In some embodiments, the self-expanding body is of a mesh structure made of a shape memory material.

In some embodiments, an outside and/or inside of the mesh structure is covered with a biocompatible film.

In some embodiments, the self-expanding body has a recessed area connected to the second end of the adjusting portion, and a terminal end of the recessed area extends toward the first end of the supporting portion to the second end of the adjusting portion, or the terminal end of the recessed area extends toward the second end of the supporting portion to the second end of the adjusting portion.

In some embodiments, the adequately fitted valve clamping device further comprises a fixing part, and the second end of the adjusting portion penetrates and is fixed in the fixing part to be fixedly connected to the supporting portion through the fixing part.

In some embodiments, the self-expanding body comprises a first section, a second section and a third section successively connected;
the first section extends from the second end of the adjusting portion toward a second end of the supporting portion, and the first section surrounds an outside of the second end of the supporting portion; the second section continues to extend radially outward from the first section; and the third section extends radially inward from the second section toward a first end of the supporting portion to the first end of the adjusting portion.

In some embodiments, the self-expanding body further comprises a bending section connected between the second end of the adjusting portion and the first section.

In some embodiments, a radial dimension of the second section of the self-expanding body ranges from 4 mm to 15 mm, and a radial dimension of the first end of the adjusting portion ranges from 1 mm to 5 mm.

In some embodiments, the valve clamping device further comprises a driving portion, the clamping portion comprises at least two clamp arms, the at least two clamp arms are symmetrically provided with respect to the adjusting portion, and the driving portion is connected to each of the clamp arms to drive each of the clamp arms to be close to or away from the adjusting portion.

In some embodiments, the first end of the supporting portion is provided with a base, each of the clamp arms is rotationally connected to the base, and there is axial spacing between the first end of the supporting portion and the first end of the adjusting portion.

In some embodiments, the driving portion comprises a driving shaft, a connecting seat and at least two connecting rods; one end of each of the connecting rods is rotationally connected to one of the clamp arms, and the other end of each of the connecting rods is rotationally connected to the connecting seat; and one end of the driving shaft is connected to the connecting seat, and the other end of the driving shaft movably penetrates in the base.

In some embodiments, the valve clamping device further comprises a locking portion provided in the base, and the locking portion restricts relative movement of the driving shaft and the base.

In some embodiments, the driving portion comprises a driving shaft, an automatic closing unit and at least two connecting rods; the driving shaft movably penetrates in the supporting portion, one end of each of the connecting rods is rotationally connected to one of the clamp arms, and the other end of each of the connecting rods is rotationally connected to the driving shaft; and the automatic closing unit is connected to the clamp arm to cause the clamp arm to abut against the adjusting portion in a natural state.

In some embodiments, the driving portion comprises a driving shaft and at least two elastic driving arms, one end of the driving shaft movably penetrates in the supporting portion, and one end of each of the elastic driving arms is fixedly connected to the other end of the driving shaft, the other end of each of the elastic driving arms is connected to one of the clamp arms; and the elastic driving arms are configured to cause the clamp arms to abut against the adjusting portion in a natural state.

In some embodiments, a terminal end of the clamp arm is provided with a flanging section, the flanging section is a cambered surface overturned toward an outside of the terminal end of the clamp arm, and the self-expanding body protrudes from the flanging section in the axial direction after the clamp arm abuts against the adjusting portion.

In some embodiments, the self-expanding body is provided with an adaptation section corresponding to the flanging section, and a shape of the adaptation section toward the flanging section is complementary to the cambered surface.

In some embodiments, the valve clamping device further comprises a gripping portion provided between the clamping portion and the adjusting portion and being able to close or open with respect to the supporting portion.

In still another aspect, the present disclosure relates to an adaptive valve clamping device comprising:
a supporting portion comprising a first seat body and a second seat body connected to the first seat body;
a hollow adjusting portion, wherein the first seat body is provided in the adjusting portion, the adjusting portion comprises a first end and a second end provided oppositely, and a self-expanding body between the first end and the second end, the first end of the adjusting portion is movably sleeved outside the second seat body and is able to move axially with respect to the second seat body, the second end of the adjusting portion hangs, and the first seat body is closer to the second end of the adjusting portion than the second seat body; and
a clamping portion comprising at least two clamp arms, each of the clamp arms is rotationally connected to the supporting portion, a rotationally connected portion of the clamp arms is close to the first end of the adjusting portion, and the clamp arms rotate around the supporting portion to be close to or away from the adjusting portion.

In some embodiments, the first end of the adjusting portion is provided with a first head, and there is a clearance fit between an inner cavity surface of the first head and an outer surface of the second seat body.

In some embodiments, the clearance fit between the inner cavity surface of the first head and the outer surface of the second seat body ranges from 0.01 mm to 3 mm.

In some embodiments, the clearance fit between the inner cavity surface of the first head and the outer surface of the second seat body ranges from 0.05 mm to 1 mm.

In some embodiments, the clearance fit between the inner cavity surface of the first head and the outer surface of the second seat body ranges from 0.05 mm to 0.2 mm.

In some embodiments, a surface roughness of the inner cavity surface of the first head ranges from 0.1 µm to 2.5 µm, and/or a surface roughness of the outer surface of the second seat body ranges from 0.1 µm to 2.5 µm.

In some embodiments, the inner cavity of the first head is provided with a first rotation stop, the outer surface of the second seat body is provided with a second rotation stop corresponding to the first rotation stop, and the first rotation stop is detachably fittingly connected to the second rotation stop.

In some embodiments, the first head and the first rotation stop are integrally molded or separately connected, and the second seat body and the second rotation stop are integrally molded or separately connected.

In some embodiments, the first rotation stop comprises at least one plane surface and/or at least one cambered surface, and the second rotation stop comprises at least one plane surface and/or at least one cambered surface.

In some embodiments, the first rotation stop and the second rotation stop are of a polyhedron structure fittingly connected.

In some embodiments, one of the first rotation stop and the second rotation stop is a sliding groove extending axially, and the other is a protrusion fitting in the sliding groove.

In some embodiments, the supporting portion further comprises a third seat body connected to the second seat body, an end of the second seat body connected to the first seat body is provided with a limiting part, an inner diameter of the first head is less than an outer diameter of the limiting part, and the inner diameter of the first head is less than an outer diameter of the third seat body.

In some embodiments, the supporting portion further comprises a third seat body connected to the second seat body, an inner diameter of the first head is less than an outer diameter of the first seat body, and an inner diameter of the first head is less than an outer diameter of the third seat body.

In some embodiments, the at least two clamp arms are rotationally connected to the third seat body, and the at least two clamp arms are symmetrically provided in a circumferential direction with respect to the adjusting portion.

In some embodiments, the adaptive valve clamping device further comprises a driving portion comprising a driving shaft, a connecting seat and at least two connecting rods; one end of each of the connecting rods is connected to one of the clamp arms, and the other end of each of the connecting rods is pivotally connected to the connecting seat; and one end of the driving shaft is connected to the connecting seat, and the other end of the driving shaft movably penetrates in the third seat body.

In some embodiments, the driving portion further comprises a locking part provided in the third seat body, and the locking part is configured to limit relative movement of the driving shaft and the third seat body.

In some embodiments, the second end of the adjusting portion has an opening.

In some embodiments, the second end of the adjusting portion is further provided with a second head.

In some embodiments, the adaptive valve clamping device further comprises a gripping portion provided between the clamp arm and the adjusting portion and being able to be close to or away from the clamp arms, and the gripping portion is at least partially accommodated in an inner surface of the clamp arm in a natural state.

In still another aspect, the present disclosure relates to a valve clamping system comprising the valve clamping device of the above aspects and a delivery device, wherein the delivery device comprises: a pushing shaft with a certain axial length and a mandrel movably penetrating in the pushing shaft, the pushing shaft and the supporting portion are detachably connected, and the mandrel is connected to the driving portion to drive the clamping portion to open and close with respect to the supporting portion.

In still another aspect, the present disclosure relates to a valve clamping system comprising the valve clamping device of the above aspects and a delivery device, wherein the delivery device comprises: a pushing shaft with a certain axial length and a mandrel movably penetrating in the pushing shaft, the pushing shaft and the supporting portion are detachably connected, and the mandrel is configured to drive the clamp arms to rotate around the supporting portion.

In some embodiments, at least part of a supporting portion of a valve clamping device and a valve clamping system comprising the valve clamping device is provided in a hollow of an adjusting portion. One end of the adjusting portion is sleeved outside the supporting portion, the other end of the adjusting portion is movable with respect to the supporting portion, and the movable end is no longer completely limited by the supporting portion or other devices, which improves a deformation ability of the adjusting portion. Therefore, when the valve clamping device is radially compressed into the delivery device for in vivo delivery, the valve clamping device is not only easy to be compressed into a sheath, but also can adapt to different blood vessels during delivery in the blood vessel, so as to facilitate passage of the delivery device in the blood vessel, thereby reducing the damage to the vessel wall. In addition, after the valve clamping device is implanted, in a process of clamping the valve leaflet and the adjusting portion with the clamp arm, since the adjusting portion can be deformed, an elastic fit between the valve leaflet and the adjusting portion can be improved, and thus adaptability to physiological structures of valve leaflets in different patients can be improved.

### BRIEF DESCRIPTION OF THE DRAWINGS

The drawings herein are incorporated into the specification and constitute a part of the specification. The drawings show examples conforming to examples of the present disclosure and are used to explain the principle of the present disclosure together with the specification.

In order to explain the technical solutions more clearly in the examples of the present disclosure or the related art, the drawings that need to be used in the description of the examples or the related art are briefly explained. Obviously, one skilled in the art can obtain other drawings based on these drawings without involving creative efforts.
Fig. 1 shows a schematic diagram of a normal state of a mitral valve;
Fig. 2 shows a schematic diagram of a mitral valve in the presence of a lesion;

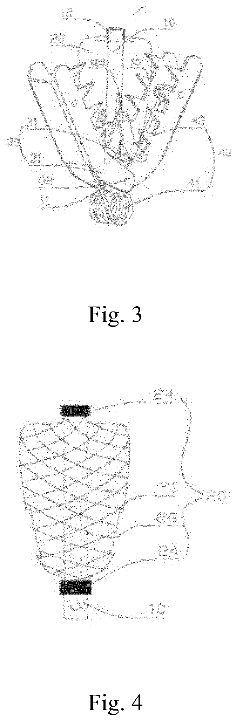

Fig. 3 and Fig. 4 show structural schematic diagrams of a valve clamping device in the related art;
Fig. 5 shows a structural schematic diagram of a valve clamping device according to a first example of a first embodiment of the present disclosure;
Fig. 6 shows a structural schematic diagram after combination of an adjusting portion and a supporting portion in Fig. 5;
Fig. 7 shows a structural schematic diagram of a supporting portion in Fig. 5;
Fig. 8 shows a structural schematic diagram of an adjusting portion in Fig. 5;
Fig. 9a shows a structural schematic diagram of a tube body for preparing an adjusting portion in Fig. 5 in an example;
Fig. 9b shows a structural schematic diagram of a frame structure of the tube body in Fig. 9a after cutting and shaping;
Fig. 9c shows a structural schematic diagram of an example of an elastomer prepared by cutting;
Fig. 9d shows a structural schematic diagram of another example of an elastomer prepared by cutting;
Fig. 9e shows a partial schematic diagram of a proximal part of the elastomer in Fig. 9d;
Fig. 9f shows a structural schematic diagram of still another example of an elastomer prepared by cutting;
Fig. 10a shows a schematic diagram of an example of mesh structure of the adjusting portion in Fig. 5;
Fig. 10b shows a schematic diagram of another example of mesh structure of the adjusting portion in Fig. 5;
Fig. 11 shows a partial schematic diagram of an adjusting portion with an annular structure in an example of Fig. 5;
Fig. 12 shows a structural schematic diagram after combination of a clamping portion and a driving portion in Fig. 5;
Fig. 13 shows a structural schematic diagram of fit of the supporting portion and a base in Fig. 5;
Fig. 14 shows a structural schematic diagram of fit of the valve clamping device in Fig. 5 and a delivery device;
Fig. 15 shows a structural schematic diagram of fit of the supporting portion of the valve clamping device in Fig. 5 and a delivery device;
Fig. 16 to Fig. 20 show schematic diagrams of a delivery process of anterogradely approaching and repairing a mitral valve via a left atrium using the valve clamping device in Fig. 5;
Fig. 21 shows a structural schematic diagram of a valve clamping device according to a second example of the first embodiment of the present disclosure;
Fig. 22 shows a structural schematic diagram of an adjusting portion in Fig. 21;
Fig. 23 shows a partial schematic diagram of a distal end of the adjusting portion in Fig. 22;
Fig. 24a shows a structural schematic diagram of a valve clamping device according to a third example of the first embodiment of the present disclosure;
Fig. 24b shows a structural schematic diagram of a preferred valve clamping device in Fig. 24a;
Fig. 25a shows a structural schematic diagram of a first curved surface side of an adjusting portion of a valve clamping device according to a fourth example of the first embodiment of the present disclosure;
Fig. 25b shows a structural schematic diagram of a second curved surface side of the adjusting portion in Fig. 25a;
Fig. 25c shows a top view of the adjusting portion in Fig. 25a;
Fig. 26a shows a structural schematic diagram of a valve clamping device according to a fifth example of the first embodiment of the present disclosure;
Fig. 26b shows a structural schematic diagram of a valve clamping device in Fig. 26a partially received in a delivery device after radial compression;

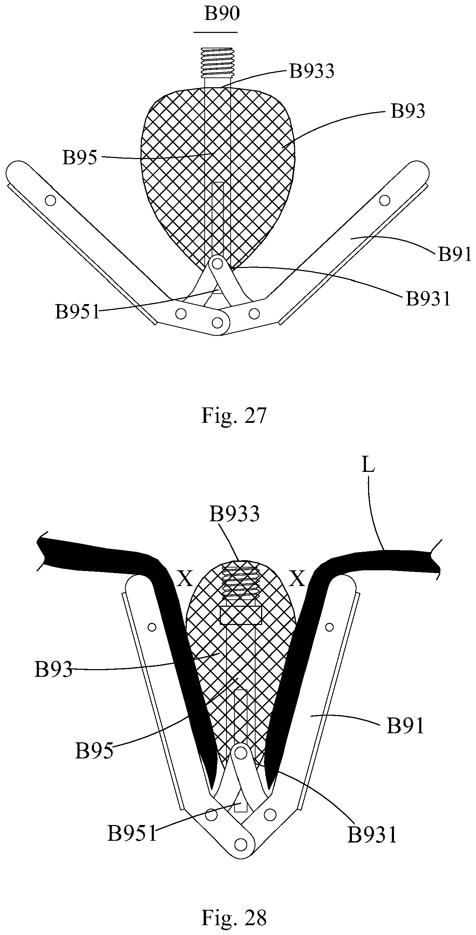

Fig. 27 shows a structural schematic diagram of a valve clamping device in the related art;
Fig. 28 shows a schematic diagram of a state when the valve clamping device shown in Fig. 27 clamps valve leaflets;
Fig. 29 shows a structural schematic diagram of a valve clamping device of a first example of a second embodiment of the present disclosure;
Fig. 30 shows a structural schematic diagram after combination of an adjusting portion and a supporting portion in Fig. 29;
Fig. 31 shows a structural schematic diagram after combination of an adjusting portion and a fixing part in Fig. 29;
Fig. 32a shows a schematic diagram of a three-dimensional structure of the adjusting portion in Fig. 29 from a visual angle;
Fig. 32b shows a schematic diagram of a three-dimensional structure of the adjusting portion in Fig. 29 from another visual angle;
Fig. 33 shows a schematic diagram of a state when the valve clamping device in Fig. 29 clamps valve leaflets;
Fig. 34 shows a structural schematic diagram after combination of the clamping portion and a driving portion in Fig. 29;
Fig. 35 shows a structural schematic diagram of the supporting portion in Fig. 29;
Fig. 36 shows a structural schematic diagram of fit of the supporting portion and a base in Fig. 29;
Fig. 37 shows a structural schematic diagram of fit of the supporting portion of the valve clamping device in Fig. 29 and a delivery device;
Fig. 38 to Fig. 42 show schematic diagrams of a process that the valve clamping device in Fig. 29 approaches anterogradely via a left atrium and performs edge-to-edge repair on a mitral valve;
Fig. 43 shows a structural schematic diagram of a valve clamping device of a second example of a second embodiment of the present disclosure;
Fig. 44 shows a structural schematic diagram after combination of an adjusting portion and a supporting portion in Fig. 43;
Fig. 45 shows a structural schematic diagram after combination of the adjusting portion and a fixing part in Fig. 43;
Fig. 46 shows a schematic diagram of a state when a valve clamping device of a third example of the second embodiment of the present disclosure clamps valve leaflets;
Fig. 47 shows a structural schematic diagram of a valve clamping device of a fourth example of the second embodiment of the present disclosure;
Fig. 48 shows a structural schematic diagram after combination of a supporting portion, a driving portion and a clamping portion in Fig. 47;
Fig. 49 shows a schematic diagram of a state when the valve clamping device in Fig. 47 clamps valve leaflets;
Fig. 50 shows a structural schematic diagram of a valve clamping device of a fifth example of the second embodiment of the present disclosure;
Fig. 51 shows a structural schematic diagram of a valve clamping device of a sixth example of the second embodiment of the present disclosure;
Fig. 52 shows a structural schematic diagram after combination of a supporting portion, a driving portion and a clamping portion in Fig. 51;
Fig. 53 shows a structural schematic diagram of a valve clamping device of a seventh example of the second embodiment of the present disclosure;
Fig. 54 shows a structural schematic diagram of fit of a valve clamping device of an eighth example of the second embodiment of the present disclosure and a delivery device;
Fig. 55 shows a schematic diagram of a state when the valve clamping device shown in Fig. 54 approaches a mitral valve via a transapical route;
Fig. 56 shows a structural schematic diagram of an adaptive valve clamping device of a first example of a third embodiment of the present disclosure;
Fig. 57 shows a structural schematic diagram of the adaptive valve clamping device of a first example of a third embodiment of the present disclosure in an opened state;
Fig. 58 shows a structural schematic diagram of the adaptive valve clamping device of a first example of a third embodiment of the present disclosure in a closed state;
Fig. 59 shows a structural schematic diagram after combination of an adjusting portion and a supporting portion in Fig. 57;
Fig. 60 shows a structural schematic diagram of the adjusting portion in Fig. 57;
Fig. 61 to Fig. 63 show different structural schematic diagrams of an adjusting portion of the first example of the third embodiment of the present disclosure;
Fig. 64 shows another structural schematic diagram of an adaptive valve clamping device of the first example of the third embodiment of the present disclosure;
Fig. 65 shows a structural schematic diagram of the supporting portion of the first example of the third embodiment of the present disclosure;
Fig. 66 shows a structural schematic diagram of a first seat body in Fig. 65;
Fig. 67 shows a structural schematic diagram of a third seat body in Fig. 65;
Fig. 68 shows a schematic diagram of a connection of a delivery device and the supporting portion of the the first example of the third embodiment of the present disclosure;
Fig. 69 shows an enlarged schematic diagram of part I in Fig. 68;
Fig. 70 to Fig. 74 show schematic diagrams of a process that the adaptive valve clamping device in Fig. 57 anterogradely approaches and repairs a mitral valve via a left atrium;
Fig. 75 and Fig. 76 show structural schematic diagrams of a supporting portion and an adjusting portion of a second example of the third embodiment of the present disclosure;
Fig. 77 shows a structural schematic diagram of the supporting portion of the second example of the third embodiment of the present disclosure;
Fig. 78 shows another structural schematic diagram of the supporting portion and the adjusting portion of the second example of the third embodiment of the present disclosure;
Fig. 79 shows an exploded schematic diagram of an adjusting portion of a third example of the third embodiment of the present disclosure; and
Fig. 80 shows an exploded schematic diagram of a supporting portion of the third example of the third embodiment of the present disclosure.

### DETAILED DESCRIPTION

The technical solutions of examples of the present disclosure are described below clearly and completely with reference to the drawings of the examples of the present disclosure. Obviously, the described examples are only part of examples of the present disclosure, rather than all of the examples. Based on the examples in the present disclosure, all other examples obtained by one skilled in the art without involving inventive work fall within the protection scope of the present disclosure.

In the description of the present disclosure, it is noted that an orientation or position relationship indicated by the terms "upper", "lower", "inner", "outer", and the like is based on an orientation or position relationship shown in the drawings, which is merely for convenience of describing the present disclosure and simplifying the description, rather than indicating or implying that the device or element referred to must have a particular orientation or be constructed and operated in a particular orientation, and thus should not be construed as limiting the disclosure. Moreover, the terms "first", "second", and the like are used for descriptive purposes only and should not be construed as indicating or implying relative importance.

In the description of the present disclosure, it is noted that in the field of interventional medical devices, a proximal end refers to an end close to an operator, and a distal end refers to an end far from the operator; an axial direction refers to a direction parallel to a line between the center of the distal end and the center of the proximal end of the medical device. The foregoing definitions are for convenience of description only and should not to be construed as limiting the present disclosure.

When an element is called "fixed to" or "provided on" another element, the element can be directly connected to another element or indirectly connected to another element through one or more connecting elements. When an element is called "connected" to another element, it can be directly connected to another element or connected to another element through one or more connecting elements.

It should be noted that in the field of interventional medical devices, a proximal end refers to an end close to an operator, and a distal end refers to an end far from the operator; an axial direction refers to a direction of the central axis of rotation of objects such as columns and tubes; a circumferential direction refers to a direction surrounding the axis of the objects such as columns and tubes (perpendicular to the axis and perpendicular to the cross-sectional radius); and a radial direction refers to a direction along the diameter or radius. It should be noted that "end" in terms of "proximal end", "distal end", "one end", "another end", "first end", "second end", "initial end", "terminal end", "both ends", "free end", "upper end" and "lower end" is not limited to an end tip, end point or end face, but also comprises a part extending a certain axial distance and/or radial distance from the end tip, end point, or end face on an element to which the end tip, end point, or end face belongs. The above definition is only for convenience of expression and cannot be construed as a limitation of the present disclosure.

The valve clamping device shown in the drawings of the specification comprises: supporting portions A110, B110 and C 110 provided along the axial direction; hollow adjusting portions A120, B120 and C120, at least part of which are provided in the adjusting portions A120, B120 and C120, an end of which is sleeved outside the supporting portions A110, B110 and C110, and the other end of which is movable with respect to the supporting portions A110, B110 and C110; clamping portions A130, B130 and C130 surrounding the outside of the adjusting portions A120, B120 and C120; and driving portions A140, B140 and C140 connected to the clamping portions to drive the clamping portions A130, B130 and C130 to be close to or away from the adjusting portions A120, B120 and C120.

In the valve clamping device provided in the present disclosure, the supporting portions A110, B110 and C110 comprise first seat bodies A112, B115 and C112 and second seat bodies A111, Bill and C114 which are connected along the axial direction. The first seat bodies A112, B115 and C112 are provided in the adjusting portions A120, B120 and C120, that is, at least part of the first seat bodies A112, B115 and C112 is provided in the adjusting portions A120, B120 and C120; and the adjusting portions A120, B120 and C120 comprise first ends A121a, B121 and C122 and second ends A121b, B123 and C124 provided opposite in the axial direction, and self-expanding bodies A120, B125 and C121 located between the first ends A121a, B121, C122 and the second ends A121b, B123 and C124. The first seat bodies A112, B115 and C112 are further away from the clamping portions A130, B130 and C130 than the second seat bodies A111, B111 and C114.

In some embodiments, the first end A121a is fixedly sleeved outside the second seat body A111, and the second end A121b hangs freely with respect to the supporting portion A110 to be movable with respect to the supporting portion. The second end A121b can also be movably sleeved outside the first seat body A112 and can move axially with respect to the supporting portion A110.

In some embodiments, the second end B123 is fixedly sleeved outside the first seat body B115. At this time, only a part of the first seat body B115 is in the adjusting portion B120, and the first end B121 is movably sleeved outside the second seat body B111 and can move along the axial direction with respect to the supporting portion B110.

In some embodiments, the first end C122 is movably sleeved outside the second seat body C114 and can move in the axial direction with respect to the supporting portion A110, and the second end C124 is sleeved outside the first seat body C112 and can move in the axial direction with respect to the supporting portion C110. The second end C124 can also hang freely with respect to the supporting portion C110 to be movable with respect to the supporting portion C110.

In some embodiments, the self-expanding bodies A120, B125 and C121 are elastomers, and the adjusting portions A120, B120 and C120 are provided with openings at the second ends A121b, B123 and C124.

In some embodiments, the elastomer is of at least one type selected from the group consisting of a mesh structure, a frame structure, a dense structure or a porous structure.

In some embodiments, the elastomer is of a mesh structure or a frame structure, and at least a part of an outer surface and/or at least a part of an inner surface of the elastomer are covered with a biocompatible film.

In some embodiments, the second ends A121b, B123 and C124 of the adjusting portions A120, B120 and C120 are heads with a central through hole, a part of the elastomers penetrates and is fixed in the head, and the central through hole forms an opening; or edges of the second ends A121b, B123 and C124 are sleeved with a hollow sleeve structure to form an opening; or the edges of the second ends A121b, B123 and C124 are enclosed to form an opening.

In some embodiments, the elastomer is of a mesh structure, the elastomer is woven from a shape memory material, and a mesh wire woven to form the mesh structure is bent back at the second ends A121b, B123 and C124 to form the openings.

In some embodiments, the elastomer is of a frame structure, the frame structure is cut from a shape memory material, the frame structure comprises a plurality of supporting rods, the adjacent supporting rods are spaced apart from or cross-linked with each other, and proximal ends of a plurality of the supporting rods converge to form an opening.

In some embodiments, the elastomer is of a dense structure made of silica gel; or the elastomer is of a porous structure made of sponge; and a second end edge of the dense structure or the porous structure forms an opening.

In some embodiments, a diameter of at least a part of the self-expanding body in a natural state gradually increases from a first end of the adjusting portion to a second end of the adjusting portion.

In some embodiments, the self-expanding bodies A120, B125 and C121 have a recessed area connected to the second end, the recessed area is recessed toward the first end, and the second end is located between two end faces of the self-expanding bodies A120, B125 and C121 along an axis.

In some embodiments, the self-expanding bodies A120, B125 and C121 comprises a first section, a second section and a third section successively connected; the first section extends from the second ends A121b, B123 and C124 of the adjusting portions A120, B120 and C120 toward a second end of the supporting portion, and the first section surrounds the outside of the second end of the supporting portion; the second section continues to extend radially outward from the first section; and the third section extends radially inward from the second section toward a first end of the supporting portion to the first ends A121a, B121 and C122 of the adjusting portion.

In some embodiments, the self-expanding bodies A120, B125 and C121 also comprise a bending section which is connected between the second ends A121b, B123 and C124 of the adjusting portions A120, B125 and C121 and the first section.

In some embodiments, a radial dimension of the second section of the self-expanding bodies A120, B125 and C121 ranges from 4 mm to 15 mm, and a radial dimension of the first ends A121a, B121 and C122 of the adjusting portions A120, B125 and C121 ranges from 1 mm to 5 mm.

In some embodiments, the self-expanding bodies A120, B125 and C121 comprises a plurality of first curved surfaces and a plurality of second curved surfaces along a circumferential direction, and the first curved surface and the second curved surface are adjacent to each other. The two oppositely disposed first curved surfaces face the clamping portions respectively, and the area of the second curved surface is less than the area of the first curved surface.

In some embodiments, the first seat bodies A112, B115 and C112 comprise an interface end connected to the second seat bodies A111, B111 and C114 and a free end provided opposite to the interface end. The free end is located in the adjusting portions A120, B120 and C120.

In some embodiments, the self-expanding bodies A120, B125 and C121 are elastomers, and the adjusting portions A120, B120 and C120 are provided with openings at the second ends A121b, B 123 and C124; and the size of the opening is less than or equal to the size of the free end.

In some embodiments, a hollow sleeve structure is sleeved outside an edge of one end of the adjusting portions A120, B120 and C120, and the sleeve structure is sleeved outside the supporting portion.

In some embodiments, the clamping portions A130, B130 and C130 comprise at least two clamp arms, the at least two clamp arms are symmetrically provided with respect to the adjusting portions A120, B120 and C120. The driving portions A140, B140 and C140 are connected to the clamp arms respectively to drive each of the clamp arms to be close to or away from the adjusting portions A120, B120 and C120.

In some embodiments, the supporting portions A110, B110 and C110 further comprises a base connected to the second seat body, each of the clamp arms is rotationally connected to the base, and there is axial spacing between the first end and a connecting position of the clamp arm and the base.

In some embodiments, the driving portions A140, B 140 and C140 comprise: a driving shaft, a connecting seat and at least two connecting rods; wherein one end of each of the connecting rods is connected to the clamping portion, and the other end of each of the connecting rods is pivotally connected to the connecting seat; and one end of the driving shaft is connected to the connecting base, and the other end of the driving shaft movably penetrates in the base.

In some embodiments, the valve clamping device further comprises a locking portion provided in the base, which restricts relative movement of the driving shaft and the base.

In some embodiments, the driving portions A140, B140 and C140 comprise: a driving shaft, an automatic closing unit and at least two connecting rods; the driving shaft movably penetrates in the supporting portion, one end of each of the connecting rods is rotationally connected to one of the clamp arms, and the other end of each of the connecting rods is rotationally connected to the driving shaft; and the automatic closing unit is connected to the clamp arm to cause the clamp arm to abut against the adjusting portion in a natural state.

In some embodiments, the driving portions A140, B 140 and C140 comprise: a driving shaft and at least two elastic driving arms. One end of the driving shaft movably penetrates in the supporting portion, one end of each of the elastic driving arms is fixedly connected to the other end of the driving shaft, and the other end of each of the elastic driving arms is connected to one of the clamp arms; and the elastic driving arms are configured to cause the clamp arm to abut against the adjusting portion in a natural state.

In some embodiments, a terminal end of the clamp arm is provided with a flanging section which is a cambered surface overturned toward the outside of the terminal end of the clamp arm, and the self-expanding body protrudes from the flanging section in the axial direction after the clamp arm abuts against the adjusting portion.

In some embodiments, the self-expanding bodies A120, B125 and C121 are provided with an adaptation section corresponding to the flanging section, and a shape of the adaptation section toward the flanging section is complementary to the cambered surface.

In some embodiments, the valve clamping device also comprises gripping portions A150, B150 and C150. The gripping portion is provided between the clamping portion and the adjusting portion and is able to be close to or away from the adjusting portion. When the gripping portion and the clamping portion are away from the adjusting portion, the gripping portion is at least partially accommodated in an inner surface of the clamping portion.

### First Embodiment

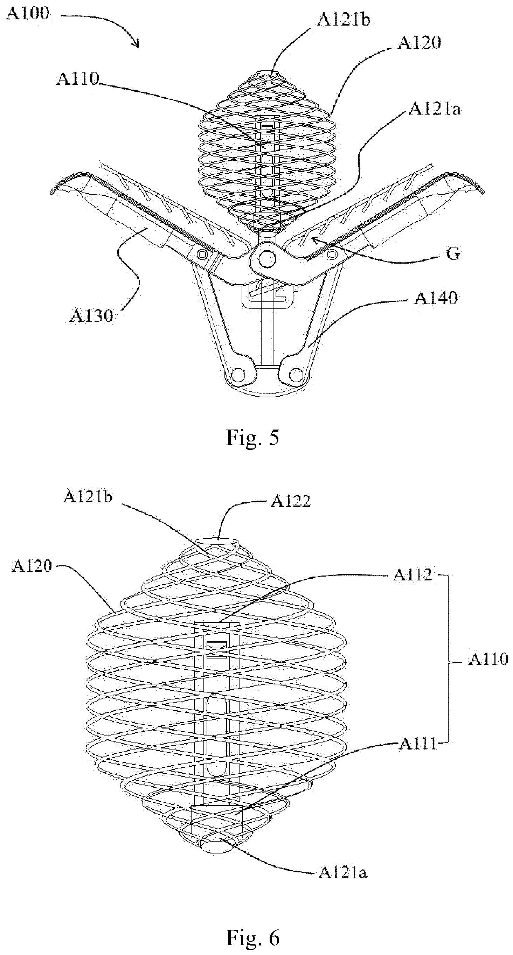

Referring to Fig. 5 to Fig. 20, a valve clamping device A100 according to the first embodiment of the present disclosure comprises: a supporting portion A110 comprising a connecting end A111 and a free end A112 disposed oppositely; a hollow adjusting portion A120, at least a part of the supporting portion A110 is disposed in the adjusting portion A120, one end A121a of the adjusting portion A120 is sleeved outside the connecting end A111 and connected to the supporting portion A110, and the other end A121b of the adjusting portion A120 hangs freely; a clamping portion A130 which is disposed around the outside of the adjusting portion A120; and a driving portion A140 which is connected to the clamping portion A130 to drive the clamping portion A130 to open or close around the adjusting portionA120.

For the valve clamping device A100, at least a part of the supporting portion A110 is provided in a hollow of the adjusting portion A120. One end A121a of the adjusting portion A120 is sleeved outside the connecting end A111 and connected to the supporting portion A110, and the other end A121b of the adjusting portion A120 hangs freely. The freely hanging end is no longer limited by the supporting portion A110 or the delivery device A200, which improves an axial deformation ability of the adjusting portion A120 enhances a bending deformation ability thereof along the axial direction. Therefore, when radially compressed into the delivery device for in vivo transportation, the valve clamping device A100 is not only easy to be compressed into the sheath, but also can adapt to blood vessels of different bending curvature when transported in blood vessels, so as to facilitate passage of the delivery device in blood vessels, thus reducing damage to vessel walls. In addition, after the valve clamping device A100 is implanted, in a process of clamping the valve leaflet and the adjusting portion A120 with the clamp arm A131, since axial deformation of the adjusting portion A120 is not limited, elastic fit between the valve leaflet and the adjusting portion A120 can be improved, and thus adaptability to physiological structures of valve leaves in different patients can be improved.

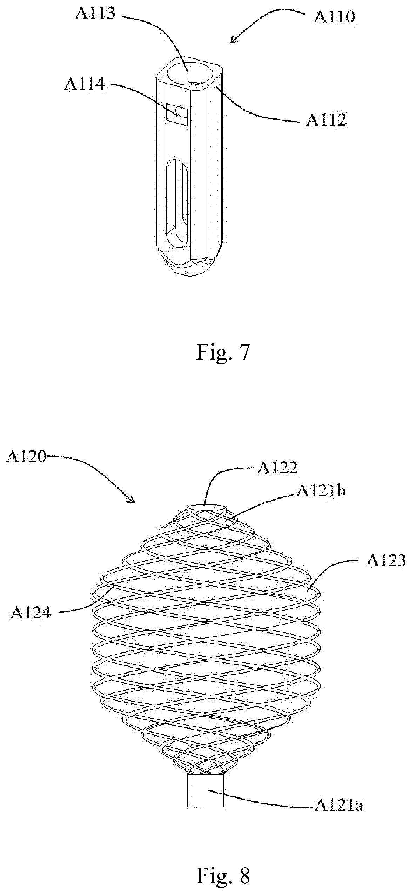

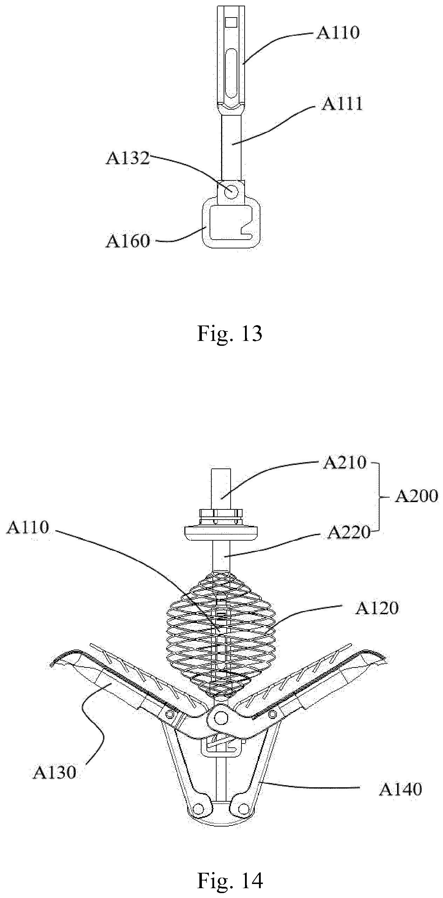

Referring to Fig. 6 and Fig. 7, the supporting portion A110 may be a circular tube body with both ends axially penetrated. The distal end of the circular tube body is the connecting end A111, and the proximal end is the free end A112. At least part of the supporting portion A110 is provided in the hollow of the adjusting portion A120. For example, the free end A112 of the supporting portion A110 is located in the adjusting portion A120, and the free end A112 is located in the adjusting portion A120 in both a delivery state and an open state, and will not be exposed from the adjusting portion A120. The supporting portion A110 is further provided with a penetrating channel A113 in an axial through hole shape to mate with the driving portion A140 and the delivery device A200. At least two locking positions A114 are provided on the tube wall of the circular tube body of the supporting portion A110 for detachable connection with the delivery device A200. For example, after a locking nose A221 (refer to Fig. 14) on the delivery device A200 snaps into the locking position A114, the delivery device A200 is snap connected to the supporting portion A110 to deliver the valve clamping device A100. When the locking nose A221 is separated from the locking position A114, the delivery device A200 is separated from the valve clamping device A100, and the valve clamping device A100 is released in the body. It should be noted that the structure of the supporting portion A110 here is only used as an example and does not limit the present disclosure. Based on teachings of the present disclosure, other structures of the supporting portion A110 adopted are within the protection scope of the present disclosure.

The free end A112 at the proximal end of the valve clamping device A100 of the present disclosure is located in the hollow adjusting portion A120 whether in the delivery state or in the released and open state. Therefore, the free end A112 will not be exposed in the delivery device A200 or in the heart, so as to avoid scouring of blood and minimize thrombosis after implantation. Moreover, after implantation, direct contact with the valve leaflet is avoided, and with long-term pulsation of the valve leaflet, wear and even perforation of the valve leaflet is avoided, and safety of implanting into patients is improved.

Referring to Fig. 6 and Fig. 8, the adjusting portion A120 comprises a deformable elastomer A123 with a hollow accommodation cavity, and at least part of the supporting portion A110 is provided in the hollow accommodation cavity. One end A121a of the elastomer A123 is connected to the supporting portion A110, and the other end A121b of the elastomer A123 has an opening A122 and hangs freely. The elastomer A123 can be deformed, so as to adapt to spacing between different valve leaflets and adjust pulling degree of the valve leaflets by the valve clamping device A100. The opening A122 of the elastomer A123 is configured to thread the distal end of the delivery device A200. It should be noted that the distal end of the delivery device A200 is connected to the proximal end (free end) of the supporting portion A110 after penetrating into the inner cavity of the elastomer A123 via the opening A122, while the opening A122 at the distal end of the elastomer A123 is not connected to the distal end of the delivery device A200 or the proximal end (free end) of the supporting portion A110, that is, the proximal end A121b of the elastomer A123 is in a freely hanging state. Thus, in the process of delivery or clamping the valve leaflets, when the clamping portion A130 is closed, the elastomer A123 in the adjusting portion A120 is not limited by the supporting portion A110 or the delivery device A200 and can be deformed in the radial and axial directions. A large deformation degree is more conducive to delivery and has a stronger adaptability to the valve leaflets; when the connection between the distal end of the delivery device A200 and the proximal end (free end) of the supporting portion A110 is released, the freely hanging end of the adjusting portion A120 has stronger deformability and stronger adaptability to the valve leaflets.

In one example, the elastomer A123 is of a mesh structure, which can be woven from a shape memory material. For example, a super elastic nickel titanium alloy material can be woven and subjected to heat setting treatment to form a compressed state and an opened state. The compressed state is maintained in the delivery device A200 and the opened state is maintained after release in the body. In one example, the elastomer A123 of the mesh structure is formed by weaving. During manufacture, 12 to 36 nickel titanium wires with a diameter of 0.02 mm to 0.15 mm are wound on a lining rod to form a tubular woven mesh with oppositely provided proximal and distal ends. At the proximal end, a plurality of mesh wires A124 are bent back to form a plurality of rings which are enclosed to form the proximal edge. A metal wire is then passed through all rings at the proximal end in turn, and then the metal wire is tightened moderately, but an opening with a moderate size is reserved. Then, a setting mold is inserted from the distal end of the self-woven mesh, and the nickel titanium wire at the distal end is wound into a bundle by a metal wire afterwards; the woven mesh and setting mold are put into an electric heating circulating air box furnace and a heat setting treatment is conducted at 450°C to 650°C (preferably 500°C) for 10 to 20 minutes; after cooling to room temperature, the metal wires at the proximal and distal ends are removed, the setting mold is taken out, and all nickel titanium wires at the distal end are inserted into the head made of stainless steel for crimping or welding to obtain the elastomer A123 of a mesh structure.

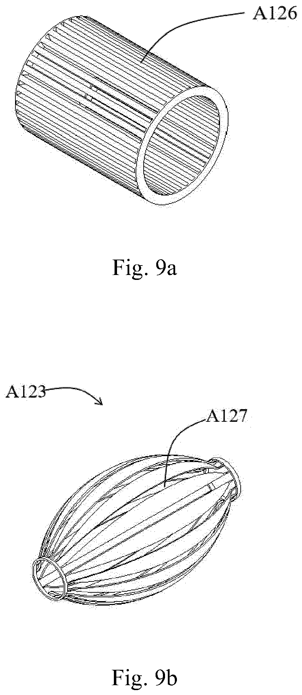

Referring to Figs. 9a and 9b, the elastomer A123 can also be of a frame structure, which is cut from hard metal or polymer materials such as stainless steel, alloy and polyvinyl chloride. An inner surface of the cut elastomer A123 is smooth and flat to avoid thrombosis in the elastomer A123 and ensure that the fixing part A220 (refer to Fig. 14) can be smoothly withdrawn from the opening A122 of the elastomer A123. The cutting method can be wire cutting or laser cutting, preferably laser cutting. During manufacture, a nickel titanium tube A126 is first cut into a required shape with a laser cutting machine. The cut nickel titanium part is pressed into the setting mold with a certain shape. Then the nickel titanium part and the setting mold are put into the electric heating circulating air box furnace for a setting heat treatment at 450°C to 650°C (preferably 500°C) for 10 to 20 minutes; after taking out and cooling to room temperature, the setting mold is removed to obtain the set elastomer A123, which is of a frame structure including a plurality of radially spaced and axially extended supporting rods A127, and proximal ends of a plurality of the supporting rods A127 converge to form a freely hanging proximal edge. Distal ends of a plurality of the supporting rods A127 converge and are welded onto the supporting portion A110. The nickel titanium tube is a pipe with certain wall thickness, and specifically, the wall thickness of the nickel titanium tube is less than 1 mm, preferably 0.02 mm to 0.15 mm, and the nickel titanium tube has certain flexibility and rigidity.

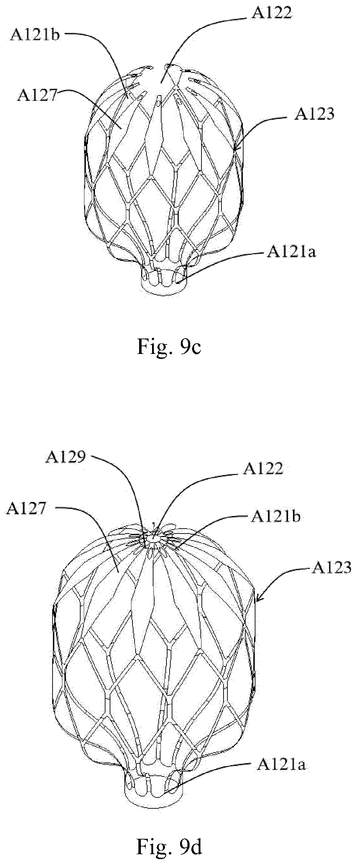

Referring to Fig. 9c, the elastomer A123 is of a frame structure formed by cutting, which is formed by fittingly connecting a plurality of the supporting rods A127. The proximal end A121b of the elastomer A123 is an opening structure, and the proximal edges of all supporting rods A127 at the proximal end A121b of the frame structure are enclosed to form the above opening A122.

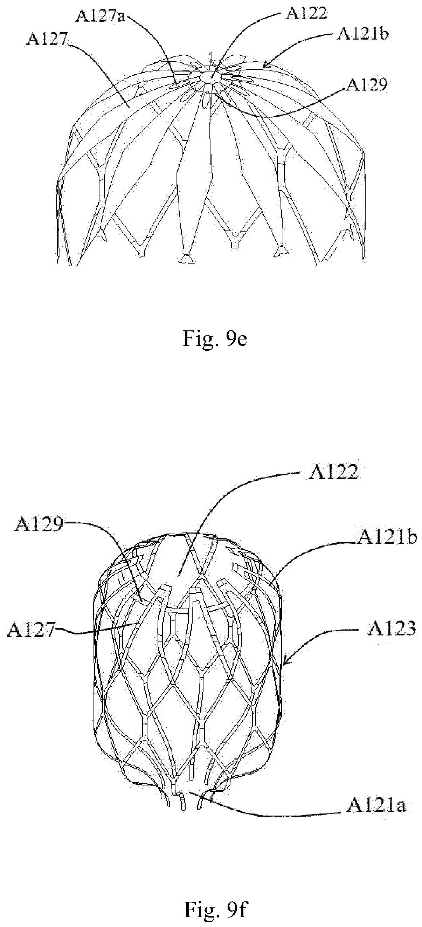

Referring to Figs. 9d and 9e, the elastomer A123 is still of a frame structure, which is formed by fittingly connecting a plurality of the supporting rods A127. The proximal end A121b of the elastomer A123 is an open structure. Each of the supporting rods A127 at the proximal end A121b may also be provided with a through hole A127a. The through holes A127a are connected in series through a flexible wire A129 to form an opening A122.

Referring to Fig. 9f, the elastomer A123 is still of a frame structure, which is formed by fittingly connecting a plurality of supporting rods A127. The proximal end A121b of the elastomer A123 is an opening structure. The adjacent supporting rods A127 of the frame structure can be cut and then cross-linked with each other. For example, the adjacent supporting rods A127 at the edge of the proximal end A121b are cut and cross-linked with each other to form a ring structure A129.

Certainly, the elastomer A123 of the adjusting portion A120 may be other elastic hollow structures. For example, the elastomer A123 may be of a dense structure or a porous structure, the dense structure is silicone colloid, the porous structure is sponge, and the proximal edge of the dense structure or porous structure forms an opening A122 for the distal end of the delivery device A200 to penetrate into the inner cavity of the elastomer A123.

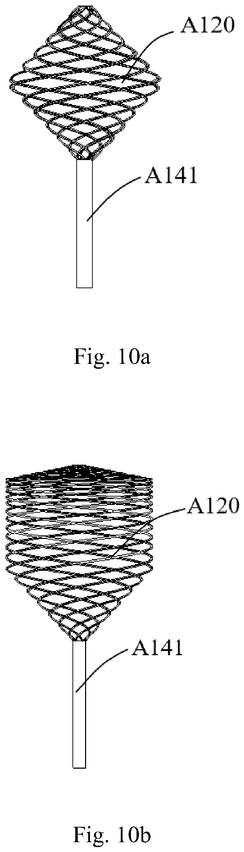

The shape of the elastomer A123 is at least one selected from the group consisting of a cylindrical shape, a conical shape, a spherical shape, a spheroidal shape, an ellipsoidal shape, a fan spherical shape and a gourd shape, or a combination of multiple shapes. In order to prevent the elastomer A123 from affecting relative opening and closing between the gripping arm A151 or the clamp arm A131 and the supporting portion A110 and to prevent a clamping effect on the valve leaflets, a diameter of a part of the elastomer A123 near the distal end should be smaller than that of other parts of the elastomer A123. For example, in the implementation mode shown in Fig. 8, the middle of the elastomer A123 is cylindrical, the two ends are cones, and cone angles of the cones at the two ends are the same. It should be noted that in other examples, the elastomer A123 can also be of any other shape, as long as the diameter of the distal end does not affect the clamping effect, for example, a spindle structure with the same cone angle at both ends shown in Fig. 10a or a structure with different cone angles at both ends shown in Fig. 10b.

The adjusting portion A120 comprises a proximal end and a distal end. In one example, a hollow sleeve structure (not shown in the figure) is sleeved on the proximal edge of the elastomer A123 to form an opening. The sleeve structure can be an existing head structure. The sleeve structure can be annular or polygonal, and can be made of hard materials such as stainless steel, so that the mesh wire of the mesh structure or the supporting rod of the frame structure can properly converge to the central axis but is not closed, so as to form an opening A122 at the center of the sleeve structure. In another example, the proximal edge of the elastomer A123 is enclosed to form the opening A122, and the size of the opening A122 is less than or equal to the size of the free end A112, so as to ensure that the free end A112 of the supporting portion A110 will not extend from the adjusting portion A120 in the compressed state and the opening state.

There are many ways for the proximal edge of the elastomer A123 to be enclosed to form the opening A122. In a specific implementation mode of the present example, the mesh structure A123 is made of nickel titanium alloy wire through weaving and heat setting treatment, and the mesh wire A124 of the mesh structure A123 is bent back at the proximal end to form the proximal edge, that is, the bent parts of all the mesh wire A124 at the proximal edge are enclosed to form the opening A122. The bent shape can be set as required, for example, bending back after bending once or bending for many times to form at least one ring, which is not described here one by one.

The proximal edge of the elastomer A123 is enclosed to form an opening A122, and the proximal head of the adjusting portion A120 of the valve clamping device A100 is canceled. When the clamping portion A130 is closed, the elastomer A123 can be deformed in the radial direction and the axial direction, and the deformation degree is large, which is more conducive to delivery; the elastomer A123 is free of the axial movement limitation imposed by the head on each mesh wire or supporting rod thereof, so the elastomer A123 can be curled or bent moderately, so as to adequately fit the valve leaflet and better adapt to the physiological structure of the valve leaflets in different patients; the risk of falling off of the proximal head part in the related art after implantation for a period of time can be avoided; and the distal end of the elastomer A123 is connected to the supporting portion A110, while the opening A122 at the proximal end is open. Therefore, the center of gravity is always located in the axial direction of the supporting portion A110 (i.e. the axial line of the elastomer A123). Therefore, the elastomer A123 has good self-centrality and is not easy to tilt.

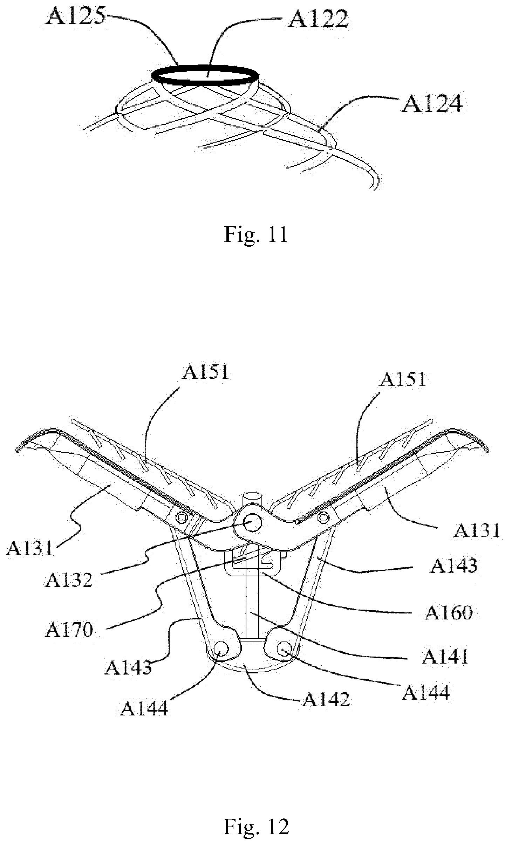

Further, referring to Fig. 11, the adjusting portion A120 can also comprise an annular structure A125 provided at the proximal edge to stabilize the shape of the opening A122. All the mesh wires A124 located at the proximal end of the mesh structure A123 are wound and connected to the annular structure A125. The annular structure A125 is made of a flexible or elastic material, and a wire diameter thereof is larger than a wire diameter of the mesh wire of the woven mesh structure A123, so as to provide a certain supporting force for the opening A122 of the mesh structure A123, but does not affect the axial deformability and bendability of the mesh structure A123.

The distal end of the adjusting portion A120 is fixedly connected to the supporting portion A110. Specifically, a hollow sleeve structure is sleeved outside the distal edge of the elastomer A123, and the sleeve structure is fixedly sleeved on the supporting portion A110. The sleeve structure of the distal end A121a (for example, the head at the distal end A121a in Fig. 8) and the supporting portion A110 are fixed together by common detachable or non-detachable connection methods such as welding, bonding, threaded connection, crimping, and bolt locking, for example, welding connection may be preferred.

Referring to Figs. 12 and 13, the clamping portion A130 comprises at least two clamp arms A131, and generally may comprise at least one group of clamp arms A131. Each group of the clamp arms A131 comprises two clamp arms A131 symmetrically provided with respect to the adjusting portion A120. The clamping portion A130 in the figures comprises a group of the clamp arms A131. It should be noted that this is only an example, one skilled in the art can select the appropriate number of the clamp arms A131 as needed, for example, two or more groups of clamp arms. The driving portion A140 is connected to each of the clamp arms A131. For example, the driving portion A140 is respectively connected to two clamp arms A131 in one group of the clamp arms A131 to drive each of the clamp arms A131 to rotate around the adjusting portion A120. It should be noted that three or more clamp arms A131 can also be set in each group as needed. For example, three valve leaflets of a tricuspid valve can be clamped by three clamp arms A131 that can be opened and closed relatively, so as to treat tricuspid regurgitation.

In the delivering state, the driving portion A140 drives the clamp arm A131 to close around the adjusting portion A120, so as to reduce the outer diameter of the valve clamping device A100 and facilitate delivering; and after the valve clamping device A100 opens in the body, the driving portion A140 drives the clamp arm A131 to clamp the valve leaflet between the clamp arm A131 and the adjusting portion A120 to realize valve clamping.

In a preferred implementation mode of this example, the valve clamping device A100 also comprises a gripping portion, which may generally comprise at least one group of the gripping arms A151, and each group of the gripping arms A151 comprises two gripping arms A151 symmetrically provided with respect to the adjusting portion A120. The gripping portion (e.g. gripping arm A151) is provided between the clamping portion A130 (e.g. clamp arm A131) and the adjusting portion A120 and may be opened or closed with respect to the adjusting portion A120. When both the gripping portion and the clamping portion A130 open, the gripping portion is at least partially accommodated in the inner surface of the clamping portion A130. Certainly, three or more gripping arms A151 may also be set in each group as required, so as to mate with the clamp arm A131 to realize the clamping function.

In the delivering state, the gripping portion is at least partially accommodated in the inner surface of the clamping portion A130, that is, the gripping arm A151 is at least partially accommodated in the inner surface of the clamp arm A131, so as to reduce the outer diameter of the valve clamping device A100 to facilitate delivering; and after the clamp arm A131 and the gripping arm A151 mate to clamp the valve leaflet, the recessed inner surface may increase the contact area between the clamp arm A131 and the valve leaflet, and cause the gripping arm A151 to press the valve leaflet into the inner surface of the clamp arm A131 to increase the clamping force on the valve leaflet.

Still referring to Figs. 12 and 13, the valve clamping device A100 also comprises a base A160 fixedly connected to the supporting portion A110, and the clamping portion A130 is rotationally connected to the base A160. Specifically, the proximal end of the base A160 is fixedly connected to the distal end A121a of the supporting portion A110. It should be noted that this part is defined as the term "base" for the convenience of description. A structure to realize the function of the base A160 may also be the distal end of the supporting portion A110, that is, an integrated structure formed with the supporting portion A110. Therefore, the definition of the term "base" should not limit the scope of the present disclosure. The clamp arms A131 in each group are connected through a pivot A132 on the base A160, so that under the drive of the driving portion A140, the clamp arms A131 cooperate with each other and may be opened and closed around the adjusting portionA120 together.

Still referring to Fig. 12 and Fig. 13, the driving portion A140 comprises a driving shaft A141, a connecting seat A142 and two connecting rods A143; wherein one end of each of the connecting rods A143 is connected to the clamping portion A130, and the other end is pivotally connected to the connecting seat A142; one end of the driving shaft A141 is connected to the connecting seat A142, and the other end movably penetrates in the base A160. Specifically, one end of each of the connecting rods A143 is connected to one clamp arm A131, and the other end is connected to the connecting seat A142 through a pivot A144, that is, each of the clamp arms A131 is rotationally connected to the distal end of the connecting seat A142 of the driving shaft A141 through the connecting rod A143 on the corresponding side. The driving shaft A141 movably passes through the base A160. When the driving shaft A141 slides axially with respect to the base A160, the connecting rod A143 rotates and drives the clamp arm A131 to open and close with respect to the base A160.

Specifically, the driving portion A140 comprises at least one group of connecting rods A143. The number setting of the connecting rods A143 corresponds to the clamp arms A131 one to one. For example, two clamp arms A131 are used in the figure, and two cooperating connecting rods A143 are provided correspondingly. The distal end of the connecting rod A143 is rotationally connected to the connecting seat A142 at the distal end of the driving shaft A141 by a rotating pin or bolt A144 and the like. When the driving shaft A141 axially slides toward the distal end with respect to the base A160, the connecting rod A143 is driven to move. Under the pull of the connecting rod A143, the clamp arm A131 rotates around a pin hole A144 and opens with respect to the base A160. When the driving shaft A141 slides axially toward the proximal end with respect to the base A160, the connecting rod A143 pulls the clamp arm A131 to rotate around the pin hole A144 and close with respect to the base A160.

The connecting seat A142 is fixedly provided at the distal end of the driving shaft A141 by means such as welding, and the connecting seat A142 is provided with a pair of pins. The pin hole is used to hinge the connecting rod A143 through the pin A144, and the other end of the connecting rod A143 is connected to the clamp arm A131, so as to realize the opening and closing of the clamp arm A131 with respect to the base A160. The shape of the connecting seat A142 is any structure such as a hemisphere, a spherical crown or a warhead shape, so that the valve clamping device A100 is pushed more easily in the body. The driving shaft A141 and the connecting seat A142 may be integrated or non-integrated. In order to ensure safety after implantation, the driving shaft A141 and the connecting seat A142 are made of biocompatible materials such as polyester, silicone, stainless steel, cobalt alloy, cobalt chromium alloy and titanium alloy, preferably stainless steel or cobalt chromium alloy with high hardness.

Preferably, as shown in Fig. 12, the valve clamping device A100 also comprises a locking portion A170 provided in the base A160, which restricts the relative movement of the driving shaft A141 and the base A160. In the delivering state, the locking portion A170 restricts the relative movement of the driving shaft A141 and the base A160, so as to ensure that the clamping portion A130 is always closed with respect to the adjusting portion A120 and the supporting portion A110 and to avoid accidental opening of the clamping portion A130; and after the valve clamping device A100 reaches the vicinity of the mitral valve, the restriction of the locking portion A170 on the driving shaft A141 is unlocked, and the clamping portion A130 can be driven by the driving portion A140 to open with respect to the adjusting portion A120 and the supporting portion A110 and support the valve leaflet. Any suitable locking portion in the related art can be used, which is not described here.

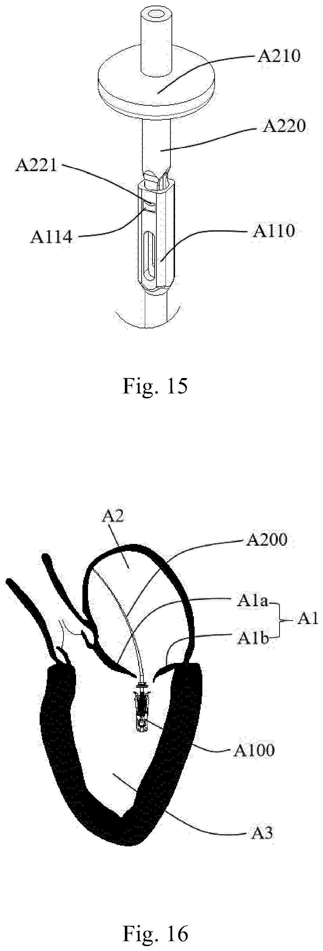

Referring to Fig. 14 and Fig. 15, the valve clamping system of the present example comprises the valve clamping device A100 and a delivery device A200. The delivery device A200 comprises a pushing shaft A210 with a certain axial length and a mandrel (not shown in the figure) movably penetrating the pushing shaft A210. The pushing shaft A210 is detachably connected to the supporting portion A110, and the mandrel is connected to the driving portion A140 to drive the clamping portion A130 to open and close with respect to the supporting portion A110. In the present example, the proximal end of the driving shaft A141 is provided with an external thread, and the mandrel is threaded with the driving shaft A141, so as to control the axial movement of the driving shaft A141 through the mandrel outside the patient. It should be noted that only part of the structure of the delivery device is described here, and any suitable structure in the related art can be adopted for other parts, which is be described here.

Specifically, the proximal outer wall of the supporting portion A110 is symmetrically provided with at least one locking position A114 connected to the cavity of the supporting portion A110. The distal end of the pushing shaft A210 is provided with a fixing part A220 comprising two branches, and the terminal end of each branch is a protruded locking nose A221. In the natural state, the two branches point to the central axis of the fixing part A220. During assembly, the fixing part A220 is inserted into the supporting portion A110, and the mandrel of the delivery device A200 is inserted into the pushing shaft A210 until the mandrel is inserted into the fixing part A220, and the two branches of the fixing part A220 are pushed up outward. The locking nose A221 at the terminal end of the branch is locked into the two locking positions A114 of the supporting portion A110, so as to connect the supporting portion A110 to the fixing part A220, that is, to connect the valve clamping device A100 to the delivery device A200. When the mandrel is withdrawn from the fixing part A220 and the pushing shaft A210, the two branches restore the inward natural state. The locking nose A221 is separated from the locking position A114 of the supporting portion A110, so that the connection between the valve clamping device A100 and the delivery device A200 is released. The fixing part A220 is made of materials with certain hardness and elasticity such as nickel and titanium. The pushing shaft A210 may be a multi-layer pipe. The mandrel is made of stainless steel.

The interior of the supporting portion A110 is provided with a through hole as the penetrating channel A113 of the driving shaft A141, and the driving shaft A141 slidingly penetrates the penetrating channel A113 of the supporting portion A110 along the axial direction. The proximal end of the driving shaft A141 is provided with an external thread for connection with the mandrel of the delivery device A200, so as to control the axial movement of the driving shaft A141 through the mandrel. After the clamping portion A130 and the gripping portion A150 cooperate and clamp the valve tissue, the mandrel drives the driving shaft A141 to move toward the proximal end in the axial direction, and the driving shaft A141 drives the connecting rod A143 to rotate. The connecting rod A143 drives the clamp arm A131 to close with respect to the supporting portion A110 until the clamp arm A131 is completely closed with respect to the supporting portion A110, so that the valve clamping device A100 is in the folded and closed state and falls below the valve. After that, the connection between the mandrel and the driving shaft A141 may be released, the mandrel is withdrawn from the fixing part A220, and the locking nose A221 is separated from the locking position A114 of the supporting portion A110, so as to release the valve clamping device A100 from the delivery device A200. In the process of release, since the connecting position (i.e., disengagement position) between the valve clamping device A100 and the delivery device A200 is located in the adjusting portion A120 of the valve clamping device A100, and the proximal end of the adjusting portion A120 is provided with an open opening A122, no part will hook the locking nose A221 at the terminal end of the branch of the fixing part A220, which facilitates the release of the valve clamping device A100. In addition, the release position is provided inside the adjusting portion A120 and is not directly scoured by blood, which can avoid failure of the mechanism at the release position and reduce the risk of thrombosis.

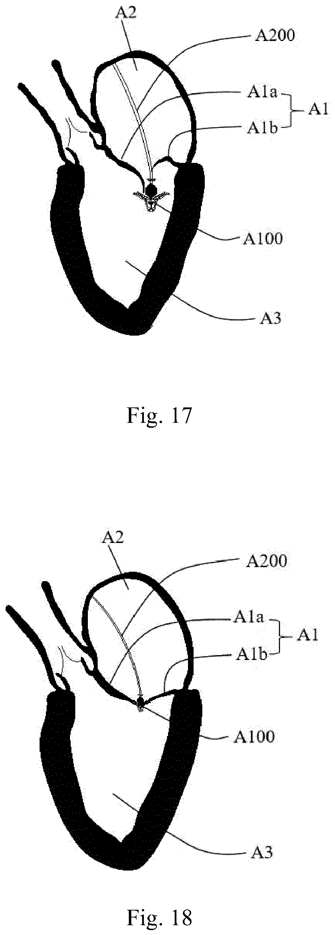

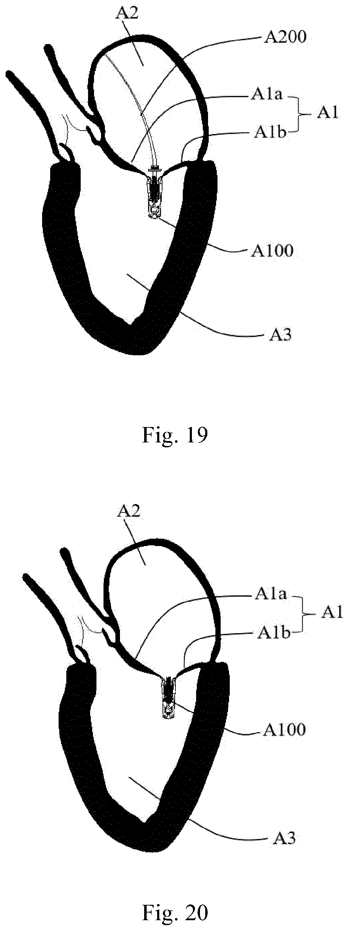

Referring to Fig. 16 to Fig. 20, a use process of the valve clamping device A100 of the present disclosure is described, taking anterograde approach and repair of the mitral valve via the left atrium as an example.
Step 1: pushing the driving shaft A141 and the valve clamping device A100 connected thereto from the left atrium A2 to the left ventricle A3 via the mitral valve A1 with a guiding device (not shown in the figure) such as a bendable sheath, as shown in Fig. 16;
Step 2: adjusting the valve clamping device A100 to approach the anterior leaflet A1a and the posterior leaflet A1b of the mitral valve A1;
Step 3: unlocking a locking portion in the base A160, pulling the mandrel and the driving shaft A141 to the proximal end, driving the clamp arm A131 to open with respect to the supporting portion A110, and adjusting the direction of the clamp arm A131, at which time a relative position between the clamp arm A131 and the anterior and posterior leaflets A1a, A1b of the mitral valve A1 can be observed by an X-ray device, and the clamp arm A131 is made perpendicular to a coapting line of the mitral valve A1, as shown in Fig. 17;
Step 4: withdrawing the entire valve clamping device A100 to the proximal end to let the clamp arm A131 hold the valve leaflet A1 on the left ventricle A3 side, and releasing the gripping arms A151 on both sides, wherein the gripping arm A151 on each side presses the valve leaflet A1 on the atrium side and cooperates with the clamp arm A131 on the side to fix the valve leaflet A1, realizing complete clamping of the valve leaflet A1, as shown in Fig 18;
Step 5: pushing the mandrel and the driving shaft A141 to the distal end when the anterior mitral leaflet A1a and the posterior mitral leaflet A1b of the mitral valve A1 are clamped between the pair of clamp arms A131 and the gripping arms A151 respectively, thereby driving the clamp arms A131 to close, as shown in Fig. 19;
Step 6: releasing the threaded connection between the mandrel and the driving shaft A141 and withdrawing the mandrel, so that the two branches of the fixing part A220 restores a state of converging to the central axis, the locking nose A221 is disengaged from the locking position A114 of the supporting portion A110, and the valve clamping device A100 is disconnected from the delivery device A200, and then withdrawing the delivery device A200 from the body, resulting in an implanted state as shown in Fig. 20, at which time the valve clamping device A100 pulls the anterior mitral leaflet A1a and the posterior mitral leaflet A1b of the mitral valve A1 toward each other to obtain a double-orifice mitral valve and complete edge-to-edge repair of the mitral valve.

After implantation of the valve clamping device A100, the elastic adjusting portion A120 is filled between the anterior mitral leaflet A1a and the posterior mitral leaflet A1b of the clamped mitral valve A1 and abuts on the clamp arm A131, the elastomer A123 of the adjustment portion A120 (e.g., a mesh structure or a porous structure) has a cushioning effect on the pulsing valve leaflet A1, so that a pulling degree of the valve leaflet A1 by the valve clamping device A100 is adjustable to avoid damage to the valve leaflet A1; the elastomer A123 may be pressed and deformed following the pulsation of the valve leaflet A1, the resulting elastic force pushes a part of the valve leaflet A1 close to the elastomer A123 in a direction away from the base A160, at which time a clamping angle between the anterior mitral leaflet and the posterior mitral leaflet of the mitral valve is less than the opening angle between the clamp arms A131 since the structure of the opening A122 of the adjusting portion A120 makes the axial movement of the elastomer A123 toward the proximal end no longer restricted, which can reduce pulling of the valve leaflet A1 by the valve clamping device A100, so that the pulling degree of the valve leaflet A1 by the valve clamping device A100 always maintains within a reasonable range; the elastomer A123 can cushion direct scouring of blood flow to the inside of the valve clamping device A100, prevent the valve clamping device A100 from falling off under the continuous scouring of blood, and also prevent blood from being deposited at a dead angle (G in Fig. 5) between the clamping portions A130 of the valve clamping device A100 to form thrombus; when the elastomer A123 is under pressure of the valve, a degree of deformation is generated, and the deformation degree increases with the increase of pressure so as to avoid that the pressing force of the clamp arm A131 on the elastomer A123 in turn acts on the clamp arm A131 after gripping the valve leaflet A1, thereby ensuring that the gripping effect of the valve clamping device A100 on the valve leaflet A1 after release remains consistent with that before release.

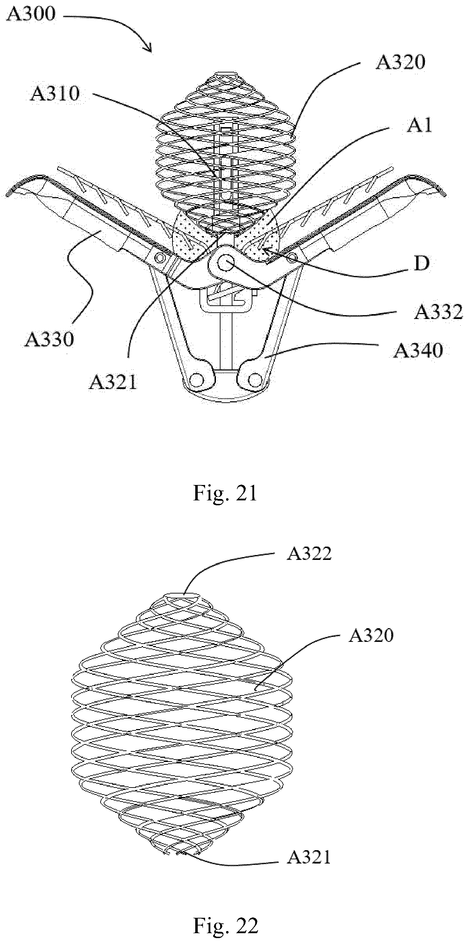

Referring to Fig. 21 to Fig. 23, compared with the valve clamping device of the first example, all mesh wires at the distal end A321 of the mesh structure of the adjusting portion A320 of the valve clamping device A300 according to the second example of the present disclosure A324 are fixedly sleeved on the supporting portion A310. That is, both ends A322 and A321 of the adjusting portion A320 are open structures without a head. During assembly of the adjusting portion A320, the mesh wires A324 at the distal end A321 of the adjusting portion A322 are directly fixed on the supporting portion A310 through common detachable or non-detachable connection means such as welding, bonding and crimping, preferably welding connection in the present example.

Since the opening or closing of the clamping portion A330 (for example, clamp arm) rotates around the pivot A332 (for example, pin) close to the distal side of the adjusting portion A320, when the clamping portion A330 is closed, the closer to the pivot A332, the smaller the space is. When the valve leaflet is clamped by the clamping portion A330, part of the valve leaflet A1 fill in and accumulate at space D, which not only affects the closing of the valve clamping device, but also causes serious damage to the valve A1 when the valve clamping device is forcibly closed due to the failure to find the filling of the valve leaflet A1 at D in time. In the present example, since the distal end A321 of the adjusting portion A320 is an open structure without a head, the adjusting portion A320 can better conform to the deformation of the valve leaflet A1 in the process of closing of the valve clamping device A300. Meanwhile, the adjusting portion A320 reduces a hard head and thus increases the space there, which is conducive to better closure of the whole valve clamping device A300 after grasping the valve leaflet A1.

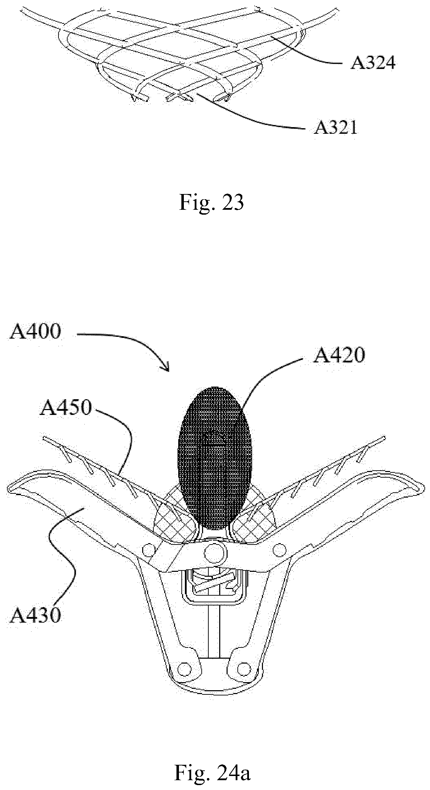

Referring to Fig. 24a, compared with the valve clamping device of the first example, at least part of the outer surface of the mesh structure of the adjusting portion A420 of the valve clamping device A400 according to the third example of the present disclosure is coated with a film. In addition, a film can be coated to at least part of the outer surfaces of the clamping portion A430 and the gripping portion A450. The film may be of a woven mesh structure and is provided with a plurality of mesh holes. The adjusting portion A420, the clamping portion A430 and the gripping portion A450 after coating have higher biocompatibility and enhanced frictional force, and thus have a better clamping effect on the valve leaflets.

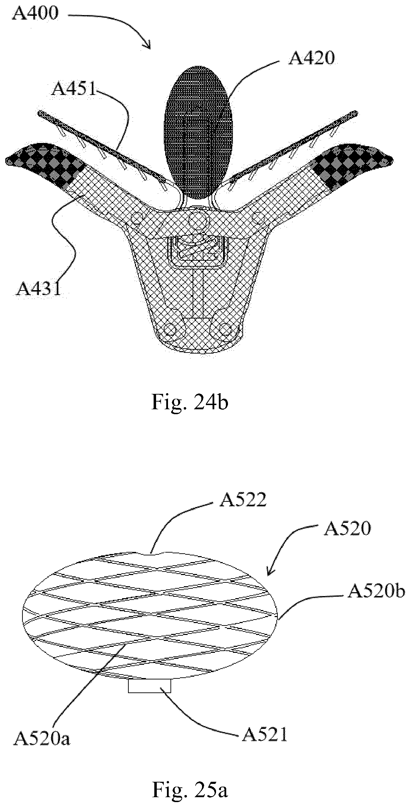

For example, referring to Fig. 24b, the outsides of the gripping arm A451 and the clamp arm A431 are covered with a first film and a second film respectively, and the outside of the elastomer of the adjusting portion A420 is covered with a third film. The relationship between the aperture ratios of the three is: the aperture ratio of the third film < the aperture ratio of the first film < the aperture ratio of the second film. The aperture ratio refers to the percentage of the aperture area in the whole film area. The aperture ratio of the second film is large, so that the second film has better elasticity and elongation rate than the first film. When the clamp arm covered with the second film is opened and closed with respect to the fixed base, the second film can produce corresponding elastic deformation with the opening and closing of the clamp arm, and the second film always adheres to the clamp arm. The aperture ratio of the third film is the smallest, so that the elastomer can substantially hinder the passage of blood flow.

The mesh holes on the first film and the second film can let the blood pass through and prevent the thrombus from passing through, and the mesh hole on the third film can neither let the blood nor the thrombus pass through. The first film can allow blood to permeate through without affecting the normal flow of blood from the left atrium to the left ventricle, avoiding blood retention in the left atrium, thereby reducing the damage of blood pressure to the left atrium cavity; and the first film can also increase the contact area between the gripping arm and blood to cushion the inflowing blood flow, so as to avoid the falling off resulted from the deformation of the gripping arm caused by the inflowing blood flow impacting the valve clamping device as much as possible. The second film can make the blood flow circulate normally between the left atrium and the left ventricle, so as to reduce the blood pressure difference between the left atrium and the left ventricle; and the second film can also block a minute amount of thrombus entering the valve clamping device through the first film and leave the thrombus in the valve clamping device, so as to prevent thrombus from entering the left ventricle and entering the human blood circulation to induce stroke.

The elastomer with the third film can not only increase the biocompatibility, avoid tissue allergy and inflammatory reaction, and improve product safety; but also form an artificial barrier on the atrial side of the valve leaflet, block the thrombus in the blood, close the opening of the whole valve clamping device toward the atrial side, and avoid the repeated scouring of blood at the internal dead corner of the valve clamping device to form thrombosis, so as to avoid thrombosis.

The first film, the second film and the third film may be made of polyethylene terephthalate, polypropylene, polytetrafluoroethylene, polyurethane and other polymer materials. The materials of the three may be the same or different. In the present example, the three are made of PET.

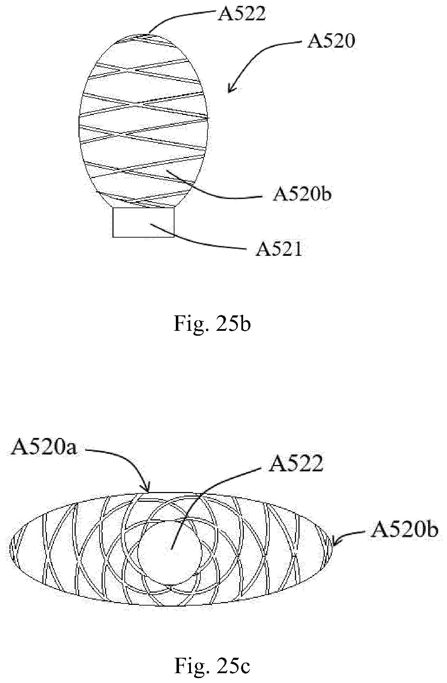

Referring to Fig. 25a to Fig. 25c, compared with the valve clamping device of the first example, the adjusting portion A520 of the valve clamping device according to the fourth example of the present disclosure has a freely hanging end and a distal head A521. The freely hanging end may have an opening A522, the adjusting portion A520 comprises a plurality of first curved surfaces A520a and a plurality of second curved surfaces A520b adjacent to and smoothly connected to each other. That is, the first curved surface A520a is only adjacent to the second curved surface A520b, and the second curved surface A520b is only adjacent to the first curved surface A520a. The two opposite first curved surfaces A520a face a clamp arm respectively, and the area of the second curved surface A520b is less than that of the first curved surface A520a.

In the present example, the first curved surface A520a with relatively small area faces the clamp arm, and the second curved surface A520b with relatively large area is smoothly connected between the two first curved surfaces A520a. With the closing process of the valve clamping device, the first curved surface A520a of the adjusting portion is pressed by the clamp arm and the valve leaflet, and the adjusting portion extends along the direction of the second curved surface A520b and gradually fits in the valve leaflet, so as to better adapt to the shape of the valve leaflet and increase the contact area between the first curved surface A520a and the valve leaflet, thereby reducing the gap between the valve clamping device and the valve leaflet and slowing down the blood flow and hinder the scouring of the blood flow to the valve clamping device. Preferably, the curvature of the first curved surface A520a may also be greater than that of the second curved surface A520b, so that the adjusting portion presents a flat ellipsoid shape to avoid affecting the closing of the clamp arm. Further, in the present implementation mode, when the clamp arm is closed, the first curved surface A520a of the adjusting portion is pressed by the clamp arm and the valve leaflet, and the adjusting portion extends along the direction of the second curved surface A520b. Since the first end of the adjusting portion is open, it will not hook the distal end of the delivery system, thereby ensuring that the valve clamping device is separated at the connecting position with the delivery device in the case of arbitrary deformation of the adjusting portion.

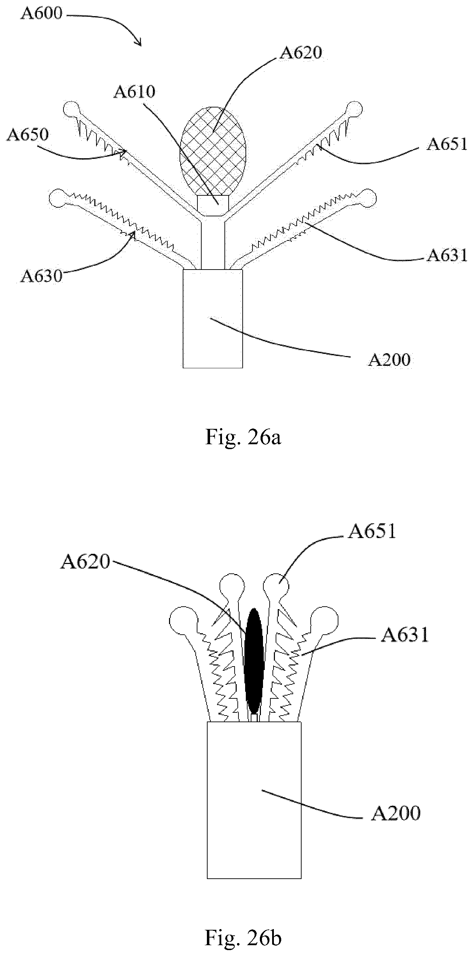

Referring to Figs. 26a and 26b, compared with the valve clamping device of the first example, the structure of the adjusting portion A620 of the valve clamping device A600 according to the fifth example of the present disclosure is the same as that of the adjusting portion A120 of the first example, except that the clamping portion A630 and the gripping portion A650 cooperate differently to grip the valve leaflet. In the fifth example, the clamping portion A630 comprises a group of clamp arms A631 that can be opened or closed with respect to the supporting portion A610 and the adjusting portion A620, the gripping portion A650 comprises a pair of gripping arms A651, and the gripping portion A650 is located between the clamping portion A630 and the adjusting portion A620.

During delivery, the clamping portion A630, the gripping portion A650 and the adjusting portion A620 are accommodated in the distal end of the delivery device A200, the delivery device A200 is sent to the left ventricle via a transapical route and then reaches the left atrium across the mitral valve orifice; the delivery device A200 is withdrawn, so that the adjusting portion A620 and the gripping portion A650 gradually extend from the delivery device A200 and open within the left atrium; the delivery device A200 continues to be withdrawn until the clamping portion A630 also extends from the delivery device A200 and opens in the left ventricle; then the clamping portion A630 is pushed to the distal end by the driving portion, the anterior and posterior leaflets of the mitral valve are respectively borne on the inner surfaces of the two clamp arms A631 of the clamping portion A630 respectively, the gripping portion A650 and the adjusting portion A620 are withdrawn toward the proximal end, that is, the gripping portion A650 is driven to move in the direction of the clamping portion A630, thereby capturing the valve leaflet between the gripping portion A650 and the clamping portion A630, then the clamping portion A630 is driven to close with respect to the adjusting portion A620 and the supporting portion A610, thereby fixing the anterior mitral leaflet and the posterior mitral leaflet respectively between one clamp arm A631 and one gripping arm A651 disposed correspondingly to the clamp arm A631, then the delivery device A200 is pushed to the distal end until the valve clamping device A600 gradually converges and is closed; and the valve clamping device is disconnected from the delivery device A200, thereby implanting the valve clamping device onto the mitral valve and drawing the anterior mitral leaflet and the posterior mitral leaflet of the mitral valve toward each other to form a double orifice structure.

### Second Embodiment

### First Example

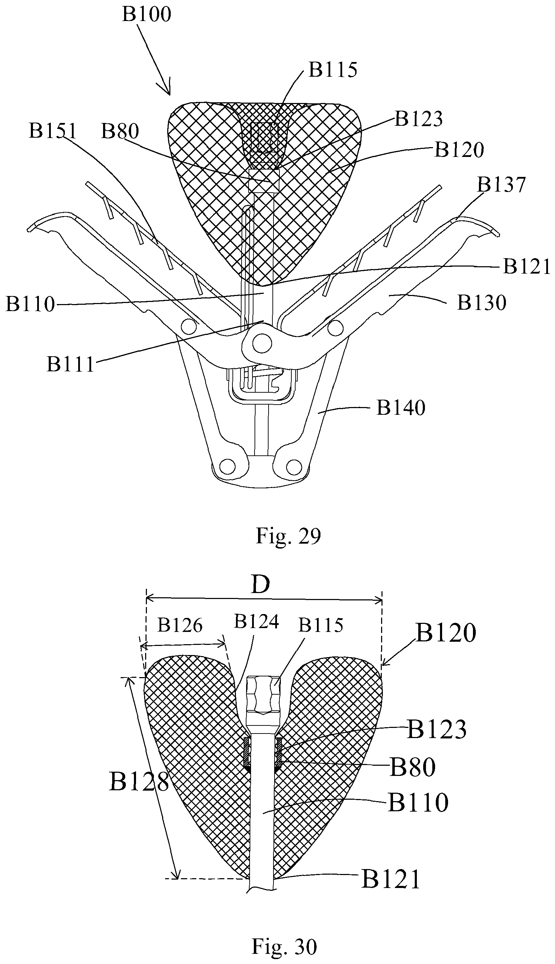

Referring to Fig. 29 to Fig. 33, an adequately fitted valve clamping device B 100 according to a first example of the present disclosure comprises:
a supporting portion B110 with a certain axial length and comprising a first end B111 and a second end B115 provided oppositely;
an adjusting portion B120 comprising a first end B121 and a second end B123 oppositely provided, and a self-expanding body B125 located between the first end B121 and the second end B123; and
a clamping portion B130, which is provided on the outside of the adjusting portion B120 and can be opened or closed with respect to the adjusting portion B120.

The first end B121 of the adjusting portion B120 of the valve clamping device B100 is movably sleeved outside the supporting portion B110, and the second end B123 of the adjusting portion B120 is sleeved outside the supporting portion B110 and is fixedly connected to the supporting portion B110. The first end B121 of the adjusting portion B120 is located between the first end Bill of the supporting portion B110 and the second end B123 of the adjusting portion B120, or the first end B121 of the adjusting portion B120 is closer to the first end B111 of the supporting portion B110 than the second end B123 of the adjusting portion B120.

Referring to Fig. 29 and Fig. 38 to Fig. 42, the valve clamping device B100 of the first example is provided with a connecting portion (not marked) detachably connected (such as threaded connection or snap connection) to the delivery device B200 on the second end B 115 of the supporting portion B 110. The delivery device B200 pushes the valve clamping device B100 into the heart via a catheter, and the second end B115 of the supporting portion B110 is a proximal end of the valve clamping device B100, the first end B111 of the supporting portion B110 is a distal end thereof, the second end B123 of the adjusting portion B120 is a proximal end thereof, and the first end B121 of the adjusting portion B120 is a distal end thereof. It should be noted that in other examples, the valve clamping device may intervene the heart via a transapical route, so the second end of the supporting portion is a distal end of the valve clamping device, the first end of the supporting portion is a proximal end thereof, the second end of the adjusting portion is a distal end thereof, and the first end of the adjusting portion is a proximal end thereof.

Please also refer to Fig. 29 to Fig. 33. The valve clamping device B100 mainly comprises two states: one is the opened state, and the other is the closed state. In the opened state, the adjusting portion B120 is in a natural state without external force, and the diameter of the self-expanding body B125 of the adjusting portion B120 in the natural state gradually increases from the first end B121 of the adjusting portion B120 to the second end B123 of the adjusting portion B120, that is, the overall shape of the adjusting portion B120 is approximately an inverted cone shape, the first end B121 of the adjusting portion B120 substantially forms the apex of the inverted cone shape, and the part near the second end B123 of the adjusting portion B120 forms the bottom of the inverted cone shape. In the first example, one end of the clamping portion B130 is rotationally connected to the first end Bill of the supporting portion B110, so that the clamping portion B130 can open or close around the adjusting portion B120 with the rotating connection part between the clamping portion B130 and the first end B111 of the supporting portion B110 as a center. There is axial spacing between the first end B121 of the adjusting portion B120 and the first end B111 of the supporting portion B110. When the adjusting portion B120 is subjected to radial pressing, the first end B121 of the adjusting portion B120 can move axially toward the first end Bill of the supporting portion B110, and when the clamping portion B 130 centers on the rotating connection part between the clamping portion B130 and the first end B111 of the supporting portion B110 and is closed around the adjusting portion B120 to clamp the valve leaflet B1, the adjusting portion B120 is subjected to radial retraction and axial elongation due to the pressing of the clamping portion B130. It should be noted that in other examples, one end of the clamping portion B130 can also be rotationally connected to other parts, as long as the part is close to the first end B111 of the supporting portion B110, so that the clamping portion B130 is provided on the outside of the supporting portion B110 and can be opened or closed with respect to the adjusting portion B120 to clamp the valve leaflet.

It should be noted that in the closed state, since the second end B123 of the adjusting portion B120 is fixedly connected to the supporting portion B110, the first end B121 of the adjusting portion B120 is movably sleeved on the supporting portion B110, the axial movement of the second end B123 of the adjusting portion B120 and its adjacent part of the self-expanding body B125 is limited. The self-expanding body B125 will extend axially toward the first end B121 of the adjusting portion B120, at which time the self-expanding body B 125 will retract radially under the pressing of the clamping portion B130. With the extension of the first end B121 of the adjusting portion B120 toward the valve edge of the valve leaflet B1, the overall shape of the adjusting portion B120 can be complementary to the shape of the opening of the clamping portion B130, and still presents an inverted cone shape. Part of the self-expanding body B125 near the second end B123 of the adjusting portion B120 is located at the conical bottom of the inverted cone shape, that is, the self-expanding body B125 gradually converges toward the central axis of the adjusting portion B120 to the first end B121 of the adjusting portion B120, and part of the self-expanding body B125 near the second end B123 of the adjusting portion B120 can fit closely in the valve leaflet B1 without a radial gap, which can increase the fitting area between the valve leaflet B1 and the adjusting portion B120, improve the elastic fit between the valve leaflet B1 and the adjusting portion B120, and make the valve leaflet B1 and the adjusting portion B120 adequately fit as compared with the related art. Accordingly, the second end B123 of the adjusting portion B120 and its adjacent part of the self-expanding body B125 can provide a large radial support force for the valve leaflet B1, and thus improve the clamping force to firmly clamp the valve leaflet B1, reduce the risk of the valve leaflet B1 falling off between the clamping portion B130 and the adjusting portion B120, and improve the implantation stability of the valve clamping device B100; meanwhile, the close fit between the part of the self-expanding body B125 near the second end B123 of the adjusting portion B120 and the valve leaflet B1 can block the blood regurgitating from the clamping gap and optimize the regurgitation treatment effect. In addition, the adjusting portion B120 can adaptively adjust its shape depending on the clamping degree of the clamping portion B130, which can ensure that the adjusting portion B120 can adequately fit in the valve leaflet under any clamping degree.

It should be noted that in the closed state, the first end B121 of the adjusting portion B120 can extend toward the valve edge to be closer to the valve edge position as compared with the related art, which further increases the fitting area between the valve leaflet B1 and the adjusting portion B120 and further improves the elastic fit between the valve leaflet B1 and the adjusting portion B120.

In addition, since the first end B121 of the adjusting portion B120 is movably sleeved on the supporting portion B110, the adjusting portion B120 has a strong axial deformation ability. When radially compressed into the delivery sheath for in vivo delivery, the valve clamping device B100 is easy to be compressed into the sheath.

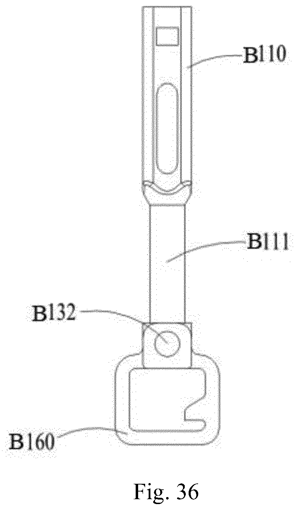

Specifically, referring to Fig. 29 and Fig. 34 to Fig. 37, the supporting portion B110 can be a circular tube body, a square column tube body or an oblate tube body with both ends axially penetrated. The present example adopts a circular tube body. As mentioned above, the distal end of the circular tube body is the first end Bill, and the proximal end is the second end B115. The second end B115 of the supporting portion B110 can be surrounded and shielded by the part of the self-expanding body B125 near the second end B123 of the adjusting portion B120 in the closed state and the opened state, so as not to be exposed from the adjusting portion B120, thereby preventing the second end B115 from directly contacting the valve leaflet, preventing the second end B115 from wearing the valve leaflet with the long-term pulsation of the valve leaflet, and improving the implantation safety.

The supporting portion B110 is also provided with an axial through-hole shaped penetrating channel B113 to cooperate with the driving portion B140 and the delivery device B200. At least two locking positions B114 are provided on the tube wall of the circular tube body of the supporting portion B110 for detachable connection with the delivery device B200. For example, a locking nose B221 is provided on the delivery device B200. After the locking nose B221 is locked into the locking position B114, the delivery device B200 is lockingly connected to the supporting portion B110 to deliver the valve clamping device B100. When the locking nose B221 is separated from the locking position B114, the delivery device B200 is separated from the valve clamping device B100. It should be noted that the structure of the supporting portion B110 is only an example but not a limitation of the present disclosure. Based on the teachings of the present disclosure, other structures of the supporting portion B110 adopted by one skilled in the art are within the protection scope of the present disclosure.

Referring to Fig. 29 to Fig. 33, the self-expanding body B125 of the adjusting portion B120 is of a mesh structure, preferably a mesh structure formed by weaving wires or cutting a pipe with a shape memory function, such as hyper-elastic materials such as nickel iron alloy wires. Under the same closed degree of the clamping portion B130, the adjusting portion B120 can adapt to the spacing between different valve leaflets and produce adaptive deformation, so as to adjust the pulling degree of the valve leaflets by the valve clamping device B100. The adjusting portion B120 has a hollow accommodation cavity (not marked), and the part of the supporting portion B110 between the second end B115 and the first end B111 is provided in the hollow accommodation cavity.

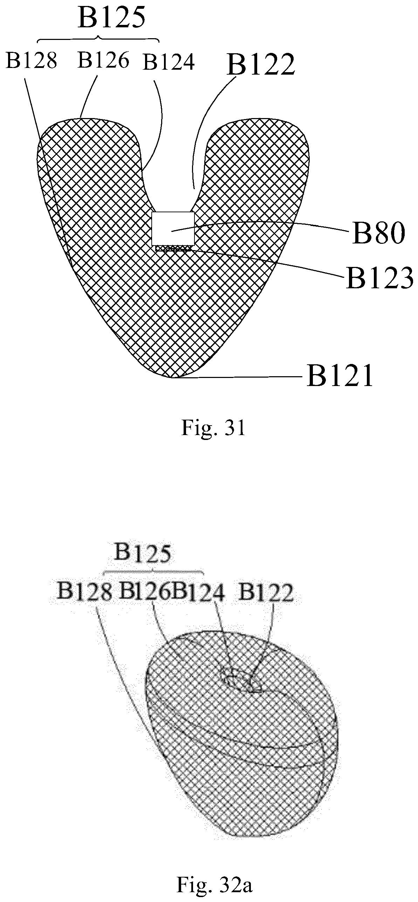

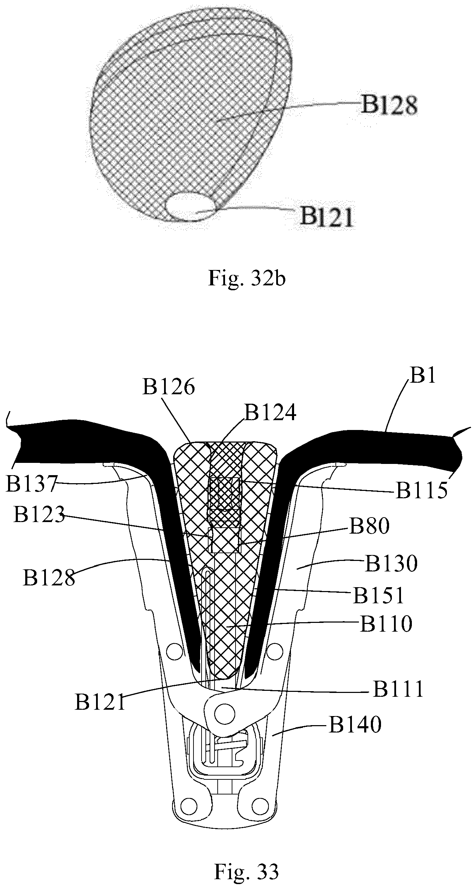

The self-expanding body B125 comprises a first section B124, a second section B126 and a third section B128 successively connected. The first section B124 extends from the second end B123 of the adjusting portion B120 toward the second end B115 of the supporting portion B110, and the first section B124 forms a bowl-like recessed area B122 with respect to the second section B126 (as shown in Fig. 31 and Fig. 32a). The first section B124 surrounds outside the second end B115 of the supporting portion B110, or the second end B115 of the supporting portion B110 is located in the recessed area B122. The second end B115 of the supporting portion B110 can be surrounded and shielded by the first section B124 of the self-expanding body B125 in the closed state and the opened state of the valve clamping device B100, so as not to be exposed from the adjusting portion B120. The second section B126 continues to extend radially outward from the first section B124. The third section B128 extends radially inward from the second section B126 toward the first end Bill of the supporting portion B110 to the first end B121 of the adjusting portion B12. In the first example, the terminal end of the recessed area B122 (the end of the first section B124 close to the second end B123 of the adjusting portion B120 is defined as the terminal end of the recessed area B122) extends toward the first end Bill of the supporting portion B110 to the second end B123 of the adjusting portion B120.

When the adjusting portion B120 is produced, first a woven mesh tube with two ends open is prepared, one end of the woven mesh tube is sleeved on a lining rod, and a molding sleeve is sleeved outside one end of the woven mesh tube to form the second end B123 of the adjusting portion B120; then the other end of the woven mesh tube is pulled outward and downward, so that the woven mesh tube is everted on the forming mold; next, heat setting treatment is carried out to form an adjusting portion B120 in an approximate inverted cone shape overall as shown in Fig. 32a to Fig. 32b, and the other end of the woven mesh tube forms a first end B121 with an opening of the adjusting portion B120 below one end of the woven mesh tube, so that the adjusting portion B120 is obtained after removing the molding sleeve and molding mold.

When the adjusting portion B120 and the supporting portion B110 are assembled, the part between the second end B115 and the first end B111 of the supporting portion B110 penetrates the hollow accommodation cavity of the adjusting portion B120. The second end B123 of the adjusting portion B120 is fixedly connected to the supporting portion B110 through the fixing part B80, and the fixing part B80 may be a steel sleeve. The second end B123 of the adjusting portion B120 penetrates between the steel sleeve and the outer surface of the supporting portion B110, then the second end B123 of the adjusting portion B120 is fixedly connected to the supporting portion B110 by laser welding the steel sleeve and the supporting portion B110. It should be noted that in other examples, the second end B123 of the adjusting portion B120 may be fixedly connected to the supporting portion B110 by welding without the fixing part B80. In the present example, the second end B123 of the adjusting portion B120 penetrates into the fixing part B80 toward the first end B111 of the supporting portion B110, and if the orientation of the second end B123 of the adjusting portion B120 in Fig. 30 is defined as up and the orientation of the first end B121 of the adjusting portion B120 is defined as down, that is, the second end B123 of the adjusting portion B120 penetrates into the fixing part B80 from up to down. The diameter of the opening of the first end B121 of the adjusting portion B120 may be equal to or slightly larger than the diameter of the supporting portion B110, so as to be movably sleeved on the supporting portion B110, so that the first end B121 of the adjusting portion B120 can slide smoothly along the axial direction of the supporting portion B110. Since the second end B123 of the adjusting portion B120 is fixedly connected to the supporting portion B110, and the first end B121 of the adjusting portion B120 is movably sleeved on the supporting portion B110, the center of gravity of the whole valve clamping device B100 is always located in the axial direction of the supporting portion B110, so the valve clamping device B100 has good self-centricity and is not easy to tilt.

More specifically, during the process of change from the opened state to the closed state of the valve clamping device B100, the radial dimension of the second section B126 of the self-expanding body B125 (marked with F in Fig. 30) is preferably 4 mm to 15 mm, more preferably 5 mm to 10 mm, and the radial dimension of the first end B121 of the adjusting portion B120 is 1 mm to 5 mm, more preferably 1.2 mm to 3 mm, so that the overall inverted cone shape of the adjusting portion B120 after being compressed can not only adapt to the spacing between different valve leaflets, but also have a fitting area with the valve leaflets as large as possible.

Referring to Fig. 29, Fig. 30 and Fig. 33, in the closed state of the valve clamping device B100, that is, when the valve leaflet B1 is clamped between the adjusting portion B120 and the clamping portion B130, since the second end B123 of the adjusting portion B120 is fixedly connected to the supporting portion B110, the first end B121 of the adjusting portion B120 is movably sleeved on the supporting portion B110, the axial movement of the second end B123 of the adjusting portion B120, the first section B124 and the second section B126 of the self-expanding body B125 is limited. The adjusting portion B120 extends axially toward the first end B121, and the self-expanding body B125 retracts radially under the pressing of the clamping portion B130, but the first section B124 resists the inward deformation of the second section B126, and the second section B126 transfers the resistance to the corresponding part of the third section B128 connected thereto. With the first end B121 of the adjusting portion B120 extending toward the valve edge of the valve leaflet B1, the overall shape of the adjusting portion B120 presents an inverted cone shape complementary to the shape of the opening of the clamping section B130. The second section B126 is located at the conical bottom of the inverted cone shape, and the third section B128 gradually converges toward the central axis of the adjusting portion B120 from the second section B126 to the first end B121. The whole third section B128 of the self-expanding body B125 can closely fit in the valve leaflet B1 without a radial gap. The elastic fit between the valve leaflet B1 and the adjusting portion B120 can be improved, so that the valve leaflet B1 and the adjusting portion B120 can adequately fit. Accordingly, the adjusting portion B120 can provide a large radial support force for the valve leaflet B1 to improve the clamping force to firmly clamp the valve leaflet B1. As shown in Fig. 33, the second section B126 of the self-expanding body B125 is not lower than the end face of the free end of the closed clamping portion, so that the length of the valve leaflet B1 clamped between the clamping portion B130 and the adjusting portion B120 is substantially the same as the length of the clamping portion B130.

It should be noted that in other examples, the outside and/or inside of the adjusting portion B120 of the mesh structure can be covered with a biocompatible film. On the one hand, the film can be used as a flow blocking film to block the blood flowing back from the clamp gap, thereby improving the effect of regurgitation treatment, and preventing the blood from entering the adjusting portion B120 to form thrombosis; on the other hand, the film can make the valve clamping device B100 more biocompatible. The material of the film may be, but is not limited to PTFE, EPTFE, polyester, silicone and other biocompatible polymers.

Certainly, the adjusting portion B120 is not limited to a mesh structure, but may also be other elastic and self-expandable hollow structures. For example, a colloidal silica of a dense structure or a sponge of a porous structure. The second end of the adjusting portion of the dense structure or the porous structure is fixedly sleeved on the supporting portion B110, and the first end is axially movably sleeved on the supporting portion B110. Similar first section, second section and third section may be set between the second end and the first end, or the valve leaflet may be adequately fitted in the adjusting portion based on the same principle.

Referring to Figs. 29 and 33 to 37, the clamping portion B130 comprises at least two clamp arms B131, and may generally comprise at least one group of the clamp arms B131, and each group of the clamp arms B131 comprises two clamp arms B131 symmetrically provided with respect to the adjusting portion B120. The clamping portion B130 in the present example comprises a group of the clamp arms B131. It should be noted that this is only an example. One skilled in the art can select an appropriate number of the clamp arms B131 as needed, for example, two or more groups of clamp arms. It should be noted that three or more clamp arms B131 may also be provided in each group as required. For example, the three valve leaflets of the tricuspid valve may be clamped with three clamp arms B131 that can be opened and closed relatively, so as to treat tricuspid regurgitation; alternatively, two of the valve leaflets of the tricuspid valve may be clamped by a pair of clamp arms B131 to reduce or treat tricuspid regurgitation.

In the present example, the valve clamping device B100 further comprises a driving portion B140 connected to the clamping portion B130 to drive the clamping portion B130 to open or close with respect to the adjusting portion B120. Specifically, the driving portion B140 is connected to each of the clamp arms B131. For example, the driving portion B140 is respectively connected to two clamp arms B131 in a group of clamp arms B131 to drive each of the clamp arms B131 to rotate around the adjusting portion B120, so that the clamp arm B131 is close to or away from the adjusting portion B120. In the delivering state, the driving portion B140 drives the clamp arm B131 to close around the adjusting portion B120, so as to reduce the outer diameter of the valve clamping device B100 and facilitate delivering; and after the valve clamping device B100 is opened in the heart, the driving portion B140 drives the clamp arm B131 to clamp the valve leaflet between the clamp arm B131 and the adjusting portion B120 to realize valve clamping.

In a preferred implementation mode of the present example, the valve clamping device B100 further comprises a gripping portion, and may generally comprise at least one group of gripping arms B151, and each group of the gripping arms B151 comprises two gripping arms B151 symmetrically provided with respect to the adjusting portion B120. The gripping portion (e.g., the gripping arm B151) is provided between the clamping portion B130 (e.g., the clamp arm B131) and the adjusting portion B120, and can be opened or closed with respect to the adjusting portion B120. The gripping portion is at least partially accommodated in the inner surface of the clamping portion B130. Certainly, three or more gripping arms B151 may also be provided in each group as required, so as to cooperate with the clamp arm B131 to realize the valve capture function.

In the delivering state, the gripping portion is at least partially accommodated in the inner surface of the clamping portion B130, that is, the gripping arm B151 is at least partially accommodated in the inner surface of the clamp arm B131, thereby reducing the outer diameter of the valve clamping device B100 and facilitating delivery. After the clamping arm B131 cooperates with the gripping arm B151 to capture the valve leaflet, the gripping arm B151 presses the valve leaflet into the inner surface of the clamping arm B131, which can increase the contact area between the clamping arm B131 and the valve leaflet and increase the clamping force on the valve leaflet.

The valve clamping device B100 further comprises a base B160 fixedly connected to the supporting portion B110, and each of the clamp arms B131 is rotationally connected to the base B160. Specifically, the proximal end of the base B160 is fixedly connected to the first end B 111 of the supporting portion B110. It should be noted that this part is defined as the term "base" for the convenience of description, and the structure to realize the function of the base B160 may also be the first end B111 itself of the supporting portion B110. Therefore, the definition of the term "base" should not limit the scope of the present disclosure. The clamp arms B131 in each group are rotationally connected together on the base B160 by a pivot B132. The first end B121 of the adjusting portion B120 is axially separated from the base B160. Driven by the driving portion B140, the clamp arms B131 cooperate with each other and can open and close around the adjusting portion B120 together.

In the present example, the driving portion B140 comprises a driving shaft B141, a connecting seat B142 and two connecting rods B143. One end of each of the connecting rods B143 is rotationally connected to the clamping portion B130, and the other end is rotationally connected to the connecting seat B142; one end of the driving shaft B141 is fixedly connected to the connecting seat B142, and the other end movably penetrates in the base B160. Specifically, one end of each connecting rod B143 is rotationally connected to the clamp arm B131, and the other end is rotationally connected to the connecting seat B142 by a pivot B144, that is, each of the clamp arms B131 is rotationally connected to the connecting seat B142 of the driving shaft B141 through the connecting rod B143 on the corresponding side. The driving shaft B141 movably passes through the base B160. When sliding axially with respect to the base B160, the driving shaft B141 drives the connecting rod B143 to rotate and drives the clamp arm B131 to open or close centered on a rotationally connected part thereof with the base B160.

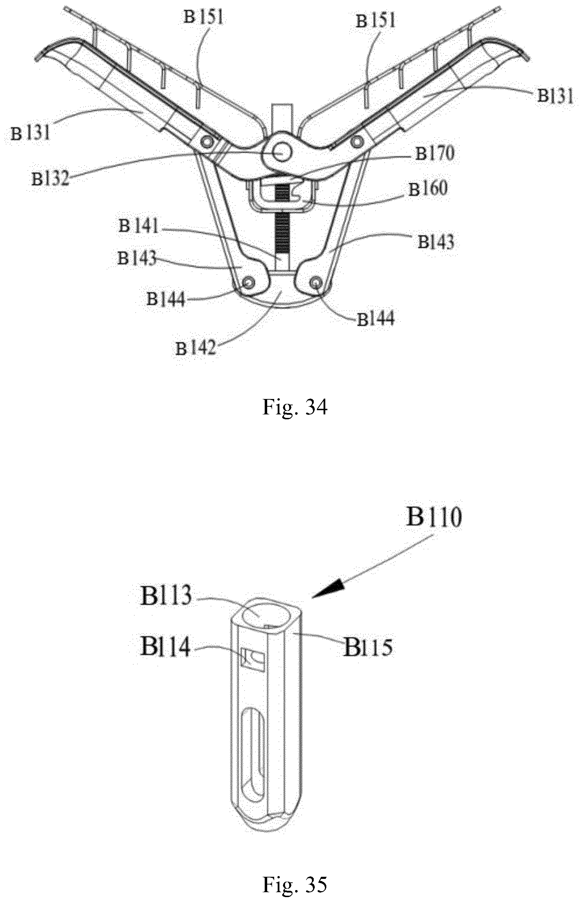

Specifically, the driving portion B140 comprises at least one group of connecting rods B143, and the setting of the connecting rods B143 corresponds to the setting of the clamp arms B131. For example, if two clamp arms B131 are used in Fig. 34, two cooperating connecting rods B143 are correspondingly provided. One end of the connecting rod B143 is rotationally connected to the connecting seat B142 through the pivot B144 such as a pin, and the other end is rotationally connected to the corresponding clamp arm B131 through a pivot such as a pin. Each of the clamp arms B131 is rotationally connected to the base B160 through the pivot B132 such as a pin. When the driving shaft B141 moves axially toward the first end B111 of the supporting portion B110 with respect to the base B160, the driving shaft B141 drives the connecting rod B143 to move. Under the pull of the connecting rod B143, the clamp arm B131 rotates around the pivot B132 and opens with respect to the base B160. When the driving shaft B141 moves axially with respect to the base B160 toward the second end B115 of the supporting portion B110, the connecting rod B143 pushes the clamp arm B131 to rotate around the pivot B132 and close with respect to the base B160. The shape of the connecting seat B142 may be any structure such as a hemisphere, a spherical crown or a warhead shape, so as to make the valve clamping device B100 easier to be pushed in the body. The driving shaft B141 and the connecting seat B142 may be an integrated structure or a non-integrated structure. In order to ensure the safety after implantation, the driving shaft B141 and the connecting seat B142 are made of biocompatible materials such as polyester, silicone, stainless steel, cobalt alloy, cobalt chromium alloy and titanium alloy, preferably stainless steel or cobalt chromium alloy with high hardness.

Preferably, as shown in Fig. 34, the valve clamping device B100 further comprises a locking portion B170 provided in the base B160, which restricts the relative movement of the driving shaft B141 and the base B160. In the delivering state, the locking portion B170 restricts the relative movement of the driving shaft B141 and the base B160, so as to ensure that the clamping portion B130 is always in a closed state with respect to the adjusting portion B120 and the supporting portion B110 and avoid accidental opening of the clamping portion B130; after reaching the vicinity of the mitral valve, the restriction of the locking portion B170 on the driving shaft B141 is unlocked, and the clamping portion B130 can be driven to open and support the valve leaflet with respect to the adjusting portion B120 and the supporting portion B110 by the driving portion B140; after clamping the valve leaflet, the locking portion B170 again restricts the relative movement of the driving shaft B141 and the base B160, so as to maintain the clamped state of the valve leaflet B1. The locking portion of any suitable structure in the related art can be adopted, which is not be described here.

Referring to Fig. 29 and Fig. 33, further, the terminal end of each clamp arm B131 (the end of the clamp arm B131 away from a rotating connection part thereof or the free end is defined as the terminal end of the clamp arm B131) is also provided with a flanging section B137. Viewed from the front view of Fig. 33, the flanging section B137 is a cambered surface overturned toward the outside of the terminal end of the clamp arm B131, and the radius of the cambered surface is preferably 1 mm to 2 mm. When the clamp arms B131 are closed with respect to the adjusting portion B120 to clamp the valve leaflet B1 therebetween, the valve leaflet B1 fits in the flanging section B137 of a cambered surface, which increases the supporting area of the terminal end of the clamp arm B131 against the valve leaflet B1, can avoid the local stress concentration of the valve leaflet B1 at the terminal end of the clamp arm B131 and effectively reduce the damage to the valve leaflet caused by the repeated friction between the terminal end edge of the clamp arm B131 and the valve leaflet B1 with the beating of the heart. When the clamp arm B131 abuts against the adjusting portion B120, the self-expanding body B125 of the adjusting portion B120 is axially protruded from the flanging section B137, or the second section B126 of the self-expanding body B125 is higher than the flanging section B137, so as to ensure that the length of the valve leaflet B1 clamped between the clamp arm B131 and the third section B128 of the self-expanding body is not less than the length of the clamp arm B131.

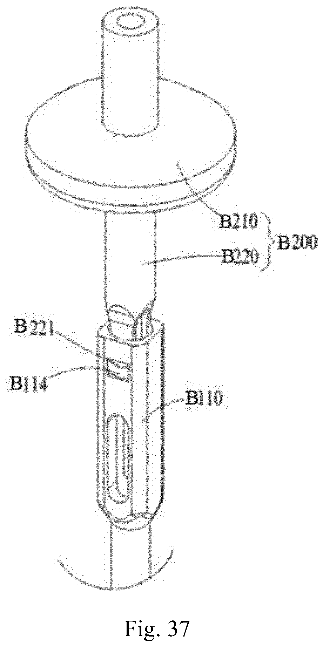

Referring to Fig. 37 to Fig. 41, the present disclosure also provides a valve clamping system comprising the valve clamping device B100 and a delivery device B200, wherein the delivery device B200 comprises a pushing sheath B210 with a certain axial length and a mandrel (not shown in the figure) movably penetrating in the pushing sheath B210. The pushing sheath B210 is detachably connected to the supporting portion B110, and the mandrel is detachably connected to the driving portion B140 for driving the opening and closing of the clamping portion B130. In the present example, the proximal end of the driving shaft B141 is provided with an external thread, and the mandrel is threadedly connected to the driving shaft B141, so that the axial movement of the driving shaft B141 can be controlled outside the patient through the mandrel. It should be noted that only part of structure of the delivery device is illustrated here, and any suitable structure in the related art can be adopted for other parts, which is not described here.

Specifically, the proximal outer wall of the supporting portion B110 is symmetrically provided with at least one locking position B114 connected to the cavity of the supporting portion B110, the distal end of the pushing sheath B210 is provided with a connecting member B220, the connecting member B220 comprises two branches, and the terminal end of each branch is a protruded locking nose B221. In the natural state, the two branches point to the central axis of connecting member B220. During assembly, the connecting member B220 is inserted into the supporting portion B110, and then the mandrel of the delivery device B200 is inserted into the pushing sheath B210 until the mandrel is inserted into the connecting member B220, and the two branches of the connecting member B220 are pushed outward. The locking noses B221 at the branch terminal ends are locked into the two locking positions B114 of the supporting portion B110, so as to connect the supporting portion B110 with the connecting member B220, that is, connect the valve clamping device B100 with the delivery device B200. When the mandrel is withdrawn from the connecting member B220 and the pushing sheath B210, the two branches restores the inward natural state, and the locking nose B221 is separated from the locking position B114 of the supporting portion B110, so that the connection between the valve clamping device B100 and the delivery device B200 is released. The connecting member B220 may be made of materials with certain hardness and elasticity such as nickel and titanium. The pushing sheath B210 may adopt a multi-layer composite tube. The mandrel may be made of stainless steel or nickel titanium alloy.

Referring to Fig. 35 and Fig. 37, the interior of the supporting portion B110 is provided with a through hole as the penetrating channel B113 of the driving shaft B141, and the driving shaft B141 axially slidingly penetrates in the penetrating channel B113 of the supporting portion B110 and is fixedly connected to the connecting seat B142. After the clamping portion B130 and the gripping portion B150 cooperate and capture the valve leaflet, the driving shaft B141 is driven to move axially by the mandrel to drive the clamp arm B131 to be completely closed with respect to the supporting portion B110, so that the valve clamping device B100 is in a closed state and falls below the valve. After that, the connection between the mandrel and the driving shaft B141 may be released, the mandrel is withdrawn from the connecting member B220, and the locking nose B221 is separated from the locking position B114 of the supporting portion B110, so as to disconnect the valve clamping device B100 from the delivery device B200.

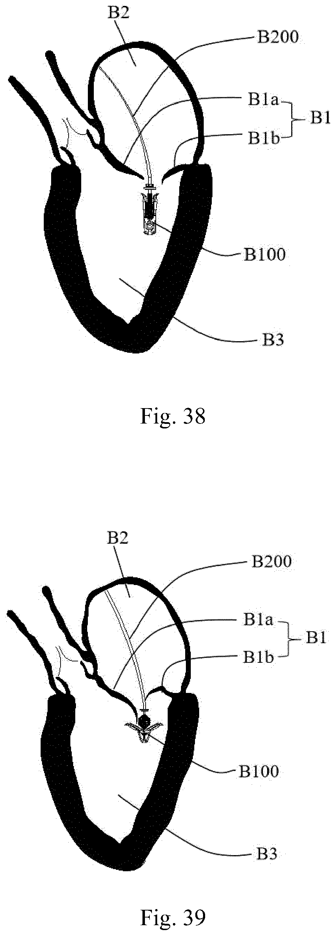

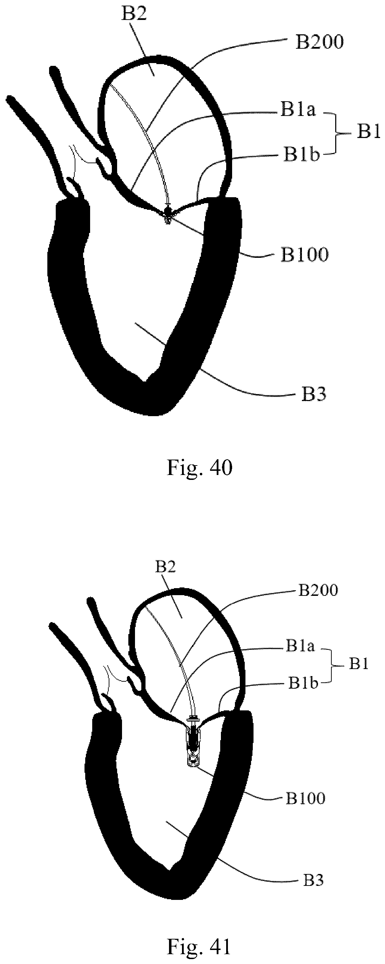

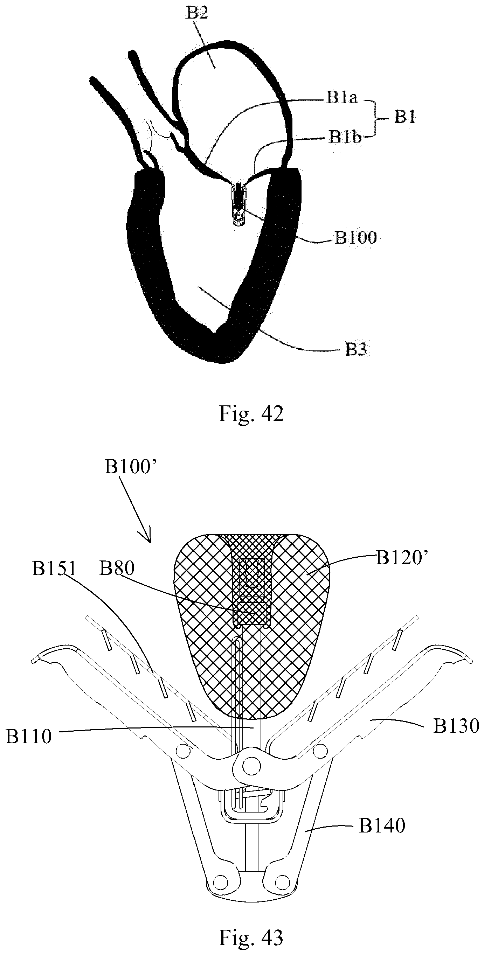

Referring to Fig. 38 to Fig. 42, a use process of the valve clamping device B100 of the present disclosure is explained below taking anterograde approach and repair of the mitral valve via the atrial septum-left atrium through the a transcatheter route as an example.
Step 1: pushing the driving shaft B141 and the valve clamping device B100 connected thereto from the left atrium B2 to the left ventricle B3 via the mitral valve B1 by a guiding device such as a bendable sheath (not shown in the figure), as shown in Fig. 38;
Step 2: adjusting the valve clamping device B100 to approach the anterior mitral leaflet B1a and the posterior mitral leaflet B1b of the mitral valve B 1;
Step 3: unlocking the locking portion B170 in the base B160, pushing the mandrel and the driving shaft B141 to the distal end, driving the clamp arm B131 to open with respect to the supporting portion B110, and adjusting the direction of the clamp arm B131, at which time a relative position of the clamp arm B 131 and the anterior and posterior mitral leaflets B1a, B1b of the mitral valve B 1 can be observed by an X-ray device, so that the clamp arm B131 is perpendicular to a coapting line of the mitral valve B1, as shown in Fig. 39;
Step 4: withdrawing the entire valve clamping device B100 to the proximal end to let the clamp arm B131 hold the valve leaflet B1 on the left ventricle B3 side, and releasing the gripping arms B151 on both sides, wherein the gripping arm B151 on each side presses the valve leaflet B1 on the atrium side and captures the valve leaflet B1 together with the clamp arm B131 on the side, as shown in Fig 40;
Step 5: pulling the mandrel and the driving shaft B141 to the proximal end when the anterior mitral leaflet B1a and the posterior mitral leaflet B1b of the mitral valve B1 are captured between a pair of the clamp arms B131 and the gripping arms B151 respectively, thereby driving the clamp arms B131 to close, as shown in Fig. 41;
Step 6: releasing the threaded connection between the mandrel and the driving shaft B141 and withdrawing the mandrel, so that the two branches of the connecting member B220 restores a state of converging to the central axis, the locking nose B221 is disengaged from the locking position B114 of the supporting portion B110, and the valve clamping device B100 is disconnected from the delivery device B200, and then withdrawing the delivery device B200 from the body, resulting in an implanted state as shown in Fig. 42, at which time the valve clamping device B100 pulls the anterior mitral leaflet B1a and the posterior mitral leaflet B1b of the mitral valve B1 toward each other to obtain a double orifice mitral valve and complete edge-to-edge repair of the mitral valve.

After the valve clamping device B100 is implanted, the elastic adjusting portion B120 is filled between the anterior mitral leaflet B1a and the posterior mitral leaflet B1b of the clamped mitral valve B1 and provides a radial support force for the valve leaflet B1. The adjusting portion B120 has a cushioning effect on the pulsating valve leaflet B1, so that the pulling degree of the valve leaflet B1 by the valve clamping device B100 can be adjusted to avoid damaging the valve leaflet B1.

### Second Example

Referring to Fig. 43 to Fig. 45, compared with the valve clamping device B100 of the first example, in the valve clamping device B100' of a second example of the present disclosure, the structure of the supporting portion B110, the clamping portion B130, the driving portion B140, the gripping portion B151, and the like remains the same, which will not be repeated here, but the structure of the adjusting portion B120' has changed.

Specifically, in the second example, the adjusting portion B120' adds a bending section B129 to the self-expanding body compared with the adjusting portion B120 in the first example. The bending section B129 is connected between the second end B123 of the adjusting portion B120' and the first section B124 of the self-expanding body. Preferably, the cross-section shape of the bending section B129 is an arc shape recessed toward the first end B121 of the adjusting portion B120'. The first section B124 and the bending section B129 still form a recessed area B122' with respect to the second section B126, and the terminal end of the recessed area B122' (one end of the bending section B129 close to the second end B123 of the adjusting portion B120' is defined as the terminal end of the recessed area B122') extends toward the second end B115 of the supporting portion B110 to the second end B123 of the adjusting portion B120'.

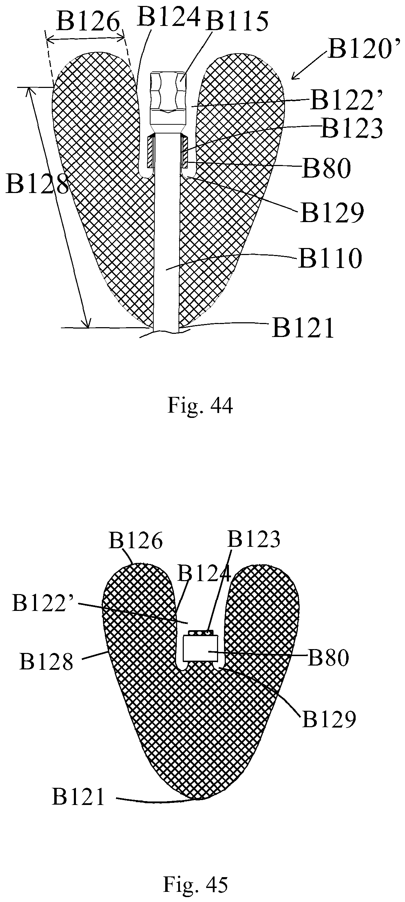

When the adjusting portion B120' is produced, first a woven mesh tube with both ends open is prepared, the woven mesh tube is sleeved on a lining rod, and then a molding sleeve is sleeved outside an upper end of the woven mesh tube to form the second end B123 of the adjusting portion B120'; then the lower end of the woven mesh tube is pushed upward (the orientation of the second end B123 of the adjusting portion B120' in Fig. 44 is defined as up, and the orientation of the first end B121 is defined as down), and heat setting treatment is carried out on the woven mesh tube with the molding mold to form the adjusting portion B120' with an overall shape still approximate to an inverted cone shape as shown in Fig. 44 and Fig. 45, and the lower end of the woven mesh tube forms a first end B121 with an opening of the adjusting portion B 120' below the upper end of the woven mesh tube, and the molding sleeve and the molding mold are removed to obtain the adjusting portion B120'.

When the adjusting portion B120' and the supporting portion B110 are assembled, the part between the second end B115 and the first end B111 of the supporting portion B 110 penetrates in the hollow accommodation cavity of the adjusting portion B120. The second end B123 of the adjusting portion B120' is fixedly connected to the supporting portion B110 through the fixing part B80, and the fixing part B80 may be a steel sleeve. The second end B123 of the adjusting portion B120 penetrates between the steel sleeve and the outer surface of the supporting portion B110, and then the second end B123 of the adjusting portion B120' is fixedly connected to the supporting portion B110 by laser welding the steel sleeve and the supporting portion B110. In the present example, the second end B123 of the adjusting portion B120' penetrates into the fixing part B80 toward the second end B 115 of the supporting portion B110, that is, the second end B123 of the adjusting portion B120' penetrates in the fixing part B80 from down to up.

When the valve clamping device B100' is closed, the bending section B129 resists the inward deformation of the first section B124, and the resistance is superimposed and transferred to the corresponding part of the third section B128 connected to the second section B126 to further improve the elastic fit between the valve leaflet and the adjusting portion B120, so that the valve leaflet can adequately fit in the adjusting portion B120. Accordingly, the adjusting portion B120 can provide a greater radial support force for the valve leaflet, and further increase the clamping force to firmly clamp the valve leaflet.

### Third Example

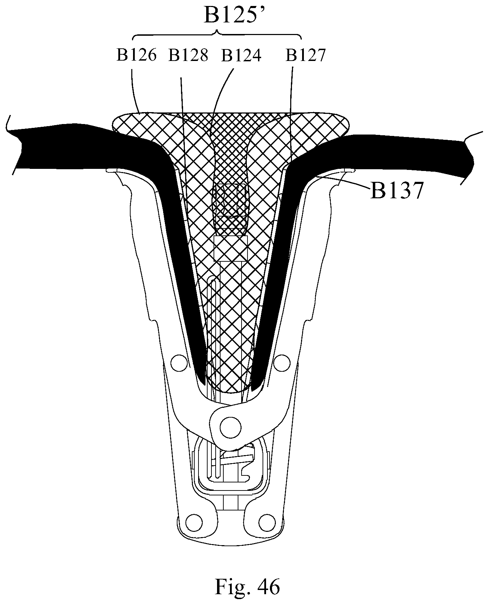

As shown in Fig. 46, compared with the valve clamping device of the first example, in the valve clamping device of a third example of the present disclosure, the structure of the supporting portion, the clamping portion, the driving portion, the gripping portion, and the like remains the same, which will not be repeated here, but the structure of the self-expanding body B 125' of the adjusting portion has changed.

The self-expanding body B125' in the third example further comprises an adaptation section B127 in addition to the first section B124, the second section B126 and the third section B128. The adaptation section B127 is connected between the second section B126 and the third section B128, and extends radially outward with respect to the third section B128 (the radially inward is toward the central axis of the supporting portion B110; and the radially outward is away from the central axis of the supporting portion B110). The adaptation section B127 is provided corresponding to the flanging section B 137 at the terminal end of the clamp arm, and the shape of a side thereof facing the flanging section B137 is complementary to the cambered surface of the flanging section B137.

When the clamp arms are closed with respect to the adjusting portion B120 to clamp the valve leaflet therebetween, the adaptation section B127 is protruded from the flanging section B137 in the axial direction, or the adaptation section B127 is higher than the flanging section B137. Part of the valve leaflet is clamped between the adaptation section B127 and the flanging section B137. On the one hand, the valve leaflet forms a fit in the cambered surface of the flanging section B137 to increase the supporting area of the clamp arm terminal end against the valve leaflet, which can avoid the local stress concentration of the valve leaflet at the terminal end of the clamp arm and effectively reduce the damage to the valve leaflet caused by repeated friction between the terminal end edge of the clamp arm and the valve leaflet with the beating of the heart; on the other hand, the valve leaflet also forms a fit in the arc-shaped adaptation section B127, which further increases the fit area between the valve leaflet and the adjusting portion, improves the elastic fit between the valve leaflet and the adjusting portion, and makes the valve leaflet fit better in the adjusting portion. Accordingly, the adjusting portion can provide a sufficient radial support force for the valve leaflet, and further increase the clamping force to firmly clamp the valve leaflet.

### Fourth Example

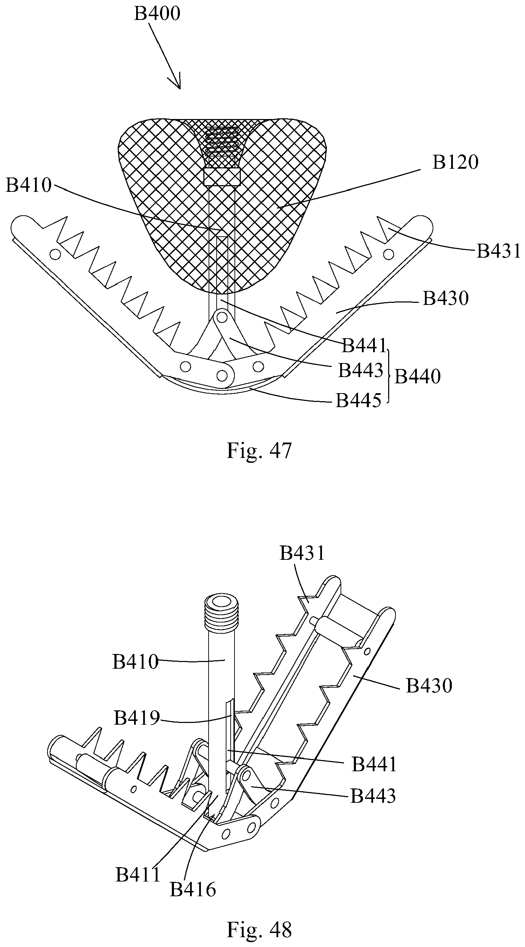

Referring to Fig. 47 to Fig. 49, compared with the valve clamping device B 100 of the first example, in a valve clamping device B400 of a fourth example of the present disclosure, the structure of the adjusting portion B120 remains the same, which will not be repeated here, but the structure of the supporting portion B410, the clamping portion B430 and the driving portion B440 has changed.

Specifically, in the fourth example, the clamping portion B430 comprises at least two clamp arms, and may generally comprise at least a group of clamp arms, each group of the clamp arms comprises two clamp arms symmetrically provided with respect to the adjusting portion B120. The clamping portion 40 in the present example comprises a group of clamp arms. Each of the clamp arms is provided with at least one anchor B431. When the clamp arm is closed with respect to the adjusting portion B120, the anchor B431 can abut on the valve leaflet and make the valve leaflet embedded in the grid of the adjusting portion B120 of a mesh structure, so as to hold the valve leaflet tissue through the anchor B431 on the basis that the adjusting portion B120 and the clamp arm clamp the valve leaflet.

The driving portion B440 comprises a driving shaft B410, an automatic closing unit B445 and at least two connecting rods B443. One end of each of the connecting rods B443 is rotationally connected to a corresponding clamp arm, and the other end is directly rotationally connected to the driving shaft B441 through a pin shaft; the driving shaft B441 movably penetrates in the supporting portion B410; and the automatic closing unit B445 is connected to two clamp arms to make the clamping portion B430 abut against the adjusting portion B120 in a natural state.

The base B416 is integrated with the first end B411 of the supporting portion B410, and the two clamp arms are rotationally connected to the base B416. The supporting portion B410 is provided with an axial groove B419 through which the pin shaft passes. When the driving shaft B441 drives the pin shaft to move toward the first end B411 of the supporting portion B410 in the axial groove B419, the connecting rod B443 is driven to overcome obstruction of the automatic closing unit B445 to open the two clamp arms relatively.

In the present example, the automatic closing unit B445 is a U-shaped elastic sheet, and two ends of the U-shaped elastic sheet are respectively connected to a clamp arm. When the driving shaft B441 does not apply thrust to the pin shaft, the U-shaped elastic sheet drives the two clamp arms to close and abut against the adjusting part B120 with its own reset. It should be noted that in other examples, the automatic closing unit B445 may also be an elastic member such as a V-shaped elastic sheet or a torsional spring.

### Fifth Example

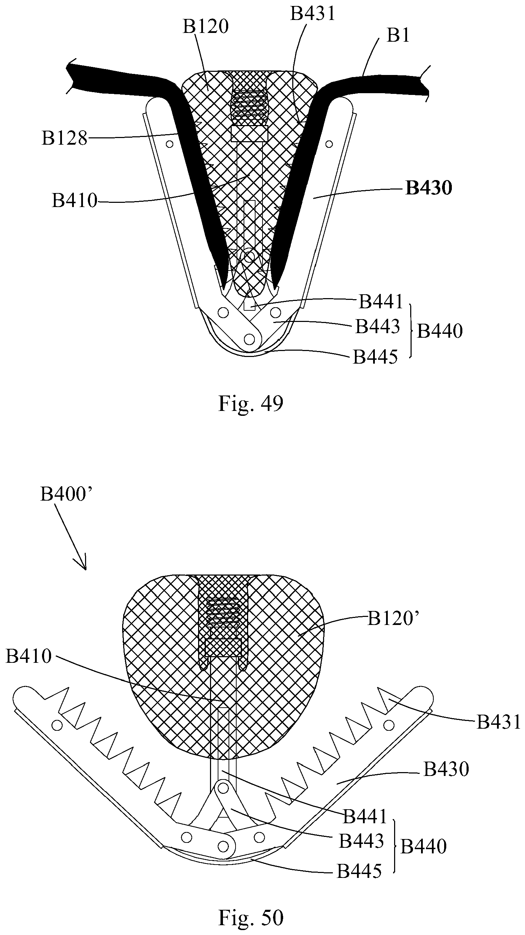

Referring to Fig. 50, compared with the valve clamping device B400 in the fourth example, a valve clamping device B400' in a fifth example of the present disclosure replaces the adjusting portion B120 in the fourth example by the adjusting portion B120' in the second example, and other structures remain the same and will not be repeated here.

### Sixth Example

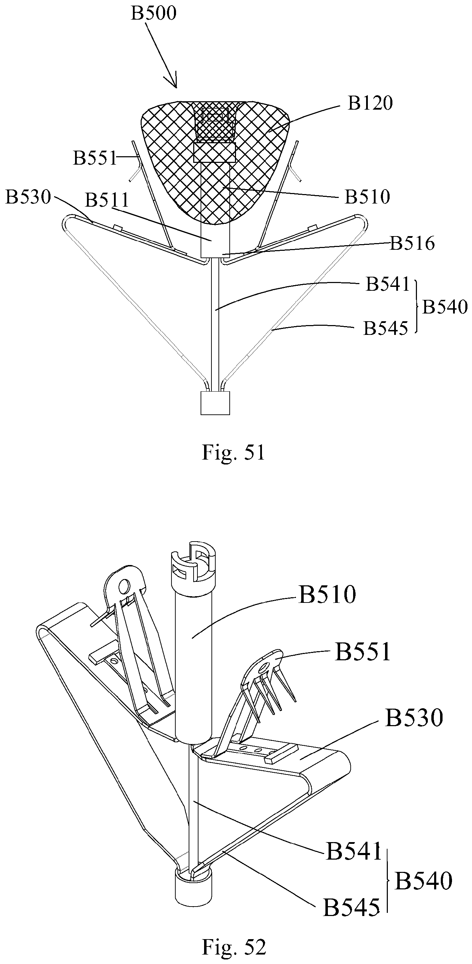

Referring to Fig. 51 and Fig. 52, compared with the valve clamping device B100 of the first example, the structure of the adjusting portion B120 of the valve clamping device B500 in a sixth example of the present disclosure remains the same and will not be repeated here, but the structure of the clamping portion B530, the driving portion B540, and the like has changed.

In the present example, the base B516 is integrated with the first end B511 of the supporting portion B510, and one end of the clamp arm of the clamping portion B530 is connected to the base B516. The driving portion B540 comprises a driving shaft B541 and at least two elastic driving arms B545. One end of the elastic driving arm B545 is fixedly connected to one end of the driving shaft B541, the other end of the elastic driving arm B545 is connected to the other end of the clamp arm, and the other end of the driving shaft B545 movably penetrates in the supporting portion B510; the elastic driving arm B545 is configured to make the clamping portion B530 abut against the adjusting portion B120 in a natural state; one end of the gripping arm B551 is connected to the clamp arm of the clamping portion B530, and when the valve clamping device B500 is opened, the gripping arm B551 is controlled by a pull wire (not shown in the figure) in the delivery device to open with respect to the clamp arm, so as to allow the valve leaflet to enter between the gripping arm B551 and the clamp arm.

In the present example, the two clamp arms and the two elastic driving arms B545 are integrated, that is, the two clamp arms themselves are elastic. When the driving shaft B541 moves toward the first end B511 of the supporting portion B510, the two clamp arms are opened with respect to each other by overcoming the obstruction of the two elastic driving arms B545; and when the driving shaft B541 does not apply thrust to the elastic driving arm B545, the two elastic driving arms B545 use their own reset to drive the two clamp arms to almost close and abut against the adjusting portion B120. It should be noted that when the driving shaft B541 continuously pushes toward the first end B511 of the supporting portion B510, the connecting position between the clamp arm and the driving arm B545 gradually approaches the driving shaft B541 until the clamp arm and the driving arm B545 are substantially in a straight line, and then a pull wire is used to control the gripping arm B551 to fit in the adjusting portion B120. In this state, it is easier to put the whole flattened valve clamping device B500 into the sheath.

In addition, the valve clamping device B500 of the present example can realize dynamic balance of the valve clamping state: when the valve leaflet applies a relatively large pulling force to the valve clamping device B500, the elastic driving arm B545 and clamp arm can adjust the clamping angle within a certain range without being separated from the valve leaflet, so as to prevent the valve leaflet from being damaged by an excessive pulling force.

### Seventh Example

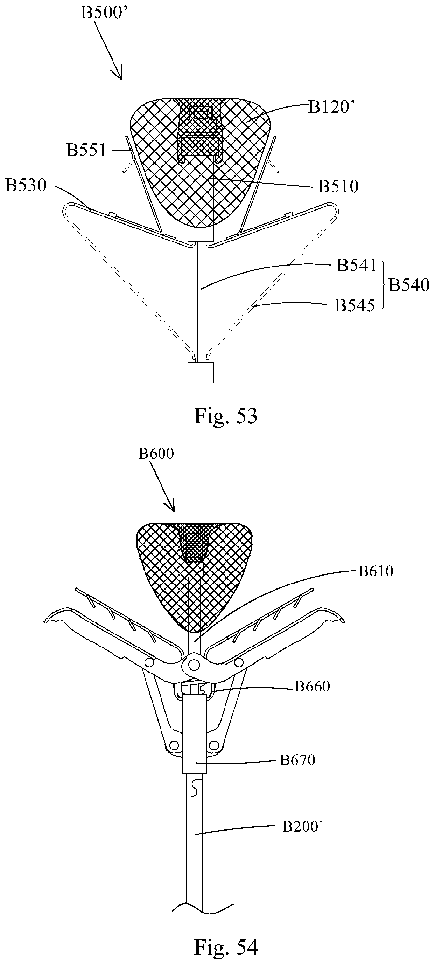

Referring to Fig. 53, compared with the valve clamping device B500 in the sixth example, a valve clamping device B500' in a seventh example of the present disclosure replaces the adjusting portion B120 in the sixth example by the adjusting portion B120' in the second example. Other structures remain the same and will not be repeated here.

### Eighth Example

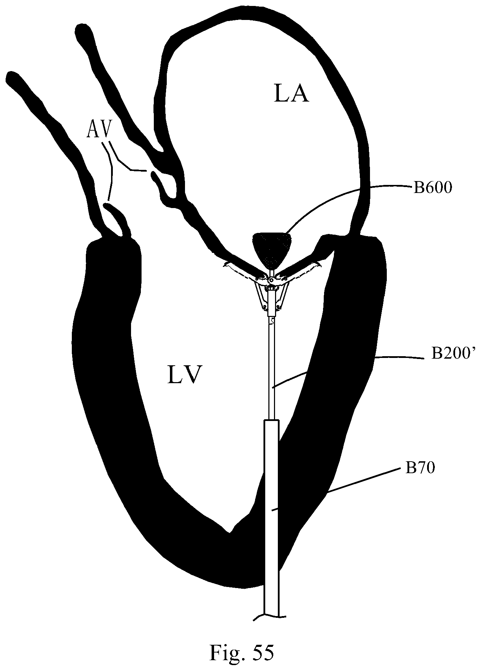

Referring to Fig. 54 and Fig. 55, compared with the valve clamping device B100 in the first example, in a valve clamping device B600 in an eighth example of the present disclosure, only the structure of the supporting portion B610 is changed, and other structures remain the same and will not be repeated here.

In the present example, the connection part between the supporting portion B610 and the delivery device B200' is no longer provided at the second end as in the first example, but a connection part B670 is provided on the base B660 at the first end of the supporting portion B610, and the connection part B670 is detachably connected to the pushing sheath of the delivery device B200'. Specifically, the connection part B670 and the pushing sheath of the delivery device B200' are respectively provided with a splicing structure with complementary shapes, and an outer sheath B70 is movably sleeved outside the pushing sheath. When the outer sheath B70 wraps the splicing structure with complementary shapes, the supporting portion B610 remains connected to the delivery device B200', and when the outer sheath B70 is withdrawn and the splicing structure with complementary shapes is exposed, the supporting portion B610 can be disconnected from the delivery device B200'.

As shown in Fig. 55, the delivery device B200' of the present example can push the valve clamping device B600 into the heart via a transapical route to perform edge-to-edge repair of the mitral valve. It should be noted that before the two clamp arms are closed, the outer sheath B70 should still wrap the splicing structure with complementary shapes; after the two clamp arms are closed, first the threaded connection between the mandrel and the driving shaft is released, and the mandrel is withdrawn, then the outer sheath B70 is withdrawn to expose the splicing structure, so that the connection between the valve clamping device B600 and the delivery device B200' can be released.

It should be noted that the valve clamping system provided in the present disclosure comprises any of the above valve clamping devices and a delivery device capable of delivering the valve clamping device from the outside of the body to the vicinity of the mitral valve and clamping the valve leaflets.

It should be noted that the valve clamping device and the valve clamping system provided in the present disclosure can also perform edge-to-edge repair of a tricuspid valve, as long as the corresponding intervention path (e.g., femoral vein to inferior vena cava to right atrium - right ventricle) is selected and an appropriate number of valve clamping devices are implanted according to the number of valve leaflets to be repaired (for example, three valve clamping devices are implanted to clamp anterior and posterior mitral leaflets, posterior and septal leaflets, and septal and anterior mitral leaflets of a tricuspid valve respectively).

### Third Embodiment

### First Example

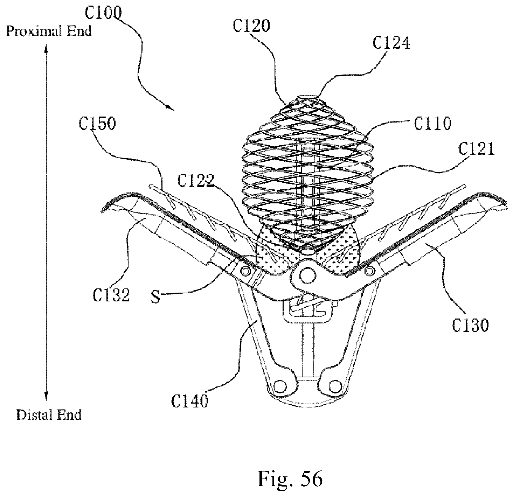

Referring to Fig. 56 to Fig. 58, an adaptive valve clamping device C100 according to a first example of the present disclosure can be used for edge-to-edge repair of a mitral or tricuspid valve to treat mitral or tricuspid regurgitation. In order to ensure safety of the adaptive valve clamping device C100 after implantation, the adaptive valve clamping device C100 is made of biocompatible materials overall. The adaptive valve clamping device C100 for edge-to-edge repair of a mitral valve is described as an example in detail below. An operator uses a delivery device C200 to push the adaptive valve clamping device C100 to the patient's mitral valve, and then remotely operate the adaptive valve clamping device C100 to clamp the anterior and posterior mitral leaflets of the mitral valve together. When the valve leaflets of the mitral valve are coapted edge-to-edge, the operator can release the connection between the delivery device C200 and the adaptive valve clamping device C100, so as to implant the adaptive valve clamping device C100 into the patient, fix the anterior and posterior mitral leaflets of the mitral valve together, and realize the "edge-to-edge repair" of the mitral valve.

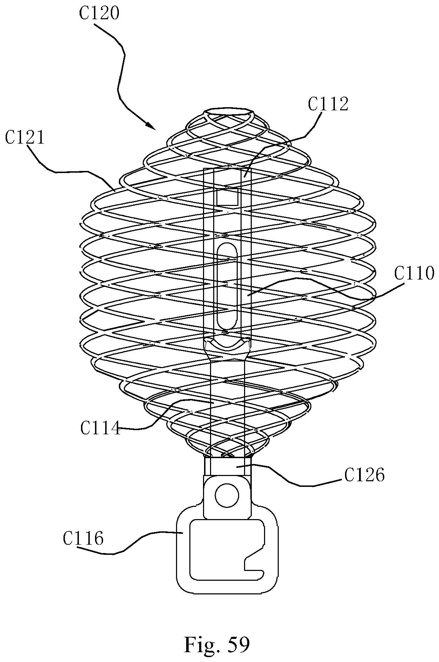

Referring to Fig. 59, the adaptive valve clamping device C100 comprises a supporting portion C110, a hollow adjusting portion C120 and a clamping portion C130. The supporting portion C110 comprises a first seat body C112 and a second seat body C114 connected to the first seat body C112. The first seat body C112 is provided in the adjusting portion C120. The adjusting portion C120 comprises a first end C122 and a second end C124 provided oppositely, and a self-expanding body C121 between the first end C122 and the second end C124. The first end C122 of the adjusting portion C120 is movably sleeved outside the second seat body C114 and can move axially with respect to the second seat body C114, and the second end C124 of the adjusting portion C120 hangs in the air. The first seat body C112 is closer to the second end C124 of the adjusting portion C120 than the second seat body C114. The clamping portion C130 comprises at least two clamp arms C132, and each of the clamp arms C132 is rotationally connected to the supporting portion C110. The rotating connection part of the clamp arm C132 is close to the first end C122 of the adjusting portion C120, and the clamp arm C132 rotates around the supporting portion C110 to be close to or away from the adjusting portion C120.

In the adaptive valve clamping device C100 of the above example, the first end C122 of the adjusting portion C120 is movably sleeved outside the second seat body C114 of the supporting portion C110, the second end C124 of the adjusting portion C120 hangs in the air and is relatively closer to the first seat body C112, and the adjusting portion C120 may move axially with respect to the supporting portion C110. When the clamp arm C132 of the clamping portion C130 approaches the adjusting portion C120 to close and clamp the valve leaflet, the whole adjusting portion C120 axially moves to the first seat body C112, which can increase space S of the rotating connection part of the clamp arm C132 close to the adjusting portion C120, so as to avoid excessive accumulation of valve leaflets in the space S. During the closing process of the clamping portion C130, the adjusting portion C120 can better adapt to the deformation of the valve leaflet at the space S to adjust the overall pulling of the valve leaflet by the clamping portion C130, so as to make the axial force of the valve leaflet more balanced, which is conducive to better closing of the clamping portion C130 after gripping the valve leaflet and avoids damage to the valve leaflet at the space S.

It should be noted that the clamp arm C132 of the clamping portion C130 is rotationally connected to the supporting portion C110, and the first end C122 of the adjusting portion C120 is movably sleeved outside the second seat body C114 of the supporting portion C110, that is, the clamping portion C130 is provided outside the adjusting portion C120. The clamp arm C132 of the clamping portion C130 rotates around the supporting portion C110 to approach the adjusting portion C120, that is, the clamping portion C130 closes with respect to the adjusting portion C120; and the clamp arm C132 of the clamping portion C130 rotates around the supporting portion C110 away from the adjusting portion C120, that is, the clamping portion C130 opens with respect to the adjusting portion C120.

The second end C124 of the adjusting portion C120 hangs in the air and is closer to the first seat body C112 of the supporting portion C110. The first seat body C112 is provided in the adjusting portion C120, so there is a gap between the second end C124 of the adjusting portion C120 and the first seat body C112 of the supporting portion C110, which can avoid interference or winding between the second end C124 of the adjusting portion C120 and the first seat body C112 of the supporting portion C110, so as to ensure the implantation safety of the instrument. The self-expanding body C121 of the adjusting portion C120 may be deformed in the radial and axial directions, so the adjusting portion C120 comprises a natural state and a compressed state. The self-expanding body C121 may be made of a shape memory material. When the clamping portion C130 opens with respect to the adjusting portion C120, the clamping portion C130 has no contact with the adjusting portion C120, and the adjusting portion C120 is in a natural state; and when the clamping portion C130 is closed with respect to the adjusting portion C120, the adjusting portion C120 is radially compressed and extends axially to the first seat body C112 of the supporting portion C110 in a compressed state. When the clamping portion C130 is closed with respect to the adjusting portion C120, the adjusting portion C120 is gradually compressed radially and extends axially to the first seat body C112 of the supporting portion C110, and the spacing between the second end C124 of the adjusting portion C120 and the first seat body C112 of the supporting portion C110 increases gradually. Since the first end C122 of the adjusting portion C120 is movably sleeved outside the second seat body C114 of the supporting portion C110, the first end C122 of the adjusting portion C120 will move to the first seat body C112 of the supporting portion C110, thereby increasing the space S of the rotating connection part of the clamp arm C132 close to the first end C122 of the adjusting portion C120, so as to avoid excessive accumulation of valve leaflets in the space S and ensure that the clamping portion C130 is normally closed without damaging the valve leaflet.

Meanwhile, since the first end C122 of the adjusting portion C120 is movably sleeved outside the second seat body C114 of the supporting portion C110, the second end C124 of the adjusting portion C120 hangs in the air, and the adjusting portion C120 is no longer limited by the supporting portion C110, which improves the axial deformation ability of the adjusting portion C120 and enhances a bending deformation ability thereof along the axial direction. Therefore, when the adaptive valve clamping device C100 is radially compressed into the delivery sheath for in vivo delivery, it is not only easy to be compressed into the sheath, but can also adapt to blood vessels with different bending curvatures during delivery in the blood vessel, so as to facilitate the passage of the delivery sheath in the blood vessel, thereby reducing the damage to the blood vessel wall. The first end C122 of the adjusting portion C120 is movably sleeved outside the second seat body C114 of the supporting portion C110, and the second end C124 of the adjusting portion C120 hangs in the air, so that the center of gravity of the adjusting portion C120 is always located in the axial direction of the supporting portion C110 (i.e. the axial line of the adjusting portion C120). Therefore, the self-centricity of the adjusting portion C120 is good and is not easy to tilt.

In addition, after the implantation of the adaptive valve clamping device C100, in the process of clamping the valve leaflet and the adjusting portion C120 by the clamping portion C130, since the axial deformation of the adjusting portion C120 is not limited, the elastic fit between the valve leaflet and the adjusting portion C120 can be improved, and then the adaptability of the physiological structure of the valve leaflet in different patients can be improved.

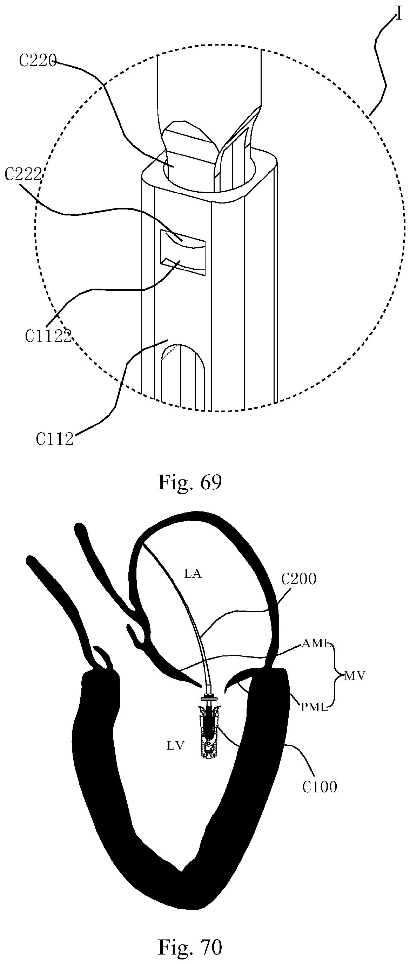

Referring to Fig. 70 to Fig. 74, in the adaptive valve clamping device C100 of the present example, the supporting portion C110 is detachably connected (such as threaded connection and snap connection) to the delivery device C200. The delivery device C200 pushes the adaptive valve clamping device C100 into the heart via a catheter. The second end C124 of the adjusting portion C120 is a proximal end of the adaptive valve clamping device C100, and the first end C122 of the adjusting portion C 120 is a distal end thereof; and the first seat body C 112 of the supporting portion C 110 is close to the proximal end of the adaptive valve clamping device C100, while the second seat body C114 is close to the distal end of the adaptive valve clamping device C100. It should be noted that in other examples, the adaptive valve clamping device C100 can intervene in the heart through a transapical route, the second end C124 of the adjusting portion C120 is a distal end of the adaptive valve clamping device C100, and the first end C122 of the adjusting portion C120 is a proximal end thereof; and the first seat body C112 of the supporting portion C110 is close to the distal end of the adaptive valve clamping device C100, while the second seat body C114 is close to the proximal end of the adaptive valve clamping device C100.

The adaptive valve clamping device C100 is closed during delivery, and can be released and open when delivered to the mitral valve of the patient to clamp the valve leaflet. The first seat body C112 of the supporting portion C110 is located in the hollow adjusting portion C120 whether in the delivering state or in the released and opening state and will not be exposed in the delivery device C200 or in the heart, so as to avoid the scouring of blood and minimize the formation of thrombosis after implantation. Moreover, after the implantation of the adaptive valve clamping device C100, the direct contact between the supporting portion C110 and the valve leaflet is avoided, and accompanied by the long-term pulsation of the valve leaflet, the supporting portion C110 is prevented from wearing the valve leaflet or even causing perforation of the valve leaflet, thus improving the safety.

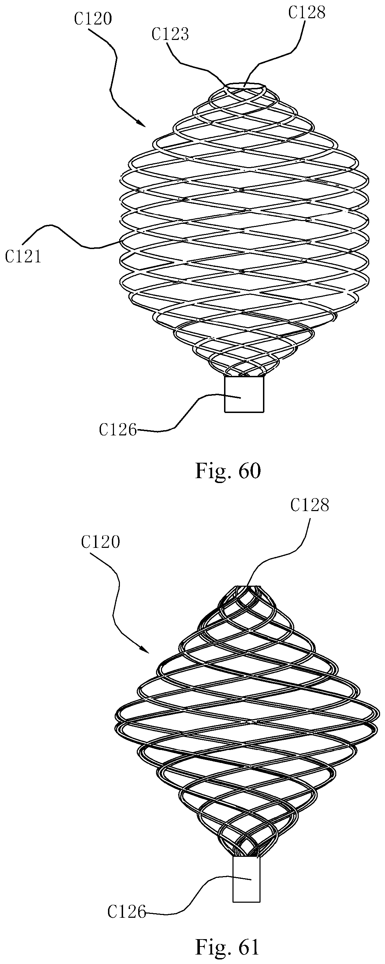

Referring to Fig. 59 and Fig. 60, the first end C122 of the adjusting portion C120 is provided with a first head C126 which is movably sleeved outside the second seat body C114. There is a clearance fit between the inner cavity surface of the first head C126 and the outer surface of the second seat body C114. In this way, the first end C122 of the adjusting portion C120 is drawn in through the first head C126, and the first head C126 can move on the second seat body C114 of the supporting portion C110, so that the adjusting portion C120 can move with respect to the supporting portion C110.

Specifically, the first head C126 is a double-layer structure comprising two coaxially nested tube bodies. One end of the sandwich cavity between the two tube bodies is closed, and the other end is open for drawing in the first end C122 of the adjusting portion C120. Both ends of the tube body with smaller radial size are open, and an inner cavity thereof is the inner cavity of the first head C126. The first head C126 may be a metal steel sleeve.

In order to ensure that the adjusting portion C120 can be movably sleeved on the second seat body C114 of the supporting portion C110, the fit clearance between the inner cavity surface of the first head C126 and the outer surface of the second seat body C114 ranges from 0.01 mm to 3 mm, preferably 0.05 mm to 3 mm. Preferably, the fit clearance between the inner cavity surface of the first head C126 and the outer surface of the second seat body C114 ranges from 0.05 mm to 1 mm, so as to avoid shaking of the first head C 126 and the adjusting portion C120 outside the supporting portion C110 caused by the excessive fit clearance. More preferably, the fit clearance between the inner cavity surface of the first head C126 and the outer surface of the second seat body C114 ranges from 0.05 mm to 0.2 mm, which can not only avoid the free sliding and rotation of the first head C 126 on the second seat body C114 caused by the excessive fit clearance, but can also make the first head C126 move axially with respect to the second seat body C114 by applying an appropriate force to the first head C126 and/or the second seat body C114.

Further, the surface roughness of the inner cavity surface of the first head C126 ranges from 0.1 µm to 2.5 µm, and/or the surface roughness of the outer surface of the second seat body C114 ranges from 0.1 µm to 2.5 µm. It should be noted that the inner cavity surface of the first head C126 and/or the outer surface of the second seat body C114 are set to be rough, so that a contact surface between the first head C126 and the second seat body C114 is not smooth, which can further ensure the effect of restricting the free sliding and rotation of the first head C126 on the second seat body C114. The range of the surface roughness is set reasonably, and the first head C126 can still move axially with respect to the second seat body C 114 by applying an appropriate force to the first head C126 and/or the second seat body C114.

The second end C124 of the adjusting portion C120 has an opening C128, which is convenient for the delivery device C200 to penetrate into the adjusting portion C120 and connect with the supporting portion C110. Meanwhile, there is no head or other parts exposed outside the second end C124, which can avoid thrombosis and wear of the valve leaflet. The size of the opening C128 is less than or equal to the size of the first seat body C112 of the supporting portion C110, so as to ensure that the first seat body C112 of the supporting portion C110 will not extend from the adjusting portion C120 in the natural state and the compressed state.

The self-expanding body C121 of the adjusting portion C120 has a hollow accommodation cavity, and the first seat body C112 of the supporting portion C110 is provided in the hollow accommodation cavity. In an implementation mode, the self-expanding body C121 is of a mesh structure, which is woven or cut from a shape memory material. Metal materials, polymer materials or metal polymer composites may be selected, preferably a shape memory metal material with certain elasticity such as stainless steel, cobalt chromium alloy or nickel titanium. For example, a hyper-elastic nickel titanium alloy material may be selected for weaving or cutting to form the natural state after heat setting treatment. Specifically, all the mesh wires of the mesh structure are fixedly drawn in the sandwich cavity of the first head C126 at the first end C122, and all the mesh wires of the mesh structure are enclosed at the bending part at the edge of the second end C124 to form an opening C128. The shape of the bending may be set as required, such as bending once, bending back after winding for several times to form at least one ring, or the like. Further, referring to Fig. 60, the adjusting portion C120 further comprises an annular structure C123 provided at the edge of the second end C124 to stabilize the shape of the opening C128. All the mesh wires of the mesh structure at the second end C 124 are wound and connected to the annular structure C123. The annular structure C123 is made of flexible or elastic materials, and its wire diameter is larger than that of the mesh wire of the woven mesh structure, so as to provide a certain support force for the opening C128 of the mesh structure, but not affect the axial deformation ability and bending ability of the mesh structure. A polytetrafluoroethylene (PTFE) coating may be applied on the surface of nickel titanium alloy by spraying, dipping and the like to have superior corrosion resistance, chemical resistance and wear resistance, and thus can play the role of surface protection, anti-corrosion, and prolongation of the service life of parts. Meanwhile, since PTFE has a good friction coefficient, the damage of self-expanding body C121 to the valve leaflet can be effectively reduced.

Certainly, the self-expanding body C121 of the adjusting portion C120 may be other elastic hollow structures. For example, the self-expanding body C121 may be of a dense structure or a porous structure, the dense structure is colloidal silica, and the porous structure is a sponge. The edge of the second end C124 of the dense structure or the porous structure forms an opening C128, and the first end C122 is provided with the first head C126.

For the self-expanding body C121 of the dense structure, the self-expanding body C121 is filled between the anterior and posterior mitral leaflets of the mitral valve, which can completely hinder the blood flow from scouring the interior of the adaptive valve clamping device C100; prevent the adaptive valve clamping device C100 from falling off due to the scouring of the high-speed flowing blood during fine tuning, and prevent the clamping portion C130 from falling off under the continuous impact of blood after implantation; and prevent the blood from depositing and forming thrombus at the dead corner between the clamping portions C130 of the adaptive valve clamping device C100. The self-expanding body C121 of the mesh structure has better elastic deformation ability, which can better adapt to the anatomical structure of the mitral valve and avoid damage to the valve leaflet caused by excessive pulling of valve leaflets.

Further, a film (not shown) is applied to at least part of the outer surface of the self-expanding body C121 of the mesh structure. The film may be a woven mesh structure with a plurality of mesh holes. The self-expanding body C121 with a film can not only further improve the blocking effect of blood flow and reduce central regurgitation, but also increase biocompatibility, avoid allergy and inflammatory reaction of the valve tissue, improve product safety, and form an artificial barrier on the atrial side of the valve leaflet, thereby blocking the outflow of thrombus formed by repeated scouring of blood at the internal dead corner of the adaptive valve clamping device C100. The film material may be made of polyethyleneglycol terephthalate (PET), polypropylene (PP), polytetrafluoroethylene, polyurethane and other polymer materials. Preferably, the film material is made of PET.

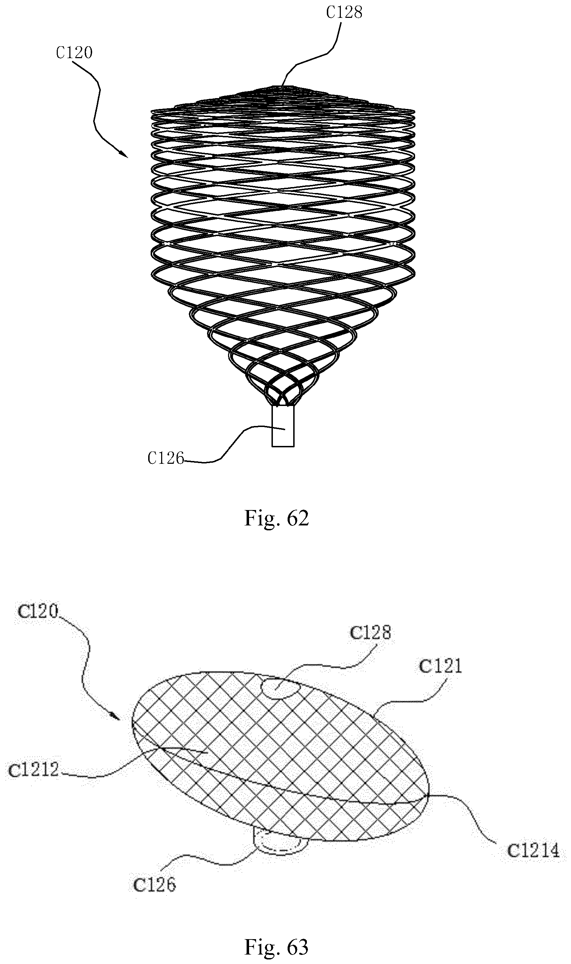

In order to prevent the self-expanding body C121 from affecting the relative opening and closing between the clamping portion C130 and the supporting portion C110 and affecting the clamping effect on the valve leaflet, the diameter of a part of the self-expanding body C 121 close to the first end C 122 should be less than the diameter of other parts of the self-expanding body C121. For example, in the example of Fig. 60, the middle of the self-expanding body C121 is cylindrical, both ends are cones, and the cone angles of the cones at both ends are the same. It should be noted that the self-expanding body C121 may also be any other shape, as long as the diameter of the part close to the first end C122 does not affect the clamping effect. For example, a spindle structure with the same cone angle at both ends shown in Fig. 61 or a structure with different cone angles at both ends shown in Fig. 62.

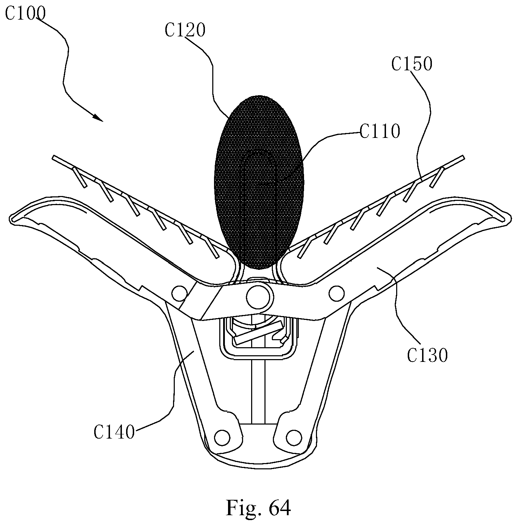

Referring to Fig. 63 and Fig. 64, the self-expanding body C121 may comprise a plurality of first curved surfaces C1212 and a plurality of second curved surfaces C1214. The first curved surface C1212 and the second curved surface C1214 are adjacent to each other and smoothly connected together, that is, the first curved surface C1212 is only adjacent to the second curved surface C1214, and the second curved surface C1214 is only adjacent to the first curved surface C1212, and ends of the plurality of first curved surfaces C1212 and the plurality of second curved surfaces C1214 are connected to each other and form an opening C128. The first curved surface C1212 faces the clamping portion C130, and the area of the second curved surface C1214 is less than that of the first curved surface C1212. With the closing of the adaptive valve clamping device C100, the first curved surface C1212 is pressed by the clamping portion C130 and the valve leaflet, and the adjusting portion C120 extends along the axial direction and gradually fits in the valve leaflet to ensure the contact area with the valve leaflet, so as to better adapt to the shape of the valve leaflet. By increasing the contact area between the first curved surface C1212 and the valve leaflet, the gap between the adaptive valve clamping device C100 and the valve leaflet is reduced, so as to slow down the blood flow and hinder the blood flow from scouring the adaptive valve clamping device C100. Preferably, the curvature of the first curved surface C1212 may also be greater than that of the second curved surface C1214, so that the self-expanding body C121 presents a flat ellipsoid shape, so as to avoid affecting the closing of the clamping portion C130 and adapt more to the anatomical structure of the valve leaflet.

In other implementation modes, the first end C122 of the adjusting portion C120 is provided with a first head C126, and the second end C124 of the adjusting portion C120 is provided with a second head. There is a clearance fit between the inner cavity surface of the first head C126 and the outer surface of the second seat body C114. In this way, the first end C122 and the second end C124 of the adjusting portion C120 are both drawn in by the head. In the present implementation mode, the specific structure of the second head is the same as that of the first head C126, which is the aforementioned double-layer structure, which will not be repeated here.

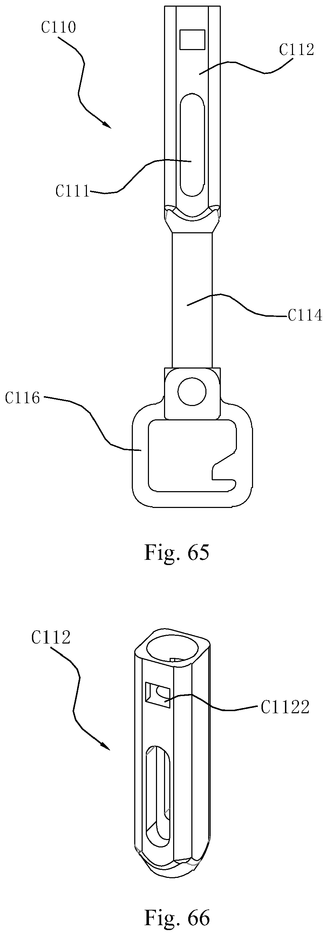

Referring to Fig. 65, the supporting portion C110 further comprises a third seat body C116 connected to the second seat body C114. The inner diameter of the first head C126 is less than the outer diameter of the first seat body C112, and the inner diameter of the first head C126 is less than the outer diameter of the third seat body C116. Thus, the movement stroke of the first head C126 at the first end C122 of the adjusting portion C120 is limited by the first seat body C112 and the third seat body C116, and the adjusting portion C120 may move axially on the second seat body C114 without falling off from the second seat body C114. Since the adjusting portion C120 has a certain weight, under the action of gravity, the initial position of the adjusting portion C120 is located at the distal end of the second seat body C114 and close to the proximal end of the third seat body C116.

Specifically, the inner diameter of the first head C126 should be at least 0.01 mm, preferably 0.05 mm to 3 mm smaller than the outer diameter of the first seat body C112. The inner diameter of the first head C126 should be at least 0.01 mm, preferably 0.05 mm to 3 mm smaller than the outer diameter of the third seat body C116. Both the first head C126 and the second seat body C114 may be of a circular tube structure, which is convenient for the axial movement of the first head C126 on the second seat body C114. The first seat body C112 and the second seat body C114 may be integrally molded, or separately molded and then fixed by welding . The third seat body C116 and the second seat body C114 may be fixed together by common detachable or non-detachable connection means such as welding, bonding, threaded connection, crimping, and bolt locking. The present example adopts welding connection. Specifically, the distal end of the second seat body C114 of the supporting portion C110 may penetrate in from the second end C124 of the adjusting portion C120, and penetrate out from the first end C122 of the adjusting portion C120; and then the distal end of the third seat body C116 and the proximal end of the second seat body C114 are connected integrally by welding.

In other implementation modes, the supporting portion C110 further comprises the third seat body C116 connecting the second seat body C114. The end of the second seat body C114 connected to the first seat body C112 is provided with a limiting part (not shown in the figure). The inner diameter of the first head C126 is less than the outer diameter of the limiting part, and the inner diameter of the first head C126 is less than the outer diameter of the third seat body C116. Thus, the movement stroke of the first head C126 at the first end C122 of the adjusting portion C120 is limited by the limiting part and the third seat body C116, and the adjusting portion C120 may move axially on the second seat body C114 without falling off from the second seat body C114.

Referring to Fig. 65 to Fig. 69, the supporting portion C 110 is provided with a penetrating channel C111 along the axial direction to cooperate with the driving portion C140 and the delivery device C200. The first seat body C112 and the second seat body C114 are circular tubes with both end faces axially penetrated. At least two locking positions C1122 are provided on the tube wall of the first seat body C112 for detachable connection with the locking nose C222 of the delivery device C200. After the locking nose C222 (referring to Fig. 69) of the delivery device C200 is locked into the locking position C1 122, the delivery device C200 is lockingly connected to the supporting portion C110 to deliver the adaptive valve clamping device C100. When the locking nose C222 is disconnected from the locking position C1122, the adaptive valve clamping device C100 is separated from the delivery device C200 and released in the body.

The distal end of the third seat body C116 is a square structure and the proximal end thereof is a trapezoid structure. The distal end of the third seat body C116 is radially provided with an accommodation cavity C1162 penetrating the two opposite sides of the third seat body C116. The proximal and distal ends of the second seat body C114 are provided with a through hole axially penetrating the accommodation cavity C1162. Two opposite planes of the trapezoid structure of the proximal end of the second seat body C114 are respectively provided with a connecting block C1164, and the connecting block C1164 is provided with a connecting hole to be rotationally connected to the clamping portion C130. The cavity of the first seat body C112, the cavity of the second seat body C114, the through hole of the third seat body C116 and the accommodation cavity C1162 are connected to form a penetrating channel C111. It should be noted that the structure of the supporting portion C110 here is only an example and is not a limitation of the present disclosure. Based on the teachings of the present disclosure, other structures of the supporting portion C110 adopted by one skilled in the art are within the protection scope of the present disclosure.

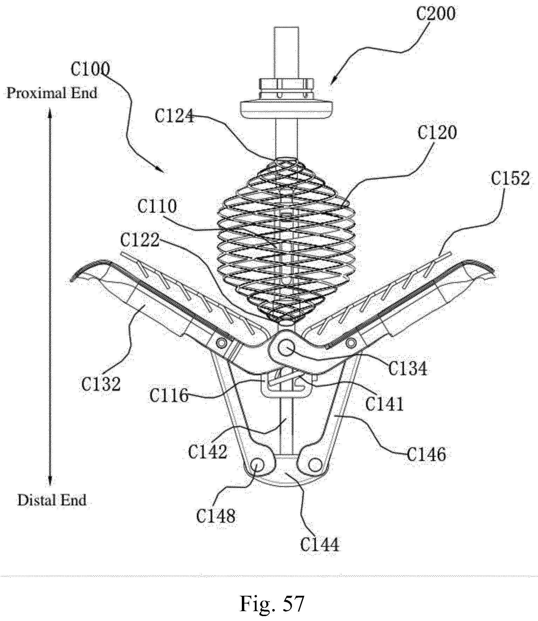

Referring to Fig. 56 and Fig. 57, the clamping portion C130 is rotationally connected to the third seat body C116 of the supporting portion C110. The clamping portion C130 comprises at least two clamp arms C132, that is, at least two clamp arms C132 are rotationally connected to the third seat body C116 of the supporting portion C110, and at least two clamp arms C132 are symmetrically provided in the circumferential direction with respect to the adjusting portion C120. Further, the adaptive valve clamping device further comprises a driving portion C140 connected to each of the clamp arms C132 to drive each of the clamp arms C132 to rotate around the supporting portion C110, so as to drive each of the clamp arms C132 to close by approaching the adjusting portion C120 or open by departing from the adjusting portion C120. The clamp arms C132 may be rotationally connected together on the third seat body C116 through the connecting shaft C134. The connecting shaft C134 passes through the connecting hole on the third seat body C116 and each of the clamp arms C132, so as to rotationally connect the clamp arm C132 to the third seat body C116. Then, driven by the driving portion C140, the clamp arms C132 cooperate with each other and open or close with respect to rotation of the supporting portion C110. When the clamp arm C132 is closed, the second end C124 of the adjusting portion C120 is slightly lower than the end face of the free end (i.e., the proximal end) of the clamp arm C132; in this way, the adjusting portion C120 will not be exposed from the proximal end face of the clamp arm C132 after closing, thereby ensuring that the flanging terminal end of the clamp arm C132 abuts against the valve leaflet to increase the contact area with the valve leaflet, comply with the angle and direction of the valve leaflet, and avoid the risk of thrombosis caused by excessive exposure of the adjusting portion C120 in the left atrium.

In the illustrated example, the clamping portion C130 comprises two clamp arms C132 symmetrically provided in the circumferential direction with respect to the adjusting portion C120 for clamping the two valve leaflets of the mitral valve. In other implementation modes, the clamping portion C130 may comprise three clamp arms C132 provided circumferentially around the adjusting portion C120 for clamping three valve leaflets of the tricuspid valve. It should be noted that this is only an example, and one skilled in the art can select an appropriate number of clamp arms C132 as needed, such as two, three or more clamp arms C132. In the delivering state, the driving portion C140 drives the clamp arm C132 to close around the adjusting portion C120, so as to reduce the outer diameter of the adaptive valve clamping device C100 and facilitate delivering; and after the adaptive valve clamping device C100 opens in the body, the driving portion C140 drives the clamp arm C132 to clamp the valve leaflets between the clamp arm C132 and the adjusting portion C120 to realize valve leaflet clamping.

Further, the adaptive valve clamping device C100 comprises a gripping portion C150, which is provided between the clamp arm C132 and the adjusting portion C120 and may be close to or away from the clamp arm C132 and at least partially accommodated in the inner surface of the clamp arm C132 (as shown in Fig. 56) in the natural state. It should be noted that in some examples, the gripping portion C150 has a shape memory function, so as to be close to the clamp arm C132 in the natural state; and in other examples, the gripping portion C150 may be made of a material that does not have a shape memory function and drives the gripping portion C150 to be close to the clamp arm C132 by means of a push rod or the like. The gripping portion C150 comprises at least two gripping arms C152. Generally, the number of the gripping arms C152 is the same as the number of the clamp arms C132, and setting modes thereof are the same. In this way, the gripping arm C152 cooperates with the clamp arm C132 to realize the clamping function.

Preferably, the gripping arm C152 is made of a shape memory material such as nickel titanium alloy, and the free end of the gripping arm C152 is provided with an adjusting wire hole of an adjusting wire (not shown in the figure) for connecting the delivery device C200. The free end of the gripping arm C152 may be controlled by the adjusting wire extending to the outside of the patient. In the delivering state, the free end of the gripping arm C152 is tensioned by the adjusting wire and fits in the adjusting portion C120; and when the clamp arm C132 opens in the body and clamps the valve leaflet, the control of the adjusting wire on the free end is released to release the gripping arm C152, and the gripping arm C152 restores the natural state due to its shape memory function and presses the valve leaflet to the clamp arm C132.

In the natural state, the gripping portion C150 is at least partially accommodated in the inner surface of the clamping portion C130, that is, the gripping arm C152 is at least partially accommodated in the inner surface of the clamp arm C132, so that the outer diameter of the adaptive valve clamping device C100 in the delivering state can be reduced after the adaptive valve clamping device C100 is closed, which is conducive to delivering. After the clamp arm C132 and the gripping arm C152 cooperate to clamp the valve leaflet, the recessed inner surface of the clamp arm C132 can increase the contact area between the clamp arm C132 and the valve leaflet, and causes the gripping arm C152 to press the valve leaflet into the inner surface of the clamp arm C132 to increase the clamping force on the valve leaflet.

Referring to Fig. 57, the driving portion C140 comprises a driving shaft C142, a connecting seat C144 and at least two connecting rods C146. One end of each of the connecting rods C146 is connected to one clamp arm C132, and the other end is pivotally connected to the connecting seat C144. One end of the driving shaft C142 is connected to the connecting seat C144, and the other end thereof movably penetrates in the third seat body C116. Specifically, the number of the connecting rods C146 is consistent with the number of the clamp arms C132. One end of each of the connecting rods C146 is connected to one clamp arm C132, and the other end thereof is connected to the connecting seat C144 through the pivot C148. The driving shaft C142 passes through the penetrating channel C111 of the supporting portion C110 along the axial direction and movably penetrates the third seat body C116 to be connected to the connecting seat C144. When the driving shaft C142 moves axially with respect to the third seat body C116, the connecting rod C146 rotates and drives the clamp arm C132 to open and close with respect to the third seat body C116. When the driving shaft C142 moves axially to the distal end with respect to the third seat body C116, the connecting rod C146 rotates and drives the clamp arm C132 to open, and the adaptive valve clamping device C100 is in the opened state; and when the driving shaft C 142 moves axially to the proximal end with respect to the third seat body C116, the connecting rod C146 rotates and drives the clamp arm C132 to close, and the adaptive valve clamping device C100 is in the closed state.

In the illustrated example, the clamping portion C130 comprises two clamp arms C132, and two cooperating connecting rods C146 are correspondingly provided. The distal end of the clamp arm C132 is rotationally connected to the third seat body C116 through the connecting shaft C134 such as a pin or bolt, the distal end of the connecting rod C146 is rotationally connected to the connecting seat C144 through the pivot C148 such as a pin or bolt, and the proximal end of the connecting rod C146 is connected to the clamp arm C132. When the driving shaft C142 moves axially to the distal end with respect to the third seat body C116, the connecting rod C146 rotates and drives the clamp arm C 132 to rotate around the connecting shaft C 134 and open with respect to the third seat body C116, and the adaptive valve clamping device C100 is in the opened state. When the driving shaft C142 moves axially to the proximal end with respect to the third seat body C116, the connecting rod C146 rotates and drives the clamp arm C132 to rotate around the connecting shaft C134 and close with respect to the third seat body C116.

The shape of the connecting seat C144 is of any structure such as a hemisphere, a spherical crown or a warhead shape, so as to make the adaptive valve clamping device C100 easier to be pushed in the body. The driving shaft C142 and the connecting seat C144 may be an integrated structure or a non-integrated structure. The connecting seat C144 may be fixedly provided at the distal end of the driving shaft C142 by welding or other means. In order to ensure the safety after implantation, the driving shaft C142 and the connecting seat C144 are made of biocompatible materials such as polyester, silicone, stainless steel, cobalt alloy, cobalt chromium alloy and titanium alloy, preferably stainless steel or cobalt chromium alloy with high hardness.

Further, the driving portion C140 further comprises a locking part C141 provided in the third seat body C116 which restricts the relative movement of the driving shaft C 142 and the third seat body C116. In the delivering state, the locking part C141 restricts the relative movement of the driving shaft C142 and the third seat body C116, so as to ensure that the clamping portion C130 is always closed with respect to the adjusting portion C120 and the supporting portion C110, and avoid accidental opening of the clamping portion C130. After the adaptive valve clamping device C100 reaches the vicinity of the mitral valve, the restriction of the locking part C141 on the driving shaft C142 is unlocked, so that the clamping portion C130 can be driven by the driving portion C140 to open with respect to the adjusting portion C120 and the supporting portion C110 and clamp the valve leaflet. The locking part C141 may be a combination of a deformed elastic sheet and a steel sheet in the related art, which will not be described here.

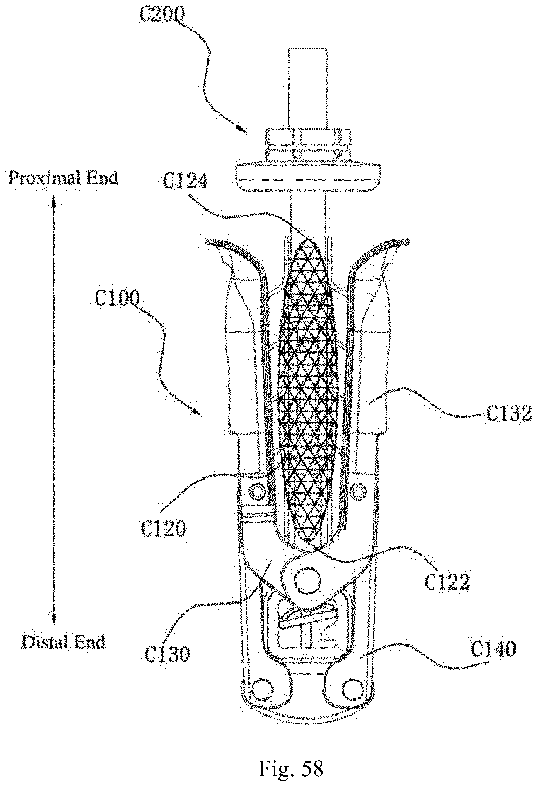

Referring to Fig. 57 and Fig. 58, the first example of the present disclosure further provides a valve clamping system. The valve clamping system comprises the adaptive valve clamping device C100 and the delivery device C200. The delivery device C200 comprises a pushing shaft C210 with a certain axial length and a mandrel (not shown in the figure) movably penetrating in the pushing shaft C210. The pushing shaft C210 is detachably connected to the supporting portion C110, and the mandrel is configured to drive the clamp arm C132 of the clamping portion C130 to rotate around the supporting portion C110. It should be noted that only part of structure of the delivery device C200 is listed here, and other parts can adopt any suitable structure in the related art, which will not be described here.

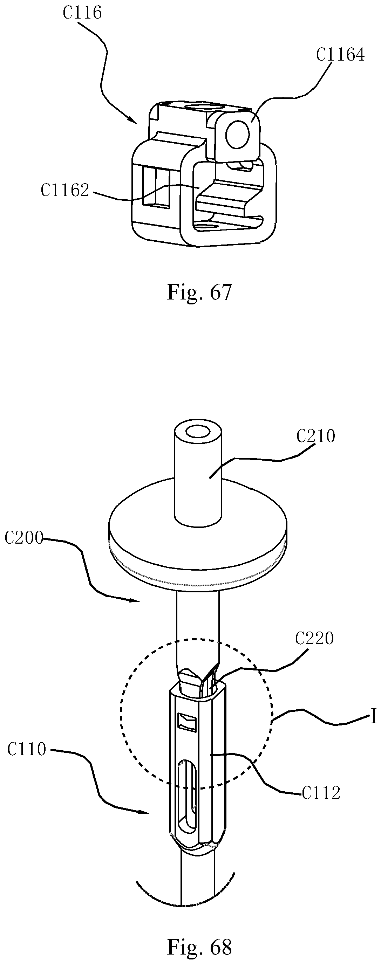

Specifically, the mandrel is detachably connected to the driving portion C140. The mandrel is configured to drive the clamp arm C132 of the clamping portion C130 by the driving portion C140 to rotate around the supporting portion C110, so as to drive the clamping portion C130 to open or close. Referring to Fig. 68 and Fig. 69, the tube wall at the proximal end of the first seat body C112 of the supporting portion C110 is symmetrically provided with two locking positions C1122 connected to the cavity. The distal end of the pushing shaft C210 is provided with a fixing part C220. The fixing part C220 comprises two branches, and the terminal end of each branch is a protruded locking nose C222. In the natural state, the two branches point to the central axis of the fixing part C220. When the adaptive valve clamping device C100 and the delivery device C200 are assembled, the fixing part C220 at the distal end of the pushing shaft C210 is inserted into the first seat body C112 of the supporting portion C110, and then the mandrel is inserted into the pushing shaft C210 until the mandrel is inserted into the fixing part C220 to push the two branches of the fixing part C220 outward, so that the locking noses C222 at the terminal end of the branches are respectively locked into the locking positions C1122, and the supporting portion C110 is connected to the pushing shaft C210, that is, the adaptive valve clamping device C100 is connected to the delivery device C200. The proximal end of the driving shaft C142 is provided with an external thread, and the mandrel is provided with an internal thread. The mandrel is threadedly connected to the driving shaft C142 after being inserted into the fixing part C220, so that the axial movement of the driving shaft C142 can be controlled through the mandrel.

When the mandrel is detached from the driving shaft C142 and the mandrel is withdrawn from the fixing part C220 and the pushing shaft C210, the two branches of the fixing part C220 restore the inward natural state, and the locking nose C222 is detached from the locking position C1122 of the first seat body C112, so that the connection between the adaptive valve clamping device C100 and the delivery device C200 is released. The fixing part C220 may be made of a material with certain hardness and elasticity such as nickel titanium. The pushing shaft C210 may adopt a multi-layer composite tube. The mandrel may be made of stainless steel.

It should be noted that after the adaptive valve clamping device C100 is connected to the delivery device C200, the adaptive valve clamping device C100 is closed, and the adaptive valve clamping device C100 is delivered to the patient's mitral valve through the delivery device C200. Then the driving shaft C142 is driven by the mandrel to move to the distal end along the axial direction. The driving shaft C142 drives the connecting rod C146 to rotate. The connecting rod C146 drives the clamp arm C132 to open until the clamp arm C132 is fully opened with respect to the adjusting portion C120 and the supporting portion C110, so that the adaptive valve clamping device C100 is in an opened state. After the clamping portion C130 and the gripping portion C150 cooperate and clamp the valve leaflets of the valve tissue, the driving shaft C142 is driven by the mandrel to move toward the proximal end along the axial direction. The driving shaft C142 drives the connecting rod C146 to rotate, and the connecting rod C146 drives the clamp arm C132 to close until the clamp arm C132 is completely closed with respect to the adjusting portion C120 and the supporting portion C110, so that the adaptive valve clamping device C100 is closed and falls below the valve. After that, the connection between the mandrel and the driving shaft C142 may be released, the mandrel is withdrawn from the fixing part C220, and the locking nose C222 is separated from the locking position C1122 of the supporting portion C110, so as to disengage the adaptive valve clamping device C100 from the delivery device C200.

Since the connecting position (i.e. disengagement position) between the adaptive valve clamping device C100 and the delivery device C200 is located in the adjusting portion C120 of the adaptive valve clamping device C100, when the second end C 124 of the adjusting portion C120 is provided with the opening C128, no part will hook the locking nose C222 at the terminal end of the branch of the fixing part C220 to facilitate the release of the adaptive valve clamping device C100. In addition, the disengagement position is provided inside the adjusting portion C120, which can reduce the axial size of the disengagement position, so as to reduce the weight of the whole adaptive valve clamping device C100 and reduce the load on the heart; the disengagement position is not directly scoured by blood, which can avoid damage to the valve leaflet caused by repeated wear of the valve leaflet at the disengagement position and reduce the risk of thrombosis.

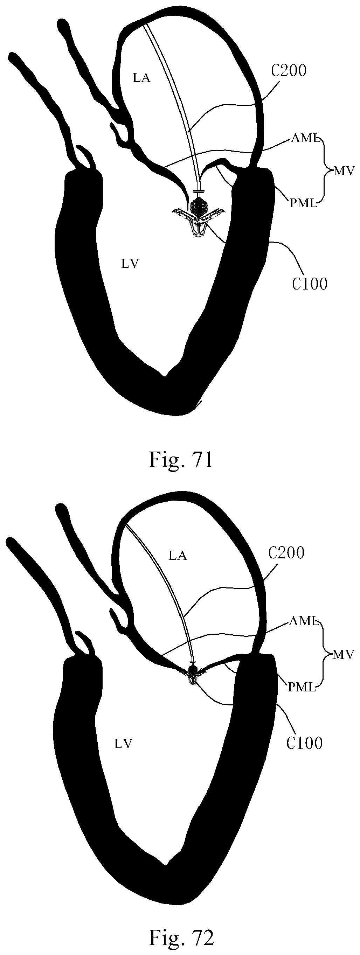

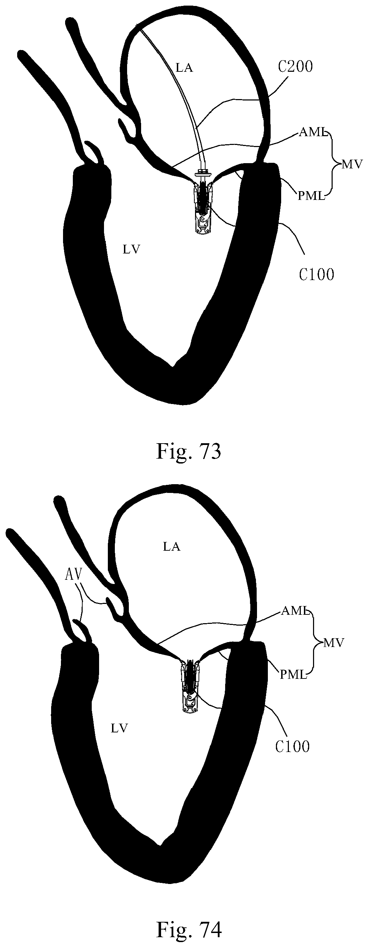

Referring to Fig. 70 to Fig. 74, a use process of the adaptive valve clamping device C100 of the present disclosure is described taking anterograde approach and repair of the mitral valve via the left atrium as an example:
Step 1: pushing the delivery device C200 and the adaptive valve clamping device C100 connected thereto from the left atrium LA to the left ventricle LV via the mitral valve MV by a guiding device (not shown) such as a bendable sheath, as shown in Fig. 70;
Step 2: adjusting the adaptive valve clamping device C100 to approach the anterior mitral leaflet AML and the posterior mitral leaflet PML of the mitral valve MV;
Step 3: unlocking the locking part C141 in the third seat body C116, pushing the mandrel and the driving shaft C142 to the distal end, driving the clamp arm C132 to open with respect to the supporting portion C110 and the adjusting portion C120, and adjusting the direction of the clamp arm C132, at which time a relative position of the clamp arm C132 and the anterior mitral leaflet AML and the posterior mitral leaflet PML of the mitral valve MV can be observed by medical imaging equipment such as X-ray, so that the clamp arm C132 is perpendicular to a coapting line of the mitral valve MV, as shown in Fig. 71;
Step 4: withdrawing the entire adaptive valve clamping device C100 to the proximal end to make the clamp arm C132 hold the valve leaflet on the left ventricle LV side, and loosening the adjusting wire to release the gripping arms C152 on both sides, wherein the gripping arm C152 on each side presses the valve leaflet on the atrium side and cooperates with the clamp arm C132 on the side to fix the valve leaflet to realize complete clamping of the valve leaflet, as shown in Fig 72;
Step 5: when the anterior mitral leaflet AML and the posterior mitral leaflet PML of the mitral valve MV are respectively clamped between a pair of clamp arms C132 and a pair of gripping arms C152, pulling the mandrel and driving shaft C142 to the proximal end to drive the clamp arm C132 to close, as shown in Fig. 73;
Step 6: releasing the threaded connection between the mandrel and the driving shaft C142, withdrawing the mandrel to release the connection between the adaptive valve clamping device C100 and the delivery device C200, and then withdrawing the delivery device C200 from the body, resulting in an implanted state as shown in Fig. 74, at which time the adaptive valve clamping device C100 pulls the anterior mitral leaflet AML and the posterior mitral leaflet PML of the mitral valve MV toward each other to obtain a double-orifice mitral valve and complete edge-to-edge repair of the mitral valve.

After the adaptive valve clamping device C100 is implanted, the elastic adjusting portion C120 is filled between the anterior mitral leaflet AML and the posterior mitral leaflet PML of the clamped mitral valve MV and abuts against the clamp arm C 132, so as to reduce the central regurgitation and improve the treatment effect. The self-expanding body C121 (such as of mesh structure or porous structure) of the adjusting portion C120 has a cushioning effect on the pulsating valve leaflets, so as to adjust the adaptivity to the pulling degree of the valve leaflets by the adaptive valve clamping device C100 and avoid damaging the valve leaflets. The self-expanding body C121 may be compressed and deformed following the pulsation of the valve leaflet, and the generated elastic force pushes the part of the valve leaflet close to the self-expanding body C121 away from the supporting portion C110, so that the clamping angle between the anterior and posterior mitral leaflets of the mitral valve is less than the opening angle of the clamp arm C132, which can reduce pulling of the valve leaflet by the clamping portion C130 and keep the pulling degree of the valve leaflet by the adaptive valve clamping device C100 always within a reasonable range. In addition, when pressed by the valve leaflet, the adjusting portion C120 will be deformed to a certain extent, and the deformation degree increases with the increase of pressure, so as to avoid the self-expanding body C121 being extruded by the clamp arm C132 and acting on the clamp arm C132 in turn after the valve leaflet is gripped, and to ensure that the gripping effect of the adaptive valve clamping device C100 on the valve leaflet after release is consistent with that before release.

### Second Example

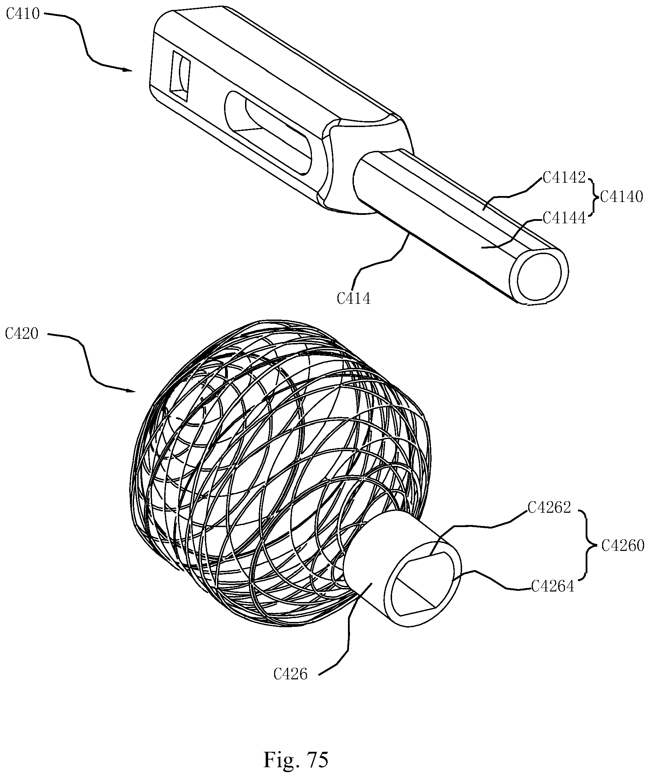

Referring to Fig. 75 to Fig. 78, an adaptive valve clamping device of a second example of the present disclosure is different from the adaptive valve clamping device C100 of the first example in structures of the first head C426 of the adjusting portion C420 and the second seat body C414 of the supporting portion C410.

Specifically, the first head C426 is movably sleeved outside the second seat body C414 of the supporting portion C410, the inner cavity of the first head C426 is provided with a first rotation stop C4260, the outer surface of the second seat body C414 is provided with a second rotation stop C4140 corresponding to the first rotation stop C4260, and the first rotation stop C4260 is detachably fittingly connected to the second rotation stop C4140. In this way, the rotation of the adjusting portion C420 around the axial direction is limited by providing the first rotation stop C4260 and the second rotation stop C4140 which are detachably fittingly connected to avoid the adjusting portion C420 rotating with respect to the second seat body C414, resulting in reduction of the contact area between the adjusting portion C420 and the valve leaflet and affecting the clamping effect on the valve leaflet.

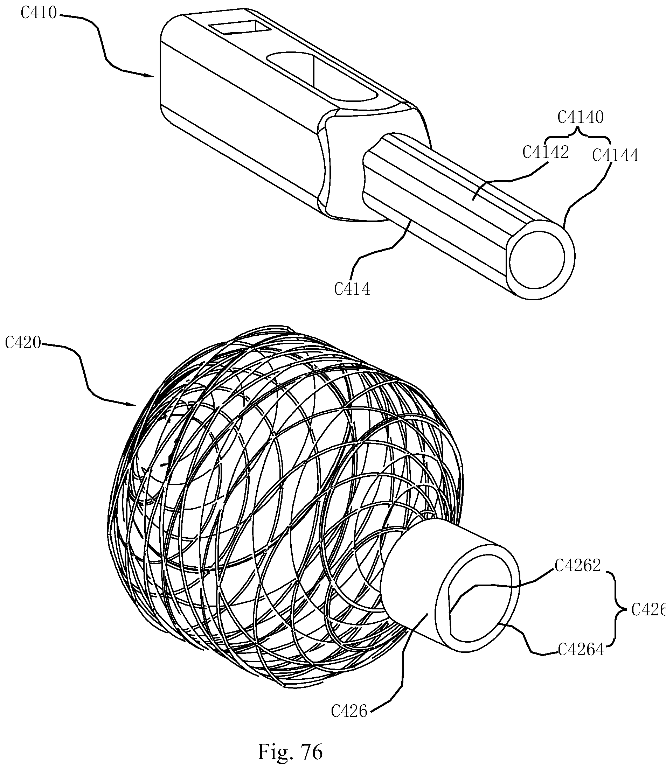

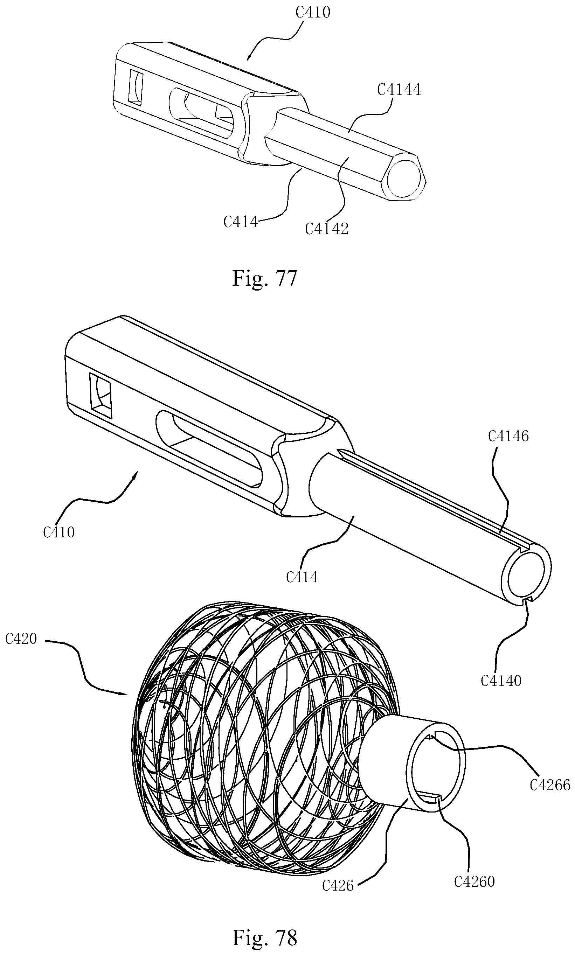

Referring to Fig. 75 to Fig. 77, the first rotation stop C4260 comprises at least one plane surface C4262 and/or at least one cambered surface C4264, and the second rotation stop C4140 comprises at least one plane surface C4142 and/or at least one cambered surface C4144. In the example of Fig. 75, the first rotation stop C4260 comprises two opposite plane surfaces C4262 and two opposite cambered surfaces C4264, that is, the inner cavity of the first head C426 is provided with two opposite plane surfaces C4262 and two opposite cambered surfaces C4264. Correspondingly, the second rotation stop C4140 comprises two opposite plane surfaces C4142 and two opposite cambered surfaces C4144, that is, the outer surface of the second seat body C414 is provided with two opposite plane surfaces C4142 and two opposite cambered surfaces C4144. During assembly, the plane surface C4262 of the first head C426 faces the plane surface C4142 of the second seat body C414, and the cambered surface C4264 of the first head C426 faces the cambered surface C4144 of the second seat body C414. In this way, the first head C426 is sleeved outside the second seat body C414, which can ensure that the axial movement of the adjusting portion C420 is not affected. Meanwhile, the two facing plane surfaces C4142 of the second seat body C414 and the first head C426 can prevent the adjusting portion C420 from rotating axially on the second seat body C414. The spacing between the two plane surfaces C4262 of the first head C426 should be at least 0.01 mm, preferably 0.02 mm to 1 mm larger than the spacing between the two plane surfaces C4142 of the second seat body C414. The spacing between the two cambered surfaces C4264 of the first head C426 should be at least 0.01 mm, preferably 0.05 mm to 3 mm larger than the spacing between the two cambered surfaces C4144 of the second seat body C414. In the example of Fig. 76, the first rotation stop C4260 comprises one plane surface C4262 and one cambered surface C4264, and the second rotation stop C4140 comprises two plane surfaces C4142 and one cambered surface C4144. In the example of Fig. 77, the second rotation stop C4140 comprises three plane surfaces C4142 and three cambered surfaces C4144. Preferably, the cambered surface may be a circular cambered surface. Certainly, alternatively, the first rotation stop C4260 comprises one cambered surface C4264, and the second rotation stop C4140 comprises a plurality of plane surfaces C4142, that is, the inner cavity surface of the first head C426 is a circumferential cambered surface, and the cross-sectional contour of the outer surface of the second seat body C414 is a polygon.

Certainly, the first rotation stop C4260 and the second rotation stop C4140 may be of a polyhedron structure fittingly connected. For example, both the first rotation stop C4260 and the second rotation stop C4140 are of a triangular prism structure, that is, the inner cavity of the first head C426 is provided with three plane surfaces connected to each other, and the outer surface of the second seat body C414 is correspondingly provided with three plane surfaces connected to each other.

Referring to Fig. 78, one of the first rotation stop C4260 and the second rotation stop C4140 is a sliding groove extending axially, and the other is a protrusion fitting in the sliding groove. The axial rotation of the adjusting portion C420 is limited by fit between the sliding groove and the protrusion, and the axial movement of the adjusting portion C420 is not affected. In the figure, the second seat body C414 is provided with a sliding groove C4146 extending along the axial direction, and the inner cavity of the first head C426 is provided with a protrusion C4266 protruded inward and movable along the sliding groove. Preferably, the second seat body C414 is provided with two opposite sliding grooves C4146, and the first head C426 is provided with two protrusions C4266 protruded inward. Certainly, alternatively, the second seat body C414 is provided with an outward protrusion, and the first head C426 is provided with a sliding groove.

In other implementation modes, the second seat body C414 may be provided with an outward protrusion, and the inner cavity of the first head C426 may be provided with an inward protrusion. The protruding structures of the two fit and have a track guiding the movement, and can also prevent the adjusting portion C420 from rotating along the supporting portion C410.

It should be noted that in the second example, the first head C426 is integrally molded with the first rotation stop C4260, and the second seat body C414 is integrally molded with the second rotation stop C4140.

### Third Example

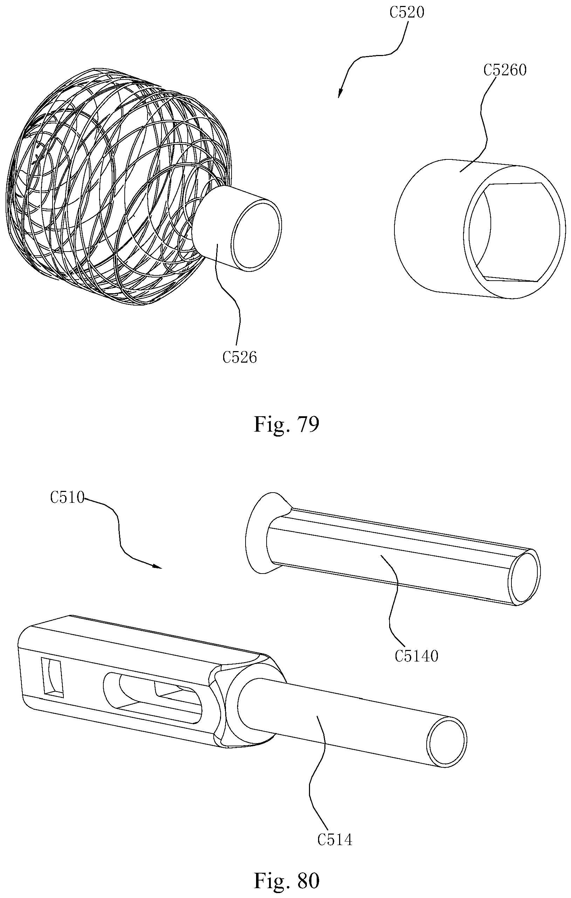

Referring to Fig. 79 and Fig. 80, the difference between an adaptive valve clamping device of a third example and the adaptive valve clamping device of the second example in the present disclosure is that structures of the first head C526 of the adjusting portion C520 and the second seat body C514 of the supporting portion C510 are different. In the present example, the first head C526 is connected to the first rotation stop C5260, and/or the second seat body C524 is connected to the second rotation stop C5140. Specifically, the first head C526 and the first rotation stop C5260 can be fixed together by common detachable or non-detachable connection means such as welding, bonding, threaded connection, crimping, or bolt locking. The second seat body C514 and the second rotation stop C5140 can be fixed together by common detachable or non-detachable connection means such as welding, bonding, threaded connection, crimping, or bolt locking. The present example adopts welding connection.

It can be understood that the valve clamping system of the present disclosure comprises any one of the adaptive valve clamping devices described above and a delivery device capable of delivering the adaptive valve clamping device from outside the body to the vicinity of the mitral or tricuspid valve and clamping the valve leaflet. The above description of the valve clamping device is for purpose of example only and is not limitation of the present disclosure, and a valve clamping device and a valve clamping system comprising the valve clamping device obtained by one skilled in the art based upon the teachings of the present disclosure are within the scope of the present disclosure.

It should be noted that in this specification, relational terms such as "first" and "second" are only used to distinguish one entity or operation from another entity or operation, and do not necessarily require or imply that there is any such actual relationship or sequence among the entities or operations. Moreover, the terms "comprise", "include" or any other variants thereof are intended to cover non-exclusive inclusion, so that a process, method, article, or device that comprises a series of elements comprises not only those elements, but also other elements that are not explicitly listed, or further comprises elements inherent to the process, method, article or device. Without more limitations, the element defined by the phrase "comprise a ..." does not exclude the existence of other same elements in the process, method, article, or device that comprises the element.

The above are only detailed description of the present disclosure to enable one skilled in the art to understand or implement the disclosure. Various modifications to these examples will be obvious to one skilled in the art, and the general principles defined herein can be implemented in other examples without departing from the spirit or scope of the present disclosure. Therefore, the present disclosure will not be limited to the examples shown in the present disclosure, but should conform to the widest scope consistent with the principles and novel characteristics of the present disclosure.

## Claims

1. A valve clamping device comprising:
a supporting portion provided along an axial direction;
a hollow adjusting portion, wherein at least a part of the supporting portion is provided in the adjusting portion, one end of the adjusting portion is sleeved outside the supporting portion, and the other end of the adjusting portion is movable with respect to the supporting portion;
a clamping portion surrounding an outside of the adjusting portion; and
a driving portion connected to the clamping portion to drive the clamping portion to be close to or away from the adjusting portion.

2. The valve clamping device of claim 1, wherein the supporting portion comprises a first seat body and a second seat body connected along the axial direction, and the first seat body is provided in the adjusting portion; and the adjusting portion comprises a first end and a second end provided opposite along the axial direction and a self-expanding body between the first end and the second end.

3. The valve clamping device of claim 2, wherein the first end is fixedly sleeved outside the second seat body.

4. The valve clamping device of claim 3, wherein the second end hangs freely with respect to the supporting portion to be movable with respect to the supporting portion.

5. The valve clamping device of claim 3, wherein the second end is movably sleeved outside the first seat body and is movable along the axial direction with respect to the supporting portion.

6. The valve clamping device of claim 2, wherein the second end is fixedly sleeved outside the first seat body, the first end is movably sleeved outside the second seat body and is movable along the axial direction with respect to the supporting portion.

7. The valve clamping device of claim 2, wherein the first end is movably sleeved outside the second seat body and is movable along the axial direction with respect to the supporting portion.

8. The valve clamping device of claim 7, wherein the second end is sleeved outside the first seat body and is movable along the axial direction with respect to the supporting portion.

9. The valve clamping device of claim 7, wherein the second end hangs freely with respect to the supporting portion to be movable with respect to the supporting portion.

10. The valve clamping device of any one of claims 2 to 9, wherein the self-expanding body is an elastomer, and the adjusting portion is provided with an opening at the second end.

11. The valve clamping device of claim 10, wherein the elastomer is of at least one structure selected from the group consisting of a mesh structure, a frame structure, a dense structure and a porous structure.

12. The valve clamping device of claim 10, wherein the elastomer is of a mesh structure or a frame structure, and at least part of an outer surface and/or at least a part of an inner surface of the elastomer are covered with a biocompatible film.

13. The valve clamping device of any one of claims 10 to 12, wherein the second end of the adjusting portion is a head with a central through hole, a part of the elastomer is penetrated and fixed in the head, and the central through hole forms the opening; or an edge of the second end is sleeved with a hollow sleeve structure to form the opening; or the edge of the second end is enclosed to form the opening.

14. The valve clamping device of claim 10, wherein the elastomer is of a mesh structure, the elastomer is woven from a shape memory material, and a mesh wire woven to form the mesh structure is bent back at the second end to form the opening.

15. The valve clamping device of claim 10, wherein the elastomer is of a frame structure, the frame structure is cut from a shape memory material, the frame structure comprises a plurality of supporting rods, the adjacent supporting rods are spaced apart from or cross-linked with each other, and proximal ends of a plurality of the supporting rods converge to form the opening.

16. The valve clamping device of claim 10, wherein the elastomer is of a dense structure made of silica gel, or the elastomer is of a porous structure made of sponge; and an edge of the second end of the dense structure or the porous structure forms the opening.

17. The valve clamping device of any one of claims 2 to 16, wherein a diameter of at least a part of the self-expanding body in a natural state gradually increases from the first end of the adjusting portion to the second end of the adjusting portion.

18. The valve clamping device of any one of claims 2 to 17, wherein the self-expanding body has a recessed area connected to the second end, the recessed area is recessed toward the first end, and the second end is located between two end faces of the self-expanding body along an axis.

19. The valve clamping device of any one of claims 2 to 18, wherein the self-expanding body comprises a first section, a second section and a third section successively connected;
the first section extends from the second end of the adjusting portion toward a second end of the supporting portion, and the first section surrounds an outside of the second end of the supporting portion; the second section continues to extend radially outward from the first section; and the third section extends radially inward from the second section toward a first end of the supporting portion to the first end of the adjusting portion.

20. The valve clamping device of claim 19, wherein the self-expanding body further comprises a bending section connected between the second end of the adjusting portion and the first section.

21. The adequately fitted valve clamping device of claim 20, wherein a radial dimension of the second section of the self-expanding body ranges from 4 mm to 15 mm, and a radial dimension of the first end of the adjusting portion ranges from 1 mm to 5 mm.

22. The valve clamping device of any one of claims 2 to 21, wherein the self-expanding body comprises a plurality of first curved surfaces and a plurality of second curved surfaces along a circumferential direction, and the first curved surface and the second curved surface are adjacent to each other, the two oppositely provided first curved surfaces face the clamping portion respectively, and an area of the second curved surface is less than an area of the first curved surface.

23. The valve clamping device of claim 4 or 9, wherein the first seat body comprises an interface end connected to the second seat body and a free end provided opposite to the interface end, and the free end is located in the adjusting portion.

24. The valve clamping device of claim 23, wherein the self-expanding body is an elastomer, and the adjusting portion is provided with an opening at the second end; and a size of the opening is less than or equal to a size of the free end.

25. The valve clamping device of any one of claims 1 to 24, wherein a hollow sleeve structure is sleeved outside an edge of the end of the adjusting portion, and the sleeve structure is sleeved outside the supporting portion.

26. The valve clamping device of any one of claims 1 to 25, wherein the clamping portion comprises at least two clamp arms, the at least two clamp arms are symmetrically provided with respect to the adjusting portion, and the driving portion is respectively connected to each of the clamp arms to drive each of the clamp arms to be close to or away from the adjusting portion.

27. The valve clamping device of claim 26, wherein the supporting portion further comprises a base connected to the second seat body, each of the clamp arms is rotationally connected to the base, and there is axial spacing between the first end and a connecting position of the clamp arm and the base .

28. The valve clamping device of claim 27, wherein the driving portion comprises a driving shaft, a connecting seat and at least two connecting rods; wherein one end of each of the connecting rods is connected to the clamping portion, and the other end of each of the connecting rods is pivotally connected to the connecting seat; and one end of the driving shaft is connected to the connecting base, and the other end of the driving shaft movably penetrates in the base.

29. The valve clamping device of claim 28 further comprising a locking portion provided in the base, and the locking portion restricts relative movement of the driving shaft and the base.

30. The valve clamping device of claim 27, wherein the driving portion comprises a driving shaft, an automatic closing unit and at least two connecting rods; the driving shaft movably penetrates in the supporting portion, one end of each of the connecting rods is rotationally connected to one of the clamp arms, and the other end of each of the connecting rods is rotationally connected to the driving shaft; and the automatic closing unit is connected to the clamp arm to cause the clamp arm to abut against the adjusting portion in a natural state.

31. The valve clamping device of claim 27, wherein the driving portion comprises a driving shaft and at least two elastic driving arms, one end of the driving shaft movably penetrates in the supporting portion, and one end of each of the elastic driving arms is fixedly connected to the other end of the driving shaft, the other end of each of the elastic driving arms is respectively connected to one of the clamp arms; and the elastic driving arms are configured to cause the clamp arms to abut against the adjusting portion in a natural state.

32. The valve clamping device of any one of claims 26 to 31, wherein a terminal end of the clamp arm is provided with a flanging section which is a cambered surface overturned toward an outside of the terminal end of the clamp arm, and the self-expanding body protrudes from the flanging section in the axial direction after the clamp arm abuts against the adjusting portion.

33. The valve clamping device of claim 32, wherein the self-expanding body is provided with an adaptation section corresponding to the flanging section, and a shape of the adaptation section toward the flanging section is complementary to the cambered surface.

34. The valve clamping device of any one of claims 1 to 33, further comprising a gripping portion provided between the clamping portion and the adjusting portion, the gripping portion is able to be close to or away from the adjusting portion, and when the gripping portion and the clamping portion are away from the adjusting portion, the gripping portion is at least partially accommodated in an inner surface of the clamping portion.

35. A valve clamping device comprising:
a supporting portion comprising a connecting end and a free end provided oppositely;
a hollow adjusting portion, at least a part of the supporting portion is provided in the adjusting portion, one end of the adjusting portion is sleeved outside the connecting end and connected to the supporting portion, and the other end of the adjusting portion hangs freely;
a clamping portion surrounding an outside of the adjusting portion; and
a driving portion connected to the clamping portion to drive the clamping portion to open or close around the adjusting portion.

36. The valve clamping device of claim 35, wherein a free end of the supporting portion is within the adjustment portion.

37. The valve clamping device of claim 35 or 36, wherein the adjusting portion comprises an elastomer, one end of the elastomer is connected to the supporting portion, and the other end of the elastomer has an opening and hangs freely.

38. The valve clamping device of claim 37, wherein a size of the opening is less than or equal to a size of the free end.

39. The valve clamping device of claim 37 or 38, wherein a proximal edge of the elastomer is sleeved with a hollow sleeve structure to form the opening.

40. The valve clamping device of claim 37 or 38, wherein a proximal edge of the elastomer is enclosed to form the opening.

41. The valve clamping device of any one of claims 37 to 40, wherein the elastomer is of at least one structure selected from the group consisting of a mesh structure, a frame structure, a dense structure and a porous structure.

42. The valve clamping device of claim 41, wherein when the elastomer is of the mesh structure or the frame structure, at least part of an outer surface of the elastomer is covered with a film.

43. The valve clamping device of claim 41, wherein when the elastomer is of the mesh structure or the frame structure, the elastomer is woven or cut from a shape memory material.

44. The valve clamping device of claim 43, wherein when the elastomer is the mesh structure, a mesh wire of the mesh structure is bent back at a proximal end to form the proximal edge.

45. The valve clamping device of claim 43, wherein when the elastomer is of the frame structure, adjacent supporting rods of the frame structure are spaced apart from or cross-linked with each other, and the supporting rods of the frame structure converge at a proximal end to form the proximal edge.

46. The valve clamping device of claim 41, wherein when the elastomer is of the dense structure, the dense structure is made of silica gel; when the elastomer is of the porous structure, the porous structure is made of sponge; and a proximal edge of the dense structure or the porous structure forms the opening.

47. The valve clamping device of any one of claims 37 to 46, wherein a distal end of the elastomer is fixedly sleeved on the supporting portion, or a hollow sleeve structure is sleeved outside a distal edge of the elastomer, and the sleeve structure is fixedly sleeved on the supporting portion.

48. The valve clamping device of any one of claims 35 to 47, wherein the clamping portion comprises at least two clamp arms, the at least two clamp arms are symmetrically provided with respect to the adjusting portion, and the driving portion is connected to each of the clamp arms to drive each of the clamp arms to rotate around the adjusting portion.

49. The valve clamping device of any one of claims 35 to 38, wherein the adjusting portion comprises a plurality of first curved surfaces and a plurality of second curved surfaces, the first curved surfaces and the second curved surfaces are adjacent to each other, the two oppositely provided first curved surfaces face one of the clamp arms respectively, and an area of the second curved surface is smaller than an area of the first curved surface.

50. The valve clamping device of any one of claims 35 to 59, further comprising a gripping portion provided between the clamping portion and the adjusting portion and being able to close or open with respect to the adjusting portion, and when both of the gripping portion and the clamping portion open, the gripping portion is at least partially accommodated in an inner surface of the clamping portion.

51. The valve clamping device of any one of claims 35 to 50 further comprising a base fixedly connected to the supporting portion, and the clamping portion is rotationally connected to the base.

52. The valve clamping device of claim 51, wherein the driving portion comprises a driving shaft, a connecting seat and at least two connecting rods; wherein one end of each of the connecting rods is connected to the clamping portion, and the other end of each of the connecting rods is pivotally connected to the connecting seat; and one end of the driving shaft is connected to the connecting base, and the other end of the driving shaft movably penetrates in the base.

53. The valve clamping device of claim 52 further comprising a locking portion provided in the base, and the locking portion restricts relative movement of the driving shaft and the base.

54. An adequately fitted valve clamping device comprising:
a supporting portion with a certain axial length comprising a first end and a second end provided oppositely;
an adjusting portion comprising a first end and a second end provided oppositely and a self-expanding body between the first end and the second end of the adjusting portion; the first end of the adjusting portion is movably sleeved outside the supporting portion, the second end of the adjusting portion is sleeved outside the supporting portion and fixedly connected to the supporting portion, and the first end of the adjusting portion is located between the first end of the supporting portion and the second end of the adjusting portion; and
a clamping portion provided on the outside of the supporting portion and being able to open or close with respect to the adjusting portion.

55. The adequately fitted valve clamping device of claim 54, wherein a diameter of the self-expanding body in a natural state gradually increases from the first end of the adjusting portion to the second end of the adjusting portion.

56. The adequately fitted valve clamping device of claim 55, wherein the self-expanding body is of a mesh structure made of a shape memory material.

57. The adequately fitted valve clamping device of claim 56, wherein an outside and/or inside of the mesh structure are covered with a biocompatible film.

58. The adequately fitted valve clamping device of any one of claims 54 to 57, wherein the self-expanding body has a recessed area connected to the second end of the adjusting portion, and an terminal end of the recessed area extends toward the first end of the supporting portion to the second end of the adjusting portion, or the terminal end of the recessed area extends toward the second end of the supporting portion to the second end of the adjusting portion.

59. The adequately fitted valve clamping device of claim 58 further comprising a fixing part, and the second end of the adjusting portion penetrates and is fixed in the fixing part to be fixedly connected to the supporting portion through the fixing part.

60. The adequately fitted valve clamping device of any one of claims 54 to 59, wherein the self-expanding body comprises a first section, a second section and a third section successively connected;
the first section extends from the second end of the adjusting portion toward a second end of the supporting portion, and the first section surrounds an outside of the second end of the supporting portion; the second section continues to extend radially outward from the first section; and the third section extends radially inward from the second section toward a first end of the supporting portion to the first end of the adjusting portion.

61. The adequately fitted valve clamping device of claim 60, wherein the self-expanding body further comprises a bending section connected between the second end of the adjusting portion and the first section.

62. The adequately fitted valve clamping device of claim 60 or 61, wherein a radial dimension of the second section of the self-expanding body ranges from 4 mm to 15 mm, and a radial dimension of the first end of the adjusting portion ranges from 1 mm to 5 mm.

63. The adequately fitted valve clamping device of any one of claims 54 to 62, further comprising a driving portion, wherein the clamping portion comprises at least two clamp arms, the at least two clamp arms are symmetrically provided with respect to the adjusting portion, and the driving portion is connected to each of the clamp arms to drive each of the clamp arms to be close to or away from the adjusting portion.

64. The adequately fitted valve clamping device of claim 63, wherein the first end of the supporting portion is provided with a base, each of the clamp arms is rotationally connected to the base, and there is axial spacing between the first end of the supporting portion and the first end of the adjusting portion.

65. The adequately fitted valve clamping device of claim 64, wherein the driving portion comprises a driving shaft, a connecting seat and at least two connecting rods; one end of each of the connecting rods is rotationally connected to one of the clamp arms, and the other end of each of the connecting rods is rotationally connected to the connecting seat; and one end of the driving shaft is connected to the connecting seat, and the other end of the driving shaft movably penetrates in the base.

66. The adequately fitted valve clamping device of claim 65 further comprising a locking portion provided in the base, and the locking portion restricts relative movement of the driving shaft and the base.

67. The adequately fitted valve clamping device of any one of claims 64 to 66, wherein the driving portion comprises a driving shaft, an automatic closing unit and at least two connecting rods; the driving shaft movably penetrates in the supporting portion, one end of each of the connecting rods is rotationally connected to one of the clamp arms, and the other end of each of the connecting rods is rotationally connected to the driving shaft; and the automatic closing unit is connected to the clamp arm to cause the clamp arm to abut against the adjusting portion in a natural state.

68. The adequately fitted valve clamping device of any one of claims 64 to 66, wherein the driving portion comprises a driving shaft and at least two elastic driving arms, one end of the driving shaft movably penetrates in the supporting portion, one end of each of the elastic driving arms is fixedly connected to the other end of the driving shaft, and the other end of each of the elastic driving arms is respectively connected to one of the clamp arms; and the elastic driving arms are configured to cause the clamp arms to abut against the adjusting portion in a natural state.

69. The adequately fitted valve clamping device of any one of claims 63 to 68, wherein a terminal end of the clamp arm is provided with a flanging section, the flanging section is a cambered surface overturned toward an outside of the terminal end of the clamp arm, and the self-expanding body protrudes from the flanging section in the axial direction after the clamp arm abuts against the adjusting portion.

70. The adequately fitted valve clamping device of claim 68, wherein the self-expanding body is provided with an adaptation section corresponding to the flanging section, and a shape of the adaptation section toward the flanging section is complementary to the cambered surface.

71. The adequately fitted valve clamping device of any one of claims 54 to 70 further comprising a gripping portion provided between the clamping portion and the adjusting portion and being able to close or open with respect to the supporting portion.

72. An adaptive valve clamping device comprising:
a supporting portion comprising a first seat body and a second seat body connected to the first seat body;
a hollow adjusting portion, wherein the first seat body is provided in the adjusting portion, the adjusting portion comprises a first end and a second end provided oppositely, and a self-expanding body between the first end and the second end, the first end of the adjusting portion is movably sleeved outside the second seat body and is able to move axially with respect to the second seat body, the second end of the adjusting portion hangs and the first seat body is closer to the second end of the adjusting portion than the second seat body; and
a clamping portion comprising at least two clamp arms, each of the clamp arms is rotationally connected to the supporting portion, a rotationally connected portion of the clamp arms is close to the first end of the adjusting portion, and the clamp arms rotate around the supporting portion to be close to or away from the adjusting portion.

73. The adaptive valve clamping device of claim 72, wherein the first end of the adjusting portion is provided with a first head, and there is a clearance fit between an inner cavity surface of the first head and an outer surface of the second seat body.

74. The adaptive valve clamping device of claim 73, wherein the clearance fit between the inner cavity surface of the first head and the outer surface of the second seat body ranges from 0.01 mm to 3 mm.

75. The adaptive valve clamping device of claim 74, wherein the clearance fit between the inner cavity surface of the first head and the outer surface of the second seat body ranges from 0.05 mm to 1 mm.

76. The adaptive valve clamping device of claim 75, wherein the clearance fit between the inner cavity surface of the first head and the outer surface of the second seat body ranges from 0.05 mm to 0.2 mm.

77. The adaptive valve clamping device of any one of claims 74 to 76, wherein a surface roughness of the inner cavity surface of the first head ranges from 0.1 µm to 2.5 µm, and/or a surface roughness of the outer surface of the second seat body ranges from 0.1 µm to 2.5 µm.

78. The adaptive valve clamping device of any one of claims 73 to 77, wherein the inner cavity of the first head is provided with a first rotation stop, the outer surface of the second seat body is provided with a second rotation stop corresponding to the first rotation stop, and the first rotation stop is detachably fittingly connected to the second rotation stop.

79. The adaptive valve clamping device of claim 78, wherein the first head and the first rotation stop are integrally molded or separately connected, and the second seat body and the second rotation stop are integrally molded or separately connected.

80. The adaptive valve clamping device of claim 78 or 79, wherein the first rotation stop comprises at least one plane surface and/or at least one cambered surface, and the second rotation stop comprises at least one plane surface and/or at least one cambered surface.

81. The adaptive valve clamping device of claim 80, wherein the first rotation stop and the second rotation stop are of a polyhedron structure fittingly connected.

82. The adaptive valve clamping device of any one of claims 78 to 81, wherein one of the first rotation stop and the second rotation stop is a sliding groove extending axially, and the other is a protrusion fitting in the sliding groove.

83. The adaptive valve clamping device of any one of claims 73 to 82, wherein the supporting portion further comprises a third seat body connected to the second seat body, an end of the second seat body connected to the first seat body is provided with a limiting part, an inner diameter of the first head is less than an outer diameter of the limiting part, and the inner diameter of the first head is less than an outer diameter of the third seat body.

84. The adaptive valve clamping device of any one of claims 73 to 81, wherein the supporting portion further comprises a third seat body connected to the second seat body, an inner diameter of the first head is less than an outer diameter of the first seat body, and an inner diameter of the first head is less than an outer diameter of the third seat body.

85. The adaptive valve clamping device of claim 83 or 84, wherein the at least two clamp arms are rotationally connected to the third seat body, and the at least two clamp arms are symmetrically provided in a circumferential direction with respect to the adjusting portion.

86. The adaptive valve clamping device of any one of claims 83 to 85 further comprising a driving portion which comprises a driving shaft, a connecting seat and at least two connecting rods; one end of each of the connecting rods is connected to one of the clamp arms, and the other end of each of the connecting rods is pivotally connected to the connecting seat; and one end of the driving shaft is connected to the connecting seat, and the other end of the driving shaft movably penetrates in the third seat body.

87. The adaptive valve clamping device of claim 86, wherein the driving portion further comprises a locking part provided in the third seat body, and the locking part is configured to limit relative movement of the driving shaft and the third seat body.

88. The adaptive valve clamping device of any one of claims 72 to 87, wherein the second end of the adjusting portion has an opening.

89. The adaptive valve clamping device of claim 88, wherein the second end of the adjusting portion is further provided with a second head.

90. The adaptive valve clamping device of any one of claims 72 to 89 further comprising a gripping portion provided between the clamp arms and the adjusting portion and being able to be close to or away from the clamp arms, and the gripping portion is at least partially accommodated in an inner surface of the clamp arms in a natural state.

91. A valve clamping system comprising the valve clamping device of any one of claims 1 to 90 and a delivery device, wherein the delivery device comprises: a pushing shaft with a certain axial length and a mandrel movably penetrating in the pushing shaft, the pushing shaft and the supporting portion are detachably connected, and the mandrel is connected to the driving portion to drive the clamping portion to open and close with respect to the supporting portion.

92. A valve clamping system comprising the valve clamping device of any one of claims 1 to 90 and a delivery device, wherein the delivery device comprises: a pushing shaft with a certain axial length and a mandrel movably penetrating in the pushing shaft, the pushing shaft and the supporting portion are detachably connected, and the mandrel is configured to drive the clamp arms to rotate around the supporting portion.
